# EUROPÄISCHE PATENTSCHRIFT

(11) **EP 4 150 103 B1**
(45) Veröffentlichungstag und Bekanntmachung des Hinweises auf die Patenterteilung: **09.04.2025**
(21) Anmeldenummer: 21723760.1
(22) Anmeldetag: 11.05.2021
(51) Int. Cl.: C12P 7/6431, C12P 7/62, C12P 7/42, C08G 18/22, C08G 18/36, C08G 18/42, C08G 18/78, C12N 9/20, C12N 9/88, C12P 7/6427, C12P 7/6436, C12P 7/6472, C12P 7/625

(54) **VERFAHREN ZUR HERSTELLUNG VON HYDROXYFETTSÄUREBASIERTEN POLYOLEN**
METHOD FOR MANUFACTURING HYDROXY FATTY ACID-BASED POLYOLS
PROCÉDÉ DE FABRICATION DE POLYOLS À BASE D'ACIDE GRAS HYDROXY

(30) Priorität: 12.05.2020 EP 20174135
(43) Veröffentlichungstag der Anmeldung: 22.03.2023
(73) Patentinhaber: U. Windmöller Innovation GmbH & Co. KG, 32756 Detmold (DE); Universität Bielefeld, 33615 Bielefeld (DE)
(72) Erfinder: GRÖGER, Harald, 33739 Bielefeld (DE); LÖWE, Jana, 32760 Detmold (DE); HORST, Guido, 32278 Kirchlengern (DE); HOHBERG, Thomas, 33699 Bielefeld (DE)
(74) Vertreter: Vossius & Partner Patentanwälte Rechtsanwälte mbB
(86) Internationale Anmeldenummer: PCT/EP2021/062405
(87) Internationale Veröffentlichungsnummer: WO 2021/228804

(56) Entgegenhaltungen:
- WO-A1-2016/151115
- WO-A2-2011/112923
- KR-B1- 101 492 206
- US-A1- 2014 051 780
- PARK JI-YOUNG ET AL: "Production of 13S-hydroxy-9(Z)-octadecenoic acid from linoleic acid by whole recombinant cells expressing linoleate 13-hydratase fromLactobacillus acidophilus", JOURNAL OF BIOTECHNOLOGY, ELSEVIER, AMSTERDAM, NL, vol. 208, 23 May 2015 (2015-05-23), pages 1 - 10, XP029181402, ISSN: 0168-1656, DOI: 10.1016/J.JBIOTEC.2015.05.006
- WOO-RI KANG ET AL: "Gene cloning of an efficiency oleate hydratase from Stenotrophomonas nitritireducens for polyunsaturated fatty acids and its application in the conversion of plant oils to 10-hydroxy fatty acids", BIOTECHNOLOGY AND BIOENGINEERING, vol. 114, no. 1, 5 August 2016 (2016-08-05), US, pages 74 - 82, XP055744502, ISSN: 0006-3592, DOI: 10.1002/bit.26058

## Beschreibung

Die vorliegende Erfindung betrifft ein Verfahren zur Herstellung eines Hydroxyfettsäurekondensats oder eines Gemischs von Hydroxyfettsäurekondensaten, sowie Polyurethan, das erhältlich ist durch Umsetzung eines durch das Verfahren erhältlichen Hydroxyfettsäurekondensats oder Gemischs von Hydroxyfettsäurekondensaten.

### Hintergrund der Erfindung

Konventionelle Polymere basieren häufig auf petrochemischen Rohstoffen. Obwohl petrochemische Rohstoffe nur in endlicher Menge verfügbar sind, haben sich bio-basierte Rohstoffe als Ersatzstoff vor allem im Bereich der Polymere, insbesondere Polyurethane, in vielen Bereichen bis jetzt nicht durchsetzen können.

Trotz der zahlreichen positiven Eigenschaften von bio-basierten Rohstoffen bestehen vielfach noch Bedenken bezüglich der gleichbleibenden Qualität der daraus erhaltenen Produkte. Andererseits besteht zunehmend der Wunsch, Produkte des täglichen Bedarfs unabhängig von petrochemischen Rohstoffen herstellen zu können, auch wenn in vielen Bereichen die Herstellung von bio-basierten Produkten noch mit höheren Kosten verbunden ist. Die vorliegende Erfindung ist auf die Bereitstellung von bio-basierten Polyolen (hierin auch als Hydroxyfettsäurekondensate bezeichnet) insbesondere für die Herstellung von Polyurethanen gerichtet.

WO 2016/151115 A1 beschreibt ein Verfahren zur Herstellung eines oder mehrerer Ester einer oder mehrerer Hydroxyfettsäuren, wobei mindestens eine solche Hydroxyfettsäure eine Hydroxyfettsäure ist, die durch Einwirkung einer Oleathydratase auf ein ungesättigtes Fettsäuresubstrat mit einer cis C9-C10 Doppelbindung erhältlich ist, wobei das Verfahren die Verwendung einer Lipase zur Veresterung der mindestens einen Hydroxyfettsäure in einem wässrigen gepufferten Reaktionsmedium und unter Bedingungen umfasst, die mit der Herstellung der mindestens einen Hydroxyfettsäure in situ durch die Oleathydratase kompatibel sind.

WO 2011/112923 A2 betrifft ein Verfahren zur Herstellung eines Polyurethans, das die folgenden Schritte umfasst: (i) Herstellung eines Copolyester-Präpolymers, das eine oder mehrere Co-Hydroxyfettsäuren oder einen Ester davon umfasst und durch Fermentation eines Ausgangsmaterials unter Verwendung eines manipulierten Hefestamms erhalten wird; und (ii) Herstellung eines Gemischs, das das Copolyester-Präpolymer, ein Isocyanat und gegebenenfalls einen Katalysator umfasst; (iii) Bildung des Copolyester-haltigen Polyurethans; und (iv) Gewinnung des Copolyester-haltigen Polyurethanmaterials.

In Biotechnology and Bioengineering 2017, 114(1), auf den Seiten 74 bis 82, berichteten Kang et al. über die Genklonierung einer effizienten Oleathydratase aus *Stenotrophomonas nitritireducens* für mehrfach ungesättigte Fettsäuren und deren Anwendung bei der Umwandlung von Pflanzenölen in 10-Hydroxyfettsäuren.

KR 101 492 206 B1 betrifft eine Synthese von Glycerinderivaten einer Hydroxyfettsäure und insbesondere Glycerinderivate einer Hydroxyfettsäure, die durch Synthese von 7,10-Dihydroxy-8(E)-octadecensäure (DOD) und Glycerin zu einem Hydroxyfettsäuremonoglycerid unter Verwendung einer Lipase hergestellt werden, sowie ein Herstellungsverfahren dafür.

### Zusammenfassung der Erfindung

Die vorliegende Erfindung betrifft ein Verfahren zur Herstellung eines Hydroxyfettsäurekondensats oder eines Gemischs von Hydroxyfettsäurekondensaten, das Verfahren umfassend die Schritte:
- Bereitstellen einer oder mehrerer Fettsäuren mit mindestens einer C=C Doppelbindungsfunktionalität,
- biotechnologische H₂O-Addition mittels einer Hydratase an mindestens eine C=C-Doppelbindungsfunktionalität der einen oder mehreren Fettsäuren, wobei die Doppelbindung keine terminale Doppelbindung ist, unter Erhalt einer oder mehrerer Hydroxyfettsäuren,
- Umsetzung der einen oder mehreren Hydroxyfettsäure(n) mit einer oder mehreren mindestens zweiwertigen Linkergruppe(n), ausgewählt aus Polyolen, Polyaminen und (Poly)amino(poly)alkoholen, unter Erhalt eines Hydroxyfettsäurekondensats oder eines Gemischs von Hydroxyfettsäurekondensaten, wobei die Umsetzung der einen oder mehreren Hydroxyfettsäure(n) mit der einen oder den mehreren mindestens zweiwertigen Linkergruppe(n) biotechnologisch erfolgt.

Hierin offenbart, jedoch nicht als solches beansprucht, ist ein Hydroxyfettsäurekondensat oder ein Gemisch von Hydroxyfettsäurekondensaten, das durch das Verfahren gemäß der Erfindung erhältlich ist.

Weiterhin betrifft die vorliegende Erfindung ein Polyurethan, das durch Umsetzung einer Zusammensetzung umfassend das Hydroxyfettsäurekondensat oder das Gemisch von Hydroxyfettsäurekondensaten mit einem Diisocyanat, Triisocyanat, Tetraisocyanat oder sonstigen für die Herstellung von Polyurethanen üblichen Polyisocyanaten erhältlich ist.

### Definitionen

Der Begriff "Fettsäure", wie hierin verwendet, bezieht sich vorzugsweise auf aliphatische, lineare, verzweigte oder cyclische (bevorzugt lineare) Alkansäuren mit 6 bis 40 Kohlenstoffatomen, bevorzugt 10 bis 30, stärker bevorzugt 12 bis 24 Kohlenstoffatomen, wobei die Alkansäuren neben der Säuregruppe optional eine oder mehrere weitere funktionelle Gruppen ausgewählt aus Doppelbindungen, Hydroxy, Carboxy und Carbonyl enthält. Bevorzugt bezieht sich der Begriff "Fettsäure" auf aliphatische, lineare Monoalkansäuren mit 6 bis 40 Kohlenstoffatomen, bevorzugt 10 bis 30, stärker bevorzugt 12 bis 24 Kohlenstoffatomen, wobei die Alkansäure neben der Säuregruppe optional eine, zwei, drei oder vier Doppelbindungen und/oder eine oder zwei Hydroxygruppen enthält (z.B. bei einer Fettsäure mit mindestens einer C=C Doppelbindung und/oder einer Hydroxyfettsäure). Der Begriff "Fettsäure mit mindestens einer C=C Doppelbindung" wie hierin verwendet bezieht sich vorzugsweise auf aliphatische, lineare, verzweigte oder cyclische Alkansäuren mit 6 bis 40 Kohlenstoffatomen, bevorzugt 10 bis 30, stärker bevorzugt 12 bis 24 Kohlenstoffatomen, wobei die Alkansäuren neben der Säuregruppe und der mindestens einen C=C Doppelbindung optional eine oder mehrere weitere funktionelle Gruppen, ausgewählt aus Hydroxy, Carboxy und Carbonyl enthält. Bevorzugt bezieht sich der Begriff "Fettsäure mit mindestens einer C=C Doppelbindung" auf aliphatische, lineare Monoalkansäure mit 6 bis 40 Kohlenstoffatomen, bevorzugt 10 bis 30, stärker bevorzugt 12 bis 24 Kohlenstoffatomen, wobei die Alkansäure neben der Säuregruppe eine, zwei, drei oder vier C=C Doppelbindungen (vorzugsweise eine oder zwei, stärker bevorzugt eine) und optional eine oder zwei Hydroxygruppen enthält. Stärker bevorzugt bezieht sich der Begriff "Fettsäure mit mindestens einer C=C Doppelbindung" auf aliphatische, lineare Monoalkansäure mit 6 bis 40 Kohlenstoffatomen, bevorzugt 10 bis 30, stärker bevorzugt 12 bis 24 Kohlenstoffatomen, wobei die Alkansäure neben der Säuregruppe eine oder zwei C=C Doppelbindungen (vorzugsweise eine) enthält.

Der Begriff "Hydroxyfettsäure" wie hierin verwendet, bezieht sich vorzugsweise auf aliphatische, lineare, verzweigte oder cyclische Alkansäuren mit 6 bis 40 Kohlenstoffatomen, bevorzugt 10 bis 30, stärker bevorzugt 12 bis 24 Kohlenstoffatomen, wobei die Alkansäuren neben der Säuregruppe und der mindestens einen Hydroxygruppe optional eine oder mehrere weitere funktionelle Gruppen ausgewählt aus C=C Doppelbindung, Hydroxy, Carboxy und Carbonyl enthält. Bevorzugt bezieht sich der Begriff "Hydroxyfettsäure" auf aliphatische, lineare Monoalkansäure mit 6 bis 40 Kohlenstoffatomen, bevorzugt 10 bis 30, stärker bevorzugt 12 bis 24 Kohlenstoffatomen, wobei die Alkansäure neben der Säuregruppe eine oder zwei Hydroxygruppen (vorzugsweise eine) und optional eine oder zwei C=C Doppelbindungen enthält. Stärker bevorzugt bezieht sich der Begriff "Hydroxyfettsäure" auf aliphatische, lineare Monoalkansäure mit 6 bis 40 Kohlenstoffatomen, bevorzugt 10 bis 30, stärker bevorzugt 12 bis 24 Kohlenstoffatomen, wobei die Alkansäure neben der Säuregruppe eine oder zwei Hydroxygruppen (vorzugsweise eine) enthält.

Soweit nicht anders angegeben bezieht sich der Begriff "Alkansäure" vorzugsweise auf aliphatische sowie lineare, verzweigte oder cyclische (bevorzugt aliphatische lineare) Alkansäuren mit 6 bis 40 Kohlenstoffatomen, die abgesehenen von der einen Carbonsäuregruppe (und optionalen Hydroxy und/oder C=C-Doppelbindungen) nur C-C Einfachbindungen und C-H Bindungen enthält.

Soweit nicht anders angegeben bezieht sich der Begriff "Hydroxyfettsäurekondensat" auf jegliche Verbindungen, die durch Umsetzung der einen oder mehreren Hydroxyfettsäuren mit einer oder mehreren mindestens zweiwertigen Linkergruppen erhalten werden können. Bevorzugt sind dies Verbindungen, in denen eine oder mehrere Hydroxyfettsäuren mit einer oder mehreren Polyolen und/oder (Poly)amino(poly)alkoholen unter Ester- und/oder Amidbildung umgesetzt wurden. Besonders bevorzugt bezieht sich der Begriff "Hydroxyfettsäurekondensat" auf "Hydroxyfettsäureester".

Der Begriff "(Poly)amino(poly)alkohol" bezieht sich auf Verbindungen, bevorzugt aliphatische Verbindungen, die mindestens eine Aminogruppe und mindestens eine Alkoholgruppe enthalten. Die Aminogruppen können primär, sekundär oder tertiär sein, sind jedoch bevorzugt primär. Der Begriff "(Poly)amino(poly)alkohol" bezieht sich bevorzugt auf Verbindungen, bevorzugt aliphatische Verbindungen, die eine, zwei oder drei Aminogruppen und eine, zwei oder drei Alkoholgruppen enthalten.

Der Begriff "Polyamin" bezieht sich auf Verbindungen, bevorzugt aliphatische Verbindungen, die mindestens zwei Aminogruppen, bevorzugt mindestens zwei primäre oder sekundäre Aminogruppen enthalten.

Der Begriff "Polyol" bezieht sich auf Verbindungen, bevorzugt aliphatische Verbindungen, die mindestens zwei Alkoholgruppen, bevorzugt mindestens zwei primäre oder sekundäre Alkoholgruppen, stärker bevorzugt mindestens zwei primäre Alkoholgruppen, enthalten.

Soweit nicht anders angegeben bezieht sich der Begriff "Doppelbindung" auf eine C=C-Doppelbindung.

Ausdrücke enthaltend "ein oder mehrere", oder grammatikalische Abwandlungen davon, mit darauffolgendem Nomen in Pluralform bezeichnen einerseits die Möglichkeit, dass nur ein Exemplar der durch das Nomen bezeichneten Einheit vorhanden ist, andererseits jedoch auch die Möglichkeit, dass zwei oder mehr Exemplare der durch das Nomen bezeichneten Einheit vorhanden sind. Die Verwendung des Nomens im Plural in einem solchen Zusammenhang ist also nicht derart zu interpretieren, dass das Vorhandensein nur eines Exemplars der durch das Nomen bezeichneten Einheit ausgeschlossen ist.

### Ausführliche Beschreibung der Erfindung

### Das Verfahren zur Herstellung eines Hydroxyfettsäurekondensats oder eines Gemischs von Hydroxyfettsäurekondensaten

Die vorliegende Erfindung betrifft ein Verfahren zur Herstellung eines Hydroxyfettsäurekondensats oder eines Gemischs von Hydroxyfettsäurekondensaten, das Verfahren umfassend die Schritte:
- Bereitstellen einer oder mehrerer Fettsäuren mit mindestens einer C=C Doppelbindungsfunktionalität,
- biotechnologische H₂O-Addition mittels einer Hydratase an mindestens eine C=C-Doppelbindungsfunktionalität der einen oder mehreren Fettsäuren, wobei die Doppelbindung keine terminale Doppelbindung ist, unter Erhalt einer oder mehrerer Hydroxyfettsäuren,
- Umsetzung der einen oder mehreren Hydroxyfettsäuren mit einer oder mehreren mindestens zweiwertigen Linkergruppe(n), ausgewählt aus Polyolen, Polyaminen und (Poly)amino(poly)alkoholen, unter Erhalt eines Hydroxyfettsäurekondensats, oder eines Gemischs von Hydroxyfettsäurekondensaten, wobei die Umsetzung der einen oder mehreren Hydroxyfettsäure(n) mit der einen oder den mehreren mindestens zweiwertigen Linkergruppe(n) biotechnologisch erfolgt.

### Die eine oder mehreren Fettsäuren mit mindestens einer C=C Doppelbindungsfunktionalität

Die eine oder mehreren Fettsäuren mit mindestens einer C=C Doppelbindungsfunktionalität sind vorzugsweise ausgewählt aus der Gruppe bestehend aus aliphatischen C₆₋₄₀-Carbonsäuren mit einer, zwei oder drei C=C Doppelbindungsfunktionalitäten, wobei die C₆₋₄₀-Carbonsäuren bevorzugt C₁₀₋₃₀, stärker bevorzugt C₁₂₋₂₄-Carbonsäuren sind.

Es versteht sich, dass die Carbonsäuregruppe (-COOH) bei Fettsäuren bevorzugt eine terminale Carbonsäuregruppe ist. Bevorzugt ist, dass das der Carbonsäuregruppe am nächsten befindliche Kohlenstoffatom zwei Wasserstoffe als Substituenten aufweist.

Es ist bevorzugt, dass die "Fettsäure mit mindestens einer C=C Doppelbindungsfunktionalität" eine einfach oder zweifach ungesättigte Alkansäure ist. Bevorzugt ist die eine bzw. eine der beiden Doppelbindungen in cis-Stellung. Sofern eine zweite Doppelbindung enthalten ist, kann diese entweder in *cis-* oder in trans-, bevorzugt in *cis-,* Stellung sein. Dies gilt auch für eine gegebenenfalls vorhandene dritte und vierte Doppelbindung. Besonders bevorzugt sind alle C=C Doppelbindungen in cis-Stellung.

Es ist bevorzugt, dass die "Fettsäure mit mindestens einer C=C Doppelbindungsfunktionalität" eine Fettsäure mit einer C=C Doppelbindungsfunktionalität ist. Dadurch enthält ein daraus hergestelltes Polyurethan vorzugsweise keine C=C Doppelbindungsfunktionalität mehr und ist somit stabiler gegenüber Oxidation und/oder Zersetzung durch Luftsauerstoff und elektromagnetische Strahlung wie beispielsweise UV-Licht. Unter dem Gesichtspunkt eines niedrigeren Schmelzpunktes und daher verbesserter Verarbeitbarkeit kann es jedoch auch bevorzugt sein, dass die "Fettsäure mit mindestens einer C=C Doppelbindungsfunktionalität" eine Fettsäure mit zwei oder drei C=C Doppelbindungsfunktionalität ist. In diesem Fall kann ein daraus hergestelltes Polyurethan noch ein oder zwei C=C Doppelbindungsfunktionalitäten enthalten, wodurch sich üblicherweise ein niedrigerer Schmelzpunkt der Hydroxyfettsäuren und der daraus durch Umsetzung mit Linkermolekülen erhaltenen Ausgangsprodukte für z.B. Polyurethane erreichen lässt.

Es ist bevorzugt, dass die "Fettsäure mit mindestens einer C=C Doppelbindungsfunktionalität" eine aliphatische lineare Fettsäure (n-Alkensäure) ist.

Es ist weiterhin bevorzugt, dass bei Vorhandensein von zwei oder mehr Doppelbindungen die Doppelbindungen nicht konjugiert sind. Üblicherweise sind daher jeweils zwei Doppelbindungen durch einen oder mehrere sp³ hybridisierte Kohlenstoffe (wie beispielsweise eine -CH₂- Gruppe) voneinander getrennt.

Bevorzugte Beispiele von Fettsäuren mit einer C=C Doppelbindung (auch einfach ungesättigte Fettsäuren genannt) beinhalten insbesondere 3-Hexensäure, 4-Hexensäure, 3-Heptensäure, 4-Heptensäure, 5-Heptensäure, 3-Octensäure, 4-Octensäure, 5-Octensäure, 6-Octensäure, , 3-Nonensäure, 4-Nonensäure, 5-Nonensäure, 6-Nonensäure, 7-Nonensäure, , 3-Decensäure, 4-Decensäure, 5-Decensäure, 6-Decensäure, 7-Decensäure, 8-Decensäure, 3-Undecensäure, 4-Undecensäure, 5-Undecensäure, 6-Undecensäure, 7-Undecensäure, 8-Undecensäure, 9-Undecensäure, , 3-Dodecensäure, 4-Dodecensäure, 5-Dodecensäure, 6-Dodecensäure, 7-Dodecensäure, 8-Dodecensäure, 9-Dodecensäure, 10-Dodecensäure, 3 - Tridecensäure, 4-Tridecensäure, 5-Tridecensäure, 6-Tridecensäure, 7-Tridecensäure, 8-Tridecensäure, 9-Tridecensäure, 10-Tridecensäure, 11-Tridecensäure, 3-Tetradecensäure, 4-Tetradecensäure, 5-Tetradecensäure, 6-Tetradecensäure, 7-Tetradecensäure, 8-Tetradecensäure, 9-Tetradecensäure, 10-Tetradecensäure, 11-Tetradecensäure, 12-Tetradecensäure, 3-Pentadecensäure, 4-Pentadecensäure, 5-Pentadecensäure, 6-Pentadecensäure, 7-Pentadecensäure, 8-Pentadecensäure, 9-Pentadecensäure, 10-Pentadecensäure, 11-Pentadecensäure, 12-Pentadecensäure, 13-Pentadecensäure, 3-Hexadecensäure, 4-Hexadecensäure, 5-Hexadecensäure, 6-Hexadecensäure, 7-Hexadecensäure, 8-Hexadecensäure, 9-Hexadecensäure, 10-Hexadecensäure, 11-Hexadecensäure, 12-Hexadecensäure, 13-Hexadecensäure, 14-Hexadecensäure, 3-Heptadecensäure, 4-Heptadecensäure, 5-Heptadecensäure, 6-Heptadecensäure, 7-Heptadecensäure, 8-Heptadecensäure, 9-Heptadecensäure, 10-Heptadecensäure, 11-Heptadecensäure, 12-Heptadecensäure, 13-Heptadecensäure, 14-Heptadecensäure, 15-Heptadecensäure, 3-Octadecensäure, 4-Octadecensäure, 5-Octadecensäure, 6-Octadecensäure, 7-Octadecensäure, 8-Octadecensäure, 9-Octadecensäure, 10-Octadecensäure, 11-Octadecensäure, 12-Octadecensäure, 13-Octadecensäure, 14-Octadecensäure, 15-Octadecensäure, 16-Octadecensäure, 3-Nonadecensäure, 4-Nonadecensäure, 5-Nonadecensäure, 6-Nonadecensäure, 7-Nonadecensäure, 8-Nonadecensäure, 9-Nonadecensäure, 10-Nonadecensäure, 11-Nonadecensäure, 12-Nonadecensäure, 13-Nonadecensäure, 14-Nonadecensäure, 15-Nonadecensäure, 16-Nonadecensäure, 17-Nonadecensäure, , 3-Icosanensäure, 4-Icosanensäure, 5-Icosanensäure, 6-Icosanensäure, 7-Icosanensäure, 8-Icosanensäure, 9-Icosanensäure, 10-Icosanensäure, 11-Icosanensäure, 12-Icosanensäure, 13-Icosanensäure, 14-Icosanensäure, 15-Icosanensäure, 16-Icosanensäure, 17-Icosanensäure, 18-Icosanensäure, 3-Heneicosensäure, 4-Heneicosensäure, 5-Heneicosensäure, 6-Heneicosensäure, 7-Heneicosensäure, 8-Heneicosensäure, 9-Heneicosensäure, 10-Heneicosensäure, 11-Heneicosensäure, 12-Heneicosensäure, 13-Heneicosensäure, 14-Heneicosensäure, 15-Heneicosensäure, 16-Heneicosensäure, 17-Heneicosensäure, 18-Heneicosensäure, 19-Heneicosensäure, , 3-Docosensäure, 4-Docosensäure, 5-Docosensäure, 6-Docosensäure, 7-Docosensäure, 8-Docosensäure, 9-Docosensäure, 10-Docosensäure, 11-Docosensäure, 12-Docosensäure, 13-Docosensäure, 14-Docosensäure, 15-Docosensäure, 16-Docosensäure, 17-Docosensäure, 18-Docosensäure, 19-Docosensäure, 20-Docosensäure, , 3-Tricosensäure, 4-Tricosensäure, 5-Tricosensäure, 6-Tricosensäure, 7-Tricosensäure, 8-Tricosensäure, 9-Tricosensäure, 10-Tricosensäure, 11-Tricosensäure, 12-Tricosensäure, 13-Tricosensäure, 14-Tricosensäure, 15-Tricosensäure, 16-Tricosensäure, 17-Tricosensäure, 18-Tricosensäure, 19-Tricosensäure, 20-Tricosensäure, 21-Tricosensäure, 3-Tetracosensäure, 4-Tetracosensäure, 5-Tetracosensäure, 6-Tetracosensäure, 7-Tetracosensäure, 8-Tetracosensäure, 9-Tetracosensäure, 10-Tetracosensäure, 11-Tetracosensäure, 12-Tetracosensäure, 13-Tetracosensäure, 14-Tetracosensäure, 15-Tetracosensäure, 16-Tetracosensäure, 17-Tetracosensäure, 18-Tetracosensäure, 19-Tetracosensäure, 20-Tetracosensäure, 21-Tetracosensäure, 22-Tetracosensäure, 3-Pentacosensäure, 4-Pentacosensäure, 5-Pentacosensäure, 6-Pentacosensäure, 7-Pentacosensäure, 8-Pentacosensäure, 9-Pentacosensäure, 10-Pentacosensäure, 11-Pentacosensäure, 12-Pentacosensäure, 13-Pentacosensäure, 14-Pentacosensäure, 15-Pentacosensäure, 16-Pentacosensäure, 17-Pentacosensäure, 18-Pentacosensäure, 19-Pentacosensäure, 20-Pentacosensäure, 21-Pentacosensäure, 22-Pentacosensäure, 23-Pentacosensäure, 3-Hexacosensäure, 4-Hexacosensäure, 5-Hexacosensäure, 6-Hexacosensäure, 7-Hexacosensäure, 8-Hexacosensäure, 9-Hexacosensäure, 10-Hexacosensäure, 11-Hexacosensäure, 12-Hexacosensäure, 13-Hexacosensäure, 14-Hexacosensäure, 15-Hexacosensäure, 16-Hexacosensäure, 17-Hexacosensäure, 18-Hexacosensäure, 19-Hexacosensäure, 20-Hexacosensäure, 21-Hexacosensäure, 22-Hexacosensäure, 23-Hexacosensäure, 24-Hexacosensäure, 3-Heptacosensäure, 4-Heptacosensäure, 5-Heptacosensäure, 6-Heptacosensäure, 7-Heptacosensäure, 8-Heptacosensäure, 9-Heptacosensäure, 10-Heptacosensäure, 11-Heptacosensäure, 12-Heptacosensäure, 13-Heptacosensäure, 14-Heptacosensäure, 15-Heptacosensäure, 16-Heptacosensäure, 17-Heptacosensäure, 18-Heptacosensäure, 19-Heptacosensäure, 20-Heptacosensäure, 21-Heptacosensäure, 22-Heptacosensäure, 23-Heptacosensäure, 24-Heptacosensäure, 25-Heptacosensäure, 3-Octacosensäure, 4-Octacosensäure, 5-Octacosensäure, 6-Octacosensäure, 7-Octacosensäure, 8-Octacosensäure, 9-Octacosensäure, 10-Octacosensäure, 11-Octacosensäure, 12-Octacosensäure, 13-Octacosensäure, 14-Octacosensäure, 15-Octacosensäure, 16-Octacosensäure, 17-Octacosensäure, 18-Octacosensäure, 19-Octacosensäure, 20-Octacosensäure, 21-Octacosensäure, 22-Octacosensäure, 23-Octacosensäure, 24-Octacosensäure, 25-Octacosensäure, 26-Octacosensäure, , 3-Nonacosensäure, 4-Nonacosensäure, 5-Nonacosensäure, 6-Nonacosensäure, 7-Nonacosensäure, 8-Nonacosensäure, 9-Nonacosensäure, 10-Nonacosensäure, 11-Nonacosensäure, 12-Nonacosensäure, 13-Nonacosensäure, 14-Nonacosensäure, 15-Nonacosensäure, 16-Nonacosensäure, 17-Nonacosensäure, 18-Nonacosensäure, 19-Nonacosensäure, 20-Nonacosensäure, 21-Nonacosensäure, 22-Nonacosensäure, 23-Nonacosensäure, 24-Nonacosensäure, 25-Nonacosensäure, 26-Nonacosensäure, 27-Nonacosensäure, , 3 - Triacontensäure, 4-Triacontensäure, 5-Triacontensäure, 6-Triacontensäure, 7-Triacontensäure, 8- Triacontensäure, 9-Triacontensäure, 10-Triacontensäure, 11-Triacontensäure, 12-Triacontensäure, 13-Triacontensäure, 14-Triacontensäure, 15-Triacontensäure, 16-Triacontensäure, 17-Triacontensäure, 18-Triacontensäure, 19-Triacontensäure, 20-Triacontensäure, 21-Triacontensäure, 22-Triacontensäure, 23 - Triacontensäure, 24-Triacontensäure, 25-Triacontensäure, 26-Triacontensäure, 27-Triacontensäure, 28-Triacontensäure.

Diese können jeweils *cis-* oder *trans-* (bevorzugt cis-)-Konfiguration aufweisen. Die obigen Alkensäuren sind bevorzugt lineare Alkensäuren (n-Alkensäuren).

Es versteht sich, dass die einer Doppelbindung in einer ungesättigten (d.h. eine oder mehrere C=C Doppelbindungen enthaltenden) Fettsäuren zugeordnete Zahl die Position der Doppelbindung in der Fettsäure angeben. Entsprechendes gilt für mehrere Zahlen bei mehrfach ungesättigten Fettsäuren. Dabei gibt die Zahl den der Carboxylgruppe am nächsten liegenden Kohlenstoff der Doppelbindung an. Beispielweise ist eine 12-Tetradecensäure eine Fettsäure mit 14 Kohlenstoffatomen, in der sich die Doppelbindung zwischen C12 und C13 befindet (wobei das Kohlenstoffatom C1 das Kohlenstoffatom der Carboxylgruppe ist). Bevorzugte Beispiele von Fettsäuren mit zwei C=C Doppelbindung beinhalten Fettsäuren wie oben aufgeführt, in denen sich eine zusätzliche C=C Doppelbindung, bevorzugt in nichtkonjugierter Stellung, stärker bevorzugt an einer durch eine CH₂-Gruppe abgesetzten Stellung, befindet.

Es ist bevorzugt, dass sich die erste (oder einzige Doppelbindung) zwischen C9 und C10 (oder weiter entfernt von der Carboxylgruppe) befindet und dass die zu hydratisierende Doppelbindung eine *cis*-Konformation aufweist. Die Doppelbindung ist keine terminale Doppelbindung. Entsprechend sind insbesondere bevorzugt die Fettsäuren mit ein, zwei oder drei C=C Doppelbindungen ausgewählt aus 9-Undecensäure, 9-Dodecensäure, 10-Dodecensäure, 9-Tridecensäure, 10-Tridecensäure, 11-Tridecensäure, 9-Tetradecensäure, 10-Tetradecensäure, 11-Tetradecensäure, 12-Tetradecensäure, 9-Pentadecensäure, 10-Pentadecensäure, 11-Pentadecensäure, 12-Pentadecensäure, 13-Pentadecensäure, 9-Hexadecensäure, 10-Hexadecensäure, 11-Hexadecensäure, 12-Hexadecensäure, 13-Hexadecensäure, 14-Hexadecensäure, 9-Heptadecensäure, 10-Heptadecensäure, 11-Heptadecensäure, 12-Heptadecensäure, 13-Heptadecensäure, 14-Heptadecensäure, 15-Heptadecensäure, 9-Octadecensäure, 10-Octadecensäure, 11-Octadecensäure, 12-Octadecensäure, 13-Octadecensäure, 14-Octadecensäure, 15-Octadecensäure, 16-Octadecensäure, 9-Nonadecensäure, 10-Nonadecensäure, 11-Nonadecensäure, 12-Nonadecensäure, 13-Nonadecensäure, 14-Nonadecensäure, 15-Nonadecensäure, 16-Nonadecensäure, 17-Nonadecensäure, 9-Icosanensäure, 10-Icosanensäure, 11-Icosanensäure, 12-Icosanensäure, 13-Icosanensäure, 14-Icosanensäure, 15-Icosanensäure, 16-Icosanensäure, 17-Icosanensäure, 18-Icosanensäure, 9-Heneicosensäure, 10-Heneicosensäure, 11-Heneicosensäure, 12-Heneicosensäure, 13-Heneicosensäure, 14-Heneicosensäure, 15-Heneicosensäure, 16-Heneicosensäure, 17-Heneicosensäure, 18-Heneicosensäure, 19-Heneicosensäure, 9-Docosensäure, 10-Docosensäure, 11-Docosensäure, 12-Docosensäure, 13-Docosensäure, 14-Docosensäure, 15-Docosensäure, 16-Docosensäure, 17-Docosensäure, 18-Docosensäure, 19-Docosensäure, 20-Docosensäure, 9-Tricosensäure, 10-Tricosensäure, 11-Tricosensäure, 12-Tricosensäure, 13-Tricosensäure, 14-Tricosensäure, 15-Tricosensäure, 16-Tricosensäure, 17-Tricosensäure, 18-Tricosensäure, 19-Tricosensäure, 20-Tricosensäure, 21-Tricosensäure, 9-Tetracosensäure, 10-Tetracosensäure, 11-Tetracosensäure, 12-Tetracosensäure, 13-Tetracosensäure, 14-Tetracosensäure, 15-Tetracosensäure, 16-Tetracosensäure, 17-Tetracosensäure, 18-Tetracosensäure, 19-Tetracosensäure, 20-Tetracosensäure, 21-Tetracosensäure, 22-Tetracosensäure, 9-Pentacosensäure, 10-Pentacosensäure, 11-Pentacosensäure, 12-Pentacosensäure, 13-Pentacosensäure, 14-Pentacosensäure, 15-Pentacosensäure, 16-Pentacosensäure, 17-Pentacosensäure, 18-Pentacosensäure, 19-Pentacosensäure, 20-Pentacosensäure, 21-Pentacosensäure, 22-Pentacosensäure, 23-Pentacosensäure, 9-Hexacosensäure, 10-Hexacosensäure, 11-Hexacosensäure, 12-Hexacosensäure, 13-Hexacosensäure, 14-Hexacosensäure, 15-Hexacosensäure, 16-Hexacosensäure, 17-Hexacosensäure, 18-Hexacosensäure, 19-Hexacosensäure, 20-Hexacosensäure, 21-Hexacosensäure, 22-Hexacosensäure, 23-Hexacosensäure, 24-Hexacosensäure, 9-Heptacosensäure, 10-Heptacosensäure, 11-Heptacosensäure, 12-Heptacosensäure, 13-Heptacosensäure, 14-Heptacosensäure, 15-Heptacosensäure, 16-Heptacosensäure, 17-Heptacosensäure, 18-Heptacosensäure, 19-Heptacosensäure, 20-Heptacosensäure, 21-Heptacosensäure, 22-Heptacosensäure, 23-Heptacosensäure, 24-Heptacosensäure, 25-Heptacosensäure, 9-Octacosensäure, 10-Octacosensäure, 11-Octacosensäure, 12-Octacosensäure, 13-Octacosensäure, 14-Octacosensäure, 15-Octacosensäure, 16-Octacosensäure, 17-Octacosensäure, 18-Octacosensäure, 19-Octacosensäure, 20-Octacosensäure, 21-Octacosensäure, 22-Octacosensäure, 23-Octacosensäure, 24-Octacosensäure, 25-Octacosensäure, 26-Octacosensäure, 9-Nonacosensäure, 10-Nonacosensäure, 11-Nonacosensäure, 12-Nonacosensäure, 13-Nonacosensäure, 14-Nonacosensäure, 15-Nonacosensäure, 16-Nonacosensäure, 17-Nonacosensäure, 18-Nonacosensäure, 19-Nonacosensäure, 20-Nonacosensäure, 21-Nonacosensäure, 22-Nonacosensäure, 23-Nonacosensäure, 24-Nonacosensäure, 25-Nonacosensäure, 26-Nonacosensäure, 27-Nonacosensäure, 9-Triacontensäure, 10-Triacontensäure, 11-Triacontensäure, 12-Triacontensäure, 13-Triacontensäure, 14-Triacontensäure, 15-Triacontensäure, 16-Triacontensäure, 17-Triacontensäure, 18-Triacontensäure, 19-Triacontensäure, 20-Triacontensäure, 21-Triacontensäure, 22- Triacontensäure, 23-Triacontensäure, 24-Triacontensäure, 25-Triacontensäure, 26-Triacontensäure, 27-Triacontensäure, 28-Triacontensäure, Tetradeca-9,12-diensäure, Pentadeca-9,12-diensäure, Pentadeca-10,13-diensäure, Hexadeca-9,12-diensäure, Hexadeca-10,13-diensäure, Hexadeca-11,14-diensäure, Heptadeca-9,12-diensäure, Heptadeca-10,13-diensäure, Heptadeca-11,14-diensäure, Heptadeca-12,15-diensäure, Octadeca-9,12-diensäure, Octadeca-10,13-diensäure, Octadeca-11,14-diensäure, Octadeca-12,15-diensäure, Octadeca-13,16-diensäure, Nonadeca-9,12-diensäure, Nonadeca-10,13-diensäure, Nonadeca-11,14-diensäure, Nonadeca-12,15-diensäure, Nonadeca-13,16-diensäure, Nonadeca-14,17-diensäure, Icosana-9,12-diensäure, Icosana-10,13-diensäure, Icosana-11,14-diensäure, Icosana-12,15-diensäure, Icosana-13,16-diensäure, Icosana-14,17-diensäure, Icosana-15,18-diensäure, Heneicosa-9,12-diensäure, Heneicosa-10,13-diensäure, Heneicosa-11,14-diensäure, Heneicosa-12,15-diensäure, Heneicosa-13,16-diensäure, Heneicosa-14,17-diensäure, Heneicosa-15,18-diensäure, Heneicosa-16,19-diensäure, Docosa-9,12-diensäure, Docosa-10,13-diensäure, Docosa-11,14-diensäure, Docosa-12,15-diensäure, Docosa-13,16-diensäure, Docosa-14,17-diensäure, Docosa-15,18-diensäure, Docosa-16,19-diensäure, Docosa-17,20-diensäure, Tricosa-9,12-diensäure, Tricosa-10,13-diensäure, Tricosa-11,14-diensäure, Tricosa-12,15-diensäure, Tricosa-13,16-diensäure, Tricosa-14,17-diensäure, Tricosa-15,18-diensäure, Tricosa-16,19-diensäure, Tricosa-17,20-diensäure, Tricosa-18,21-diensäure, Tetracosa-9,12-diensäure, Tetracosa-10,13-diensäure, Tetracosa-11,14-diensäure, Tetracosa-12,15-diensäure, Tetracosa-13,16-diensäure, Tetracosa-14,17-diensäure, Tetracosa-15,18-diensäure, Tetracosa-16,19-diensäure, Tetracosa-17,20-diensäure, Tetracosa-18,21-diensäure, Tetracosa-19,22-diensäure, Pentacosa-9,12-diensäure, Pentacosa-10,13-diensäure, Pentacosa-11,14-diensäure, Pentacosa-12,15-diensäure, Pentacosa-13,16-diensäure, Pentacosa-14,17-diensäure, Pentacosa-15,18-diensäure, Pentacosa-16,19-diensäure, Pentacosa-17,20-diensäure, Pentacosa-18,21-diensäure, Pentacosa-19,22-diensäure, Pentacosa-20,23-diensäure, Hexacosa-9,12-diensäure, Hexacosa-10,13-diensäure, Hexacosa-11,14-diensäure, Hexacosa-12,15-diensäure, Hexacosa-13,16-diensäure, Hexacosa-14,17-diensäure, Hexacosa-15,18-diensäure, Hexacosa-16,19-diensäure, Hexacosa-17,20-diensäure, Hexacosa-18,21-diensäure, Hexacosa-19,22-diensäure, Hexacosa-20,23-diensäure, Hexacosa-21,24-diensäure, Heptacosa-9,12-diensäure, Heptacosa-10,13-diensäure, Heptacosa-11,14-diensäure, Heptacosa-12,15-diensäure, Heptacosa-13,16-diensäure, Heptacosa-14,17-diensäure, Heptacosa-15,18-diensäure, Heptacosa-16,19-diensäure, Heptacosa-17,20-diensäure, Heptacosa-18,21-diensäure, Heptacosa-19,22-diensäure, Heptacosa-20,23-diensäure, Heptacosa-21,24-diensäure, Heptacosa-22,25-diensäure, Octacosa-9,12-diensäure, Octacosa-10,13-diensäure, Octacosa-11,14-diensäure, Octacosa-12,15-diensäure, Octacosa-13,16-diensäure, Octacosa-14,17-diensäure, Octacosa-15,18-diensäure, Octacosa-16,19-diensäure, Octacosa-17,20-diensäure, Octacosa-18,21-diensäure, Octacosa-19,22-diensäure, Octacosa-20,23-diensäure, Octacosa-21,24-diensäure, Octacosa-22,25-diensäure, Octacosa-23,26-diensäure, Nonacosa-9,12-diensäure, Nonacosa-10,13-diensäure, Nonacosa-11,14-diensäure, Nonacosa-12,15-diensäure, Nonacosa-13,16-diensäure, Nonacosa-14,17-diensäure, Nonacosa-15,18-diensäure, Nonacosa-16,19-diensäure, Nonacosa-17,20-diensäure, Nonacosa-18,21-diensäure, Nonacosa-19,22-diensäure, Nonacosa-20,23-diensäure, Nonacosa-21,24-diensäure, Nonacosa-22,25-diensäure, Nonacosa-23,26-diensäure, Nonacosa-24,27-diensäure, Triaconta-9,12-diensäure, Triaconta-10,13-diensäure, Triaconta-11,14-diensäure, Triaconta-12,15-diensäure, Triaconta-13,16-diensäure, Triaconta-14,17-diensäure, Triaconta-15,18-diensäure, Triaconta-16,19-diensäure, Triaconta-17,20-diensäure, Triaconta-18,21-diensäure, Triaconta-19,22-diensäure, Triaconta-20,23-diensäure, Triaconta-21,24-diensäure, Triaconta-22,25-diensäure, Triaconta-23,26-diensäure, Triaconta-24,27-diensäure, Triaconta-25,28-diensäure, Heptadeca-9,12,15-triensäure, Octadeca-9,12,15-triensäure, Octadeca-10,13,16-triensäure, Nonadeca-9,12,15-triensäure, Nonadeca-10,13,16-triensäure, Nonadeca-11,14,17-triensäure, Icosana-9,12,15-triensäure, Icosana-10,13,16-triensäure, Icosana-11,14,17-triensäure, Icosana-12,15,18-triensäure, Heneicosa-9,12,15-triensäure, Heneicosa-10,13,16-triensäure, Heneicosa-11,14,17-triensäure, Heneicosa-12,15,18-triensäure, Heneicosa-13,16,19-triensäure, Docosa-9,12,15-triensäure, Docosa-10,13,16-triensäure, Docosa-11,14,17-triensäure, Docosa-12,15,18-triensäure, Docosa-13,16,19-triensäure, Docosa-14,17,20-triensäure, Tricosa-9,12,15-triensäure, Tricosa-10,13,16-triensäure, Tricosa-11,14,17-triensäure, Tricosa-12,15,18-triensäure, Tricosa-13,16,19-triensäure, Tricosa-14,17,20-triensäure, Tricosa-15,18,21-triensäure, Tetracosa-9,12,15-triensäure, Tetracosa-10,13,16-triensäure, Tetracosa-11,14,17-triensäure, Tetracosa-12,15,18-triensäure, Tetracosa-13,16,19-triensäure, Tetracosa-14,17,20-triensäure, Tetracosa-15,18,21-triensäure, Tetracosa-16,19,22-triensäure, Pentacosa-9,12,15-triensäure, Pentacosa-10,13,16-triensäure, Pentacosa-11,14,17-triensäure, Pentacosa-12,15,18-triensäure, Pentacosa-13,16,19-triensäure, Pentacosa-14,17,20-triensäure, Pentacosa-15,18,21-triensäure, Pentacosa-16,19,22-triensäure, Pentacosa-17,20,23-triensäure, Hexacosa-9,12,15-triensäure, Hexacosa-10,13,16-triensäure, Hexacosa-11,14,17-triensäure, Hexacosa-12,15,18-triensäure, Hexacosa-13,16,19-triensäure, Hexacosa-14,17,20-triensäure, Hexacosa-15,18,21-triensäure, Hexacosa-16,19,22-triensäure, Hexacosa-17,20,23-triensäure, Hexacosa-18,21,24-triensäure, Heptacosa-9,12,15-triensäure, Heptacosa-10,13,16-triensäure, Heptacosa-11,14,17-triensäure, Heptacosa-12,15,18-triensäure, Heptacosa-13,16,19-triensäure, Heptacosa-14,17,20-triensäure, Heptacosa-15,18,21-triensäure, Heptacosa-16,19,22-triensäure, Heptacosa-17,20,23-triensäure, Heptacosa-18,21,24-triensäure, Heptacosa-19,22,25-triensäure, Octacosa-9,12,15-triensäure, Octacosa-10,13,16-triensäure, Octacosa-11,14,17-triensäure, Octacosa-12,15,18-triensäure, Octacosa-13,16,19-triensäure, Octacosa-14,17,20-triensäure, Octacosa-15,18,21-triensäure, Octacosa-16,19,22-triensäure, Octacosa-17,20,23-triensäure, Octacosa-18,21,24-triensäure, Octacosa-19,22,25-triensäure, Octacosa-20,23,26-triensäure, Nonacosa-9,12,15-triensäure, Nonacosa-10,13,16-triensäure, Nonacosa-11,14,17-triensäure, Nonacosa-12,15,18-triensäure, Nonacosa-13,16,19-triensäure, Nonacosa-14,17,20-triensäure, Nonacosa-15,18,21-triensäure, Nonacosa-16,19,22-triensäure, Nonacosa-17,20,23-triensäure, Nonacosa-18,21,24-triensäure, Nonacosa-19,22,25-triensäure, Nonacosa-20,23,26-triensäure, Nonacosa-21,24,27-triensäure, Triaconta-9,12,15-triensäure, Triaconta-10,13,16-triensäure, Triaconta-11,14,17-triensäure, Triaconta-12,15,18-triensäure, Triaconta-13,16,19-triensäure, Triaconta-14,17,20-triensäure, Triaconta-15,18,21-triensäure, Triaconta-16,19,22-triensäure, Triaconta-17,20,23-triensäure, Triaconta-18,21,24-triensäure, Triaconta-19,22,25-triensäure, Triaconta-20,23,26-triensäure, Triaconta-21,24,27-triensäure und Triaconta-22,25,28-triensäure. Dabei hat vorzugsweise zumindest die der Carbonsäurefunktion am nächsten befindliche C=C-Doppelbindung, bevorzugt aber alle C=C-Doppelbindungen, *cis*-Konformation.

Zur Herstellung von besonders oxidationsbeständigen und UV-stabilen Produkten sind unter diesen stärker bevorzugt die Fettsäuren mit einer C=C Doppelbindung ausgewählt aus 9-Undecensäure, 9-Dodecensäure, 10-Dodecensäure, 9-Tridecensäure, 10-Tridecensäure, 11-Tridecensäure, 9-Tetradecensäure, 10-Tetradecensäure, 11-Tetradecensäure, 12-Tetradecensäure, 9-Pentadecensäure, 10-Pentadecensäure, 11-Pentadecensäure, 12-Pentadecensäure, 13-Pentadecensäure, 9-Hexadecensäure, 10-Hexadecensäure, 11-Hexadecensäure, 12-Hexadecensäure, 13-Hexadecensäure, 14-Hexadecensäure, 9-Heptadecensäure, 10-Heptadecensäure, 11-Heptadecensäure, 12-Heptadecensäure, 13-Heptadecensäure, 14-Heptadecensäure, 15-Heptadecensäure, 9-Octadecensäure, 10-Octadecensäure, 11-Octadecensäure, 12-Octadecensäure, 13-Octadecensäure, 14-Octadecensäure, 15-Octadecensäure, 16-Octadecensäure, 9-Nonadecensäure, 10-Nonadecensäure, 11-Nonadecensäure, 12-Nonadecensäure, 13-Nonadecensäure, 14-Nonadecensäure, 15-Nonadecensäure, 16-Nonadecensäure, 17-Nonadecensäure, 9-Icosanensäure, 10-Icosanensäure, 11-Icosanensäure, 12-Icosanensäure, 13-Icosanensäure, 14-Icosanensäure, 15-Icosanensäure, 16-Icosanensäure, 17-Icosanensäure, 18-Icosanensäure, 9-Heneicosensäure, 10-Heneicosensäure, 11-Heneicosensäure, 12-Heneicosensäure, 13-Heneicosensäure, 14-Heneicosensäure, 15-Heneicosensäure, 16-Heneicosensäure, 17-Heneicosensäure, 18-Heneicosensäure, 19-Heneicosensäure, 9-Docosensäure, 10-Docosensäure, 11-Docosensäure, 12-Docosensäure, 13-Docosensäure, 14-Docosensäure, 15-Docosensäure, 16-Docosensäure, 17-Docosensäure, 18-Docosensäure, 19-Docosensäure, 20-Docosensäure, 9-Tricosensäure, 10-Tricosensäure, 11-Tricosensäure, 12-Tricosensäure, 13-Tricosensäure, 14-Tricosensäure, 15-Tricosensäure, 16-Tricosensäure, 17-Tricosensäure, 18-Tricosensäure, 19-Tricosensäure, 20-Tricosensäure, 21-Tricosensäure, 9-Tetracosensäure, 10-Tetracosensäure, 11-Tetracosensäure, 12-Tetracosensäure, 13-Tetracosensäure, 14-Tetracosensäure, 15-Tetracosensäure, 16-Tetracosensäure, 17-Tetracosensäure, 18-Tetracosensäure, 19-Tetracosensäure, 20-Tetracosensäure, 21-Tetracosensäure, 22-Tetracosensäure, 9-Pentacosensäure, 10-Pentacosensäure, 11-Pentacosensäure, 12-Pentacosensäure, 13-Pentacosensäure, 14-Pentacosensäure, 15-Pentacosensäure, 16-Pentacosensäure, 17-Pentacosensäure, 18-Pentacosensäure, 19-Pentacosensäure, 20-Pentacosensäure, 21-Pentacosensäure, 22-Pentacosensäure, 23-Pentacosensäure, 9-Hexacosensäure, 10-Hexacosensäure, 11-Hexacosensäure, 12-Hexacosensäure, 13-Hexacosensäure, 14-Hexacosensäure, 15-Hexacosensäure, 16-Hexacosensäure, 17-Hexacosensäure, 18-Hexacosensäure, 19-Hexacosensäure, 20-Hexacosensäure, 21-Hexacosensäure, 22-Hexacosensäure, 23-Hexacosensäure, 24-Hexacosensäure, 9-Heptacosensäure, 10-Heptacosensäure, 11-Heptacosensäure, 12-Heptacosensäure, 13-Heptacosensäure, 14-Heptacosensäure, 15-Heptacosensäure, 16-Heptacosensäure, 17-Heptacosensäure, 18-Heptacosensäure, 19-Heptacosensäure, 20-Heptacosensäure, 21-Heptacosensäure, 22-Heptacosensäure, 23-Heptacosensäure, 24-Heptacosensäure, 25-Heptacosensäure, 9-Octacosensäure, 10-Octacosensäure, 11-Octacosensäure, 12-Octacosensäure, 13-Octacosensäure, 14-Octacosensäure, 15-Octacosensäure, 16-Octacosensäure, 17-Octacosensäure, 18-Octacosensäure, 19-Octacosensäure, 20-Octacosensäure, 21-Octacosensäure, 22-Octacosensäure, 23-Octacosensäure, 24-Octacosensäure, 25-Octacosensäure, 26-Octacosensäure, 9-Nonacosensäure, 10-Nonacosensäure, 11-Nonacosensäure, 12-Nonacosensäure, 13-Nonacosensäure, 14-Nonacosensäure, 15-Nonacosensäure, 16-Nonacosensäure, 17-Nonacosensäure, 18-Nonacosensäure, 19-Nonacosensäure, 20-Nonacosensäure, 21-Nonacosensäure, 22-Nonacosensäure, 23-Nonacosensäure, 24-Nonacosensäure, 25-Nonacosensäure, 26-Nonacosensäure, 27-Nonacosensäure, 9-Triacontensäure, 10-Triacontensäure, 11-Triacontensäure, 12- Triacontensäure, 13-Triacontensäure, 14-Triacontensäure, 15-Triacontensäure, 16-Triacontensäure, 17-Triacontensäure, 18-Triacontensäure, 19-Triacontensäure, 20- Triacontensäure, 21-Triacontensäure, 22-Triacontensäure, 23-Triacontensäure, 24-Triacontensäure, 25-Triacontensäure, 26-Triacontensäure, 27-Triacontensäure und 28-Triacontensäure. Dabei hat vorzugsweise die C=C-Doppelbindung cis-Konformation.

Um die Verarbeitbarkeit der Hydroxyfettsäuren zu verbessern, insbesondere um möglichst bei Raumtemperatur flüssige Hydroxyfettsäuren zu erhalten, ist es bevorzugt, dass die Fettsäuren mit mindestens einer C=C Doppelbindung zwei oder drei C=C Doppelbindungen enthalten. Diese können bevorzugt ausgewählt werden aus Fettsäuren mit zwei C=C Doppelbindungen, wie Tetradeca-9,12-diensäure, Pentadeca-9,12-diensäure, Pentadeca-10,13-diensäure, Hexadeca-9,12-diensäure, Hexadeca-10,13-diensäure, Hexadeca-11,14-diensäure, Heptadeca-9,12-diensäure, Heptadeca-10,13-diensäure, Heptadeca-11,14-diensäure, Heptadeca-12,15-diensäure, Octadeca-9,12-diensäure, Octadeca-10,13-diensäure, Octadeca-11,14-diensäure, Octadeca-12,15-diensäure, Octadeca-13,16-diensäure, Nonadeca-9,12-diensäure, Nonadeca-10,13-diensäure, Nonadeca-11,14-diensäure, Nonadeca-12,15-diensäure, Nonadeca-13,16-diensäure, Nonadeca-14,17-diensäure, Icosana-9,12-diensäure, Icosana-10,13-diensäure, Icosana-11,14-diensäure, Icosana-12,15-diensäure, Icosana-13,16-diensäure, Icosana-14,17-diensäure, Icosana-15,18-diensäure, Heneicosa-9,12-diensäure, Heneicosa-10,13-diensäure, Heneicosa-11,14-diensäure, Heneicosa-12,15-diensäure, Heneicosa-13,16-diensäure, Heneicosa-14,17-diensäure, Heneicosa-15,18-diensäure, Heneicosa-16,19-diensäure, Docosa-9,12-diensäure, Docosa-10,13-diensäure, Docosa-11,14-diensäure, Docosa-12,15-diensäure, Docosa-13,16-diensäure, Docosa-14,17-diensäure, Docosa-15,18-diensäure, Docosa-16,19-diensäure, Docosa-17,20-diensäure, Tricosa-9,12-diensäure, Tricosa-10,13-diensäure, Tricosa-11,14-diensäure, Tricosa-12,15-diensäure, Tricosa-13,16-diensäure, Tricosa-14,17-diensäure, Tricosa-15,18-diensäure, Tricosa-16,19-diensäure, Tricosa-17,20-diensäure, Tricosa-18,21-diensäure, Tetracosa-9,12-diensäure, Tetracosa-10,13-diensäure, Tetracosa-11,14-diensäure, Tetracosa-12,15-diensäure, Tetracosa-13,16-diensäure, Tetracosa-14,17-diensäure, Tetracosa-15,18-diensäure, Tetracosa-16,19-diensäure, Tetracosa-17,20-diensäure, Tetracosa-18,21-diensäure und Tetracosa-19,22-diensäure.

Fettsäuren mit drei C=C Doppelbindungen können bevorzugt ausgewählt werden aus Heptadeca-9,12,15-triensäure, Octadeca-9,12,15-triensäure, Octadeca-10,13,16-triensäure, Nonadeca-9,12,15-triensäure, Nonadeca-10,13,16-triensäure, Nonadeca-11,14,17-triensäure, Icosana-9,12,15-triensäure, Icosana-10,13,16-triensäure, Icosana-11,14,17-triensäure, Icosana-12,15,18-triensäure, Heneicosa-9,12,15-triensäure, Heneicosa-10,13,16-triensäure, Heneicosa-11,14,17-triensäure, Heneicosa-12,15,18-triensäure, Heneicosa-13,16,19-triensäure, Docosa-9,12,15-triensäure, Docosa-10,13,16-triensäure, Docosa-11, 14, 17-triensäure, Docosa-12,15,18-triensäure, Docosa-13,16,19-triensäure, Docosa-14,17,20-triensäure, Tricosa-9,12,15-triensäure, Tricosa-10,13,16-triensäure, Tricosa-11,14,17-triensäure, Tricosa-12,15,18-triensäure, Tricosa-13,16,19-triensäure, Tricosa-14,17,20-triensäure, Tricosa-15,18,21-triensäure, Tetracosa-9,12,15-triensäure, Tetracosa-10,13,16-triensäure, Tetracosa-11,14,17-triensäure, Tetracosa-12,15,18-triensäure, Tetracosa-13,16,19-triensäure, Tetracosa-14,17,20-triensäure, Tetracosa-15,18,21-triensäure und Tetracosa-16,19,22-triensäure.

Unter diesen sind wiederum besonders bevorzugt die Alkensäuren, die 24 oder weniger, bevorzugt 22 oder weniger, Kohlenstoffatome enthalten, wobei sich vorzugsweise die (einzige bzw. der Carboxylgruppe am weitesten entfernteste) Doppelbindung an C18=C19 oder näher an der Carboxylgruppe befindet (jedoch vorzugsweise nicht näher an der Carboxylgruppe als C9=C10).

Es ist bevorzugt, dass die "Fettsäure mit mindestens einer C=C Doppelbindungsfunktionalität" eine Fettsäure C14 bis C20 Fettsäure, weiter bevorzugt C14, C16, C18 oder C20 Fettsäure (insbesondere bevorzugt C14, C16 oder C18 Fettsäure), ist.

Stärker bevorzugt sind die eine oder mehreren Fettsäuren mit mindestens einer C=C Doppelbindungsfunktionalität ausgewählt aus der Gruppe bestehend aus einfach ungesättigten Fettsäuren, bevorzugt ausgewählt aus Undecylensäure, Myristoleinsäure, Palmitoleinsäure, Margaroleinsäure, Petroselinsäure, Ölsäure, Elaidinsäure, Vaccensäure, Gadoleinsäure, Gondosäure, Cetoleinsäure, Erucasäure, Vernolsäure, cis-5-Eicosensäure, Brassidinsäure und Nervonsäure, und/oder aus der Gruppe bestehend aus mehrfach ungesättigten Fettsäuren, bevorzugt ausgewählt aus Linolsäure, Linolensäure, Calendulasäure, Punicinsäure, Elaeostearinsäure, Arachidonsäure, Timnodonsäure, Clupanodonsäure, Lesquerolsäure, Licansäure und Cervonsäure ausgewählt.

Unter diesen sind die einfach ungesättigten Fettsäuren, bevorzugt ausgewählt aus Undecylensäure, Myristoleinsäure, Palmitoleinsäure, Margaroleinsäure, Petroselinsäure, Ölsäure, Elaidinsäure, Vaccensäure, Gadoleinsäure, Gondosäure, Cetoleinsäure, Erucasäure, Vernolsäure, cis-5-Eicosensäure, Brassidinsäure und Nervonsäure, bevorzugt.

Noch stärker bevorzugt sind die eine oder mehreren Fettsäuren mit mindestens einer C=C Doppelbindungsfunktionalität ausgewählt aus Ölsäure, Linolsäure, Linolensäure und Palmitoleinsäure, wobei die eine oder mehreren Fettsäuren bevorzugt ausgewählt sind aus Ölsäure, Linolsäure und Linolensäure, und stärker bevorzugt Ölsäure ist.

Es ist bevorzugt, dass die einen oder mehreren Fettsäuren mit mindestens einer C=C Doppelbindungsfunktionalität ein Gemisch von mindestens 2, vorzugsweise mindestens 3, stärker bevorzugt mindestens 4, noch stärker bevorzugt mindestens 5, Fettsäuren mit mindestens einer C=C Doppelbindungsfunktionalität sind.

Es ist weiter bevorzugt, dass die einen oder mehreren Fettsäuren mit mindestens einer C=C Doppelbindungsfunktionalität zu mindestens 70 mol-%, bevorzugt mindestens 85 mol-%, stärker bevorzugt mindestens 90 % eines oder mehrere, bevorzugt zwei oder drei, ausgewählt aus Ölsäure, Linolsäure und Linolensäure enthalten. Solche Fettsäuregemische können z.B. aus sog. "high oleic sunflower oil" durch Hydrolyse erhalten werden. Es ist bevorzugt, dass das Gemisch, bezogen auf alle Fettsäuren mit mindestens einer C=C Doppelbindungsfunktionalität, mindestens 40 mol-%, mindestens 50 mol-% oder sogar mindestens 60 mol-%, jedoch 90 mol-% oder weniger, bevorzugt 80 mol-% oder weniger an Ölsäure enthält.

Die in der vorliegenden Erfindung verwendeten ungesättigten Fettsäuren können insbesondere durch Hydrolyse von Triglyceriden (Fette, Öle, etc.) erhalten werden. Dabei kommt beispielsweise die alkalische Verseifung oder Hydrolyse von Trigylceriden sowie die enzymatische Hydrolyse der Estergruppen zum Einsatz. Bevorzugte Triglyceride im Sinne der vorliegenden Erfindung sind ausgewählt aus der Gruppe bestehend aus Rapsöl, Sonnenblumenöl, Sojabohnenöl, Olivenöl, Rizinusöl, Färberdistelöl, Crambeöl, Leinöl, Jatrophaöl, Palmöl, Seetieröle, Sesamöl, Rindertalg, Erdnussöl, Schmalz, verbrauchtem Frittieröl, verbrauchtem Hydraulik-Öl und verbrauchtem Industrie-Öl. Es versteht sich, dass diese Öle naturbedingt üblicherweise ein Gemisch von Triglyceriden darstellen. Dabei sind Triglyceride mit einem hohen Anteil an Ölsäure bevorzugt. Diese Öle bzw. Triglyceride können einzeln oder als Gemisch eingesetzt werden. Um ein gewünschtes Fettsäureverhältnis einzustellen, kann die Verwendung eines Gemischs von zwei oder mehr Ölen erwünscht sein. Sofern nicht ausdrücklich anders angegeben, sind alle hierin angegebenen ungesättigten Fettsäuren bevorzugt ungesättigte Fettsäuren, in denen mindestens eine Doppelbindung, bevorzugt alle Doppelbindungen cis-Konformation aufweisen.

### Die biotechnologische H₂O-Addition

Die biotechnologische H₂O-Addition mittels einer Hydratase im Sinne der vorliegenden Erfindung bezieht sich auf die Addition von H₂O an eine nicht-aromatische, nicht-terminale, sowie bevorzugt nicht konjugierte, C=C Doppelbindung unter Vermittlung (insbesondere Katalyse) eines Proteins.

Durch die biotechnologische H₂O-Addition kann beispielweise eine partielle Struktureinheit 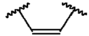 in eine partielle Struktureinheit 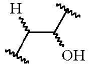 umgewandelt werden, wobei sich die Carboxygruppe der Fettsäure links oder rechts von den gezeigten partiellen Struktureinheiten befinden kann. Es ist jedoch bevorzugt, dass sich die Carboxgruppe der Fettsäure links von den gezeigten partiellen Struktureinheiten befindet. Mit anderen Worten, es ist bevorzugt, dass die C=C Doppelbindung derart hydratisiert wird, dass sich die durch die Hydratisierung gebildete Hydroxygruppe an dem Kohlenstoffatom befindet, das zuvor dasjenige der beiden zu der C=C Doppelbindung gehörenden Kohlenstoffatome war, das der Carboxygruppe weiter entfernt war. Wenn die Fettsäure mit mindestens einer C=C Doppelbindungsfunktionalität beispielweise eine 9-Octadecensäure war, ist das Produkt vorzugsweise eine 10-Hydroxyoctadecansäure.

Die dabei gebildete alkoholische OH-Gruppe kann, in Abhängigkeit von dem verwendeten biotechnologischen H₂O-Additionsmechanmismus und der Konfiguration der Doppelbindung im Substrat, die Stereokonfiguration R oder S aufweisen oder ein beliebiges Gemisch von R und S sein. Es ist bevorzugt, dass die biotechnologische H₂O-Addition zu 80 mol-% oder mehr zu nur R oder nur S Konfiguration führt. Stärker bevorzugt führt die biotechnologische H₂O-Addition zu 50 mol-% oder mehr, 70 mol-% oder mehr, 90 mol-% oder mehr oder sogar 95 mol-% oder mehr, zu nur R Konfiguration. Durch eine derartige Selektivität der biotechnologischen H₂O-Addition können einheitlichere Hydroxyfettsäuren als Produkte erhalten werden, die wiederum die Herstellung von einheitlicheren und klarer definierten Polyurethanprodukten ermöglichen.

Der Begriff "Protein" wie hierin verwendet bezieht sich auf Polypeptide (d.h. Verbindungen enthaltend mindestens 9 peptidische Bindungen, wobei eine peptidische Bindung eine Amidbindung zwischen der Carboxygruppe einer Aminosäure und der Aminogruppe des α-Kohlenstoffatoms (α-C-Atom) einer weiteren Aminosäure beschreibt) mit mindestens 10 Aminosäuren, bevorzugt mindestens 50 Aminosäuren, bevorzugt mindestens 100 Aminosäuren, noch stärker bevorzugt mindestens 200 Aminosäuren. Diese Polypeptide umfassen bevorzugt nicht mehr als 50 000 Aminosäuren, stärker bevorzugt nicht mehr als 20 000 Aminosäuren, noch stärker bevorzugt nicht mehr als 10 000 Aminosäuren, noch stärker bevorzugt nicht mehr als 5 000 Aminosäuren, am stärksten bevorzugt nicht mehr als 1 000 Aminosäuren.

Die biotechnologische H₂O-Addition im Sinne der vorliegenden Erfindung bezieht sich auf die Addition von H₂O an eine nicht-aromatische, sowie bevorzugt nicht konjugierte, C=C Doppelbindung unter Vermittlung einer Hydratase. Die Eignung von solchen Enzymen für die Hydratisierung von ungesättigten Fettsäuren unter milden Reaktionsbedingungen und unter Einsatz von Wasser als sowohl Reagenz als auch zugleich kostengünstiges und umweltfreundliches Lösungsmittel ist bekannt. Solche für die Wasser-Anlagerung an ungesättigte Fettsäuren geeigneten Hydratasen sind als sogenannte Fettsäurehydratasen bekannt und der Stand der Wissenschaft und Technik auf diesem Gebiet wurde beispielsweise kürzlich in einem ausführlichen Übersichtsartikel beschrieben (J. Löwe, H. Gröger, "Fatty Acid Hydratases: Versatile Catalysts to Access Hydroxy Fatty Acids in Efficient Syntheses of Industrial Interest", Catalysts 2020, 10, 287; DOI: 10.3390/catal10030287).

Vorliegend wird eine biotechnologische H₂O-Addition verwendet, da diese einheitlichere Produkte liefern kann und unter milderen Reaktionsbedingungen verläuft, als dies bei einer typischen chemischen Hydratisierung der Fall ist. Beispielsweise kann eine biotechnologische H₂O-Addition zu einer Hydratisierung einer bestimmten Doppelbindung (sofern mehrere Doppelbindungen pro Fettsäuremolekül vorhanden sind) führen. Außerdem kann, wie oben beschrieben, die biotechnologische H₂O-Addition zu einer stereoselektiven Hydratisierung führen. Diese Effekte führen dazu, dass die Reinheit der erhaltenen Hydroxyfettsäuren üblicherweise sehr hoch ist und insbesondere die Mengen an Regioisomeren und Stereoisomeren gering gehalten werden kann. Dadurch ist es möglich, Polyurethane mit sehr genau einstellbaren und einheitlichen Eigenschaften zu erhalten.

Aus der Literatur ist bekannt, dass viele biobasierte Rohstoffe eine unzureichend einheitliche und/oder unpassende OH-Funktionalität pro Molekül aufweisen. Solche Rohstoffe können nur anteilig oder sehr unregelmäßig in ein Polyurethan-Netzwerk eingebunden werden. Hinzu kommt, dass typische Begleitstoffe je nach Ursprung des Rohstoffes stark variieren und nachteilig bei industrieller Fertigung und Verarbeitung Anlagen beschädigen und/oder verunreinigen können. Enzymatische Modifikationen bieten hier die Möglichkeit, einen Rohstoff bei niedriger Temperatur, bevorzugt mit Wasser als Lösungsmittel und mit geringen bis gar keinen Verunreinigungen, gleichmäßig einzustellen.

Problematisch ist bei den etablierten, chemischen Synthesewegen, dass eine Hydratisierung ungesättigter C=C-Gruppen stets mit zusätzlichen, chemischen oder chemo-enzymatischen Maßnahmen, also stets mit etablierten Maßnahmen in Kombination, vorgesehen ist. So wird für die Herstellung pflanzlich basierter Polyole in 'Plant Oils as Platform Chemicals for Polyurethane Synthesis' (Biomacromolecules 2010, 11, 2825-2835) vorgeschlagen, eine Säuregruppe chemisch einzuführen, mit NaBH₄ zu reduzieren und anschließend verbliebene Doppelbindungen mit einem Platinkatalysator zu hydrieren. Die Produkte solcher Synthesen liegen dadurch stets als Gemisch verschiedener Isomere und Produkte vor. Selbst nach chemischer Fraktionierung enthalten die Fraktionen dann immer noch unregelmäßige Anteile an Stereoisomeren. Die so zugänglichen, polymeren Produkte sind in ihrer Volumenstruktur unregelmäßig und die Volumeneigenschaften schwanken. Präzise Erweichungspunkte, hoch gleichmäßige Festigkeiten oder auch elektrotechnisch (wobei Polyurethane gerne als sog. Elektrovergussmassen, bei denen die hervorragenden elektrischen Isolationseigenschaften des Polyurethans ausgenutzt werden, eingesetzt werden) einheitliche und stabile Volumeneigenschaften sind bei Synthesewegen mit etablierten, technisch-chemischen Synthesestufen nur sehr aufwändig und mit sehr hohen Kosten zugänglich.

Diese Probleme werden durch die vorliegende Erfindung mittels der biotechnologischen H₂O-Addition gelöst. Dem Fachmann sind eine Vielzahl an Enzymen aus einer großen Bandbreite Gram-negativer und Gram-positiver Bakterien (u.a. *Bifidobacterium animalis, B. breve, Chryseobacterium gleum, Desulfomicrobium baculatum, E. meningoseptica, Gemella morbillorum, Lactobacillus acidophilus, L. hammesii, L. plantarum, L. reuteri, L. rhamnosus, L. spicheri, Lysinibacillus fusiformis, Macrococcus caseolyticus, Rhodococcus erythropolis, Stenotrophomonas maltophilia, S. nitritireducens, Streptococcus pyogenes)* bekannt, die rekombinant exprimiert, charakterisiert und als Ölsäure-Hydratasen (OAH), Linolsäure-Hydratasen (LAH) oder allgemeine Fettsäure-Hydratasen identifiziert wurden (aufgeführt auf Seite 25 der Dissertation von Rebecca M. Demming an der Fakultät 4 der Universität Stuttgart vom 22.03.2018 mit dem Titel "Enzymatische Hydratisierung kurzkettiger Fettsäuren und Alkene")

In Jahre 2016 wurde darüber hinaus eine Hydratase-Datenbank (Hydratase Engineering Database; URL: https://hyed.biocatnet.de/) mit 2046 Sequenzen bekannter oder aber putativer Hydratasen erstellt. Bei der Mehrzahl dieser Hydratasen handelt es sich um bakterielle Proteine, es wurden aber auch 103 bzw. 24 Sequenzen aus Pilzen und Archaeen aufgenommen. Folglich sind dem Fachmann eine Vielzahl von Hydratasen bekannt, mit Hilfe derer sich einfach oder auch mehrfach ungesättigte Fettsäuren einfach oder auch mehrfach hydratisieren lassen.

Im Verfahren der vorliegenden Erfindung wird bevorzugt eine Ölsäurehydratase des Organismus Stenotrophomonas nitritireducens verwendet, welche von Kang et al. in Biotechnology and Bioengineering, 2017, 114, 74-82 beschrieben wurde. Diese kann als Rohextrakt, in Form von ganzen Zellen, als Lyophilisat oder in immobilisierter Form eingesetzt werden. Die Enzymimmobilisierung ist ein vielseitiges Werkzeug zur Optimierung der Katalysatorbedingungen. Dabei sind Vorteile, wie die Steigerung der Stabilität, die Wiederverwendbarkeit des geträgerten Enzyms sowie eine bessere Abtrennung von Enzym und gewünschtem Produkt zu nennen. Die bekannten Verfahren zur Enzymimmobilisierung sind die Bindung an einen Träger, der Einschluss oder die Vernetzung von Enzymen (R. A. Sheldon and S. van Pelt, Enzyme immobilisation in biocatalysis: why, what and how, Chemical Society Reviews, 2013, 42, 6223-6235). Stärker bevorzugt wird die Sn-Ohy Hydratase des Organismus *Stenotrophomonas nitritireducens* verwendet. In den erfindungsrelevanten Beispielen wurde dieser Biokatalysator für die Hydratisierung von Ölsäure als Substratkomponente eingesetzt. Zugleich ist der Fachmann frei in der Wahl der Hydratase und wird vorzugsweise diejenigen Hydratasen einsetzten, die für das jeweilige Substrat am besten geeignet sind. In der Literatur sind hierzu ausführliche Beschreibungen zum Substratspektrum der jeweiligen Hydratasen vorhanden. So ist beispielsweise neben der Ölsäure auch Linolensäure als gut umsetzbares Substrat bekannt, wobei hierfür auch eine andere, passgenau auf Linolensäure zugeschnittene Fettsäurehydratase zum Einsatz kommen kann. Eine Übersicht von solchen Fettsäurehydratasen ist beispielsweise in folgendem Übersichtsartikel gegeben: J. Löwe, H. Gröger, "Fatty Acid Hydratases: Versatile Catalysts to Access Hydroxy Fatty Acids in Efficient Syntheses of Industrial Interest", Catalysts 2020, 10, 287; DOI: 10.3390/catal10030287.

Bevorzugt wird die Hydratisierung mittels einer Hydratase durchgeführt, wobei die Konzentration der einen oder mehreren Fettsäuren mit mindestens einer C=C-Doppelbindung in einem Bereich gehalten wird, wobei der Minimalwert des Bereichs ausgewählt ist aus der Gruppe bestehend aus 100 g/L, 150 g/L, 200 g/L, und der optionale Maximalwert bevorzugt ausgewählt ist aus der Gruppe bestehend aus 250 g/L, 280 g/L, 300 g/L. Besonders bevorzugt ist die Konzentration der einen oder mehreren Fettsäuren mit mindestens einer C=C-Doppelbindung in einem Bereich von 200 bis 300 g/L.

Beispiele geeigneter Puffer für die Hydratisierung von Fettsäuren sind:
Citrat Puffer (50 mM; pH 3,0- 4,0),
Natriumsuccinat Puffer (50 mM; pH 4,0-6,0),
KPB Puffer (50 mM; pH 5,0-8,0),
Tris-HCl Puffer (50 mM; pH 7,0-9,0).

Bevorzugte Puffer zur Hydratisierung von Fettsäuren im bevorzugten pH Bereich von 5,5-7,5 wurden beispielsweise beschrieben von Kim et al., Appl. Microbiol. Biotechnol. 2012, 95 (4), 929-937; Takeuchi et al., J. Biosci. Bioeng. 2015, 119 (6), 636-641; Park et al., J. Biotechnol. 2015, 208, 1-10). Diese beinhalten insbesondere:
Phosphat-Puffer (Park et al., J. Biotechnol. 2015, 208, 1-10),
TRIS-HCl (Jo et al., J. Agric. Food Chem. 2014, 62 (28), 6736-6745),
Citrat-Puffer (Park et al., J. Biotechnol. 2015, 208, 1-10; Kang et al., Biotechnol. Bioeng. 2017, 114 (1), 74-82),
HEPES-Puffer (Kang et al., Appl. Environ. Microbiol. 2017, 83 (9)), und PIPES-Puffer (Seo et al., Appl. Microbiol. Biotechnol. 2013, 97 (20), 8987-8995).

In allen oben genannten Puffersystemen ist eine Salzkonzentration der Puffer von 50 mM oder 100 mM bevorzugt.

In bevorzugter Ausführungsform wird in der Synthese als Enzym ein rekombinantes Enzym verwendet, das mindestens ein rekombinantes Enzym ausgewählt aus der Gruppe bestehend aus Hydratase aus *Streptococcus pyogenes;* Hydratase aus *Elizabethkingia Meningoseptica*; Proteine mit einem Aufbau homolog zu *Elizabethkingia meningoseptica* ausgewählt aus mindestens einer Quelle der Gruppe von Quellen bestehend aus *Psychroflexus torquis, Rhodopseudomonas palustris, Lactobacillus plantarum, Lactobacillus acidophilus, Stenotrophomonas nitritireducens, Lactobacillus rhamnosus, Lysinibacillus fusiformis, Macrococcus caseolyticus, Stenotrophomas maltophilia, Bifidobacterium breve, Bifidobacterium animalis* ist.

Beispiele geeigneter Wirtsorganismen als Expressionsstämme sind bakterielle Systeme wie *Escherichia coli, Corynebacterium glutamicum* oder eukaryotische Systeme/Hefen wie *Saccharomyces cerevisiae* oder *Pichia pastoris.*

In bevorzugter Ausführungsform der Synthese wird mindestens eines der Enzyme vorbereitend mindestens einer Modifikation unterworfen. Die mindestens eine Modifikation ist ausgewählt aus der Gruppe von Maßnahmen bestehend aus Molekulargewichtsvergrößerung des Enzyms; Immobilisierung des Enzyms; Immobilisierung des Enzyms auf einem leicht von flüssigen Phasen abtrennbaren, partikulären Träger; Immobilisierung des Enzyms auf einem partikulären Träger umfassend superparamagnetischen Nanoteilchen, welche eine magnetische Separation der Träger ermöglichen; Immobilisierung des Enzyms auf einem polymeren Träger auf Acrylatbasis; Immobilisierung des Enzyms in einer lonentauscher-Träger-Matrix; Immobilisierung des Enzyms auf einem polypartikulären, vernetzten Kompositwerkstoffträger; Immobilisierung des Enzyms auf einem Schichtsilikat; Immobilisierung von Zellen herstellend oder enthaltend das Enzym auf einem Träger; Immobilisierung des Enzyms auf einem biobasierten Silikatgerüst; Hydrophilierung des Enzyms mit einer hydrophilen Ankergruppe; Oleophilierung des Enzyms mit einer oleophilen Ankergruppe; gentechnische Veränderung durch Einfügen mindestens einer Mutation; gentechnische Veränderung durch Einfügen mindestens einer Sequenz einer hydrophoben Gruppe; gentechnische Veränderung durch Einfügen mindestens einer Sequenz einer hydrophilen Gruppe; gentechnische Veränderung der Teilsequenz welche die aktive Tasche eines Enzyms definiert; Fusion der Gensequenz des Enzyms mit einer transportaktiven, Membran-permeierenden Eiweiß-Gensequenz; Fusion der Gensequenz des Enzyms mit einer weiteren Enzym-Gensequenz; Fusion der Gensequenz einer Hydratase mit einer Lipase-Gensequenz; Fusion der Gensequenz eines Enzyms mit einer stabilisierenden Eiweiß-Gensequenz aus einem extremophilen Wirtsorganismus unter Steigerung der thermischen Widerstandfähigkeit, Scherbeständigkeit, Salzbeständigkeit und pH-Beständigkeit; Steigerung der thermischen Widerstandsfähigkeit eines Enzyms; Fusion der Gensequenz eines Enzyms mit einer amphiphilen, Löslichkeit in verschiedenen Medien steigernden, Eiweiß-Gensequenz; randomisierte Mutation, Screening und Selektion; selektive Mutation des polaren Abschnitts der aktiven Tasche; selektive Mutation des hydrophoben Abschnitts der aktiven Tasche; selektive Mutation der endständig-hydrophilen Abschnitte des Enzyms; Micellisierung des Enzyms in einer Kaskade von Lipid-Schichten mit jeweiligem Wechsel von Lipophilie zu Hydrophilie der nach außen weisenden Endgruppen, wobei bevorzugt nach außen weisende, transportfördernde Ankergruppen eine Permeation durch Phasengrenzen unterstützen; Expression und/oder Bereitstellung des Enzyms in permeabilisierten Zellen; Expression und/oder Bereitstellung des Enzyms in periplasmatisch exprimierenden Zellen.

### Die eine oder mehreren Hydroxyfettsäuren

Die eine oder mehreren Hydroxyfettsäuren, die durch das erfindungsgemäße Verfahren erhalten werden, unterscheiden sich von der einen oder den mehreren Fettsäuren mit mindestens einer C=C Doppelbindung bevorzugt nur darin, dass mindestens eine (bevorzugt eine) der mindestens einen C=C Doppelbindung in den Fettsäuren hydratisiert ist, d.h. dass eine partielle Struktureinheit 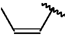 in eine partielle Struktureinheit 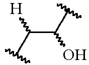 umgewandelt wurde. Sofern mehrere C=C Doppelbindungen pro Fettsäuremolekül vorhanden sind, ist bevorzugt, dass lediglich eine der C=C Doppelbindungen pro Fettsäuremolekül hydratisiert ist.

Bevorzugte Beispiele von Hydroxyfettsäuren beinhalten insbesondere 3-Hydroxy-hexansäure, 4-Hydroxy-hexansäure, 5-Hydroxy-hexansäure, 6-Hydroxy-hexansäure, 3-Hydroxy-heptansäure, 4-Hydroxy-heptansäure, 5-Hydroxy-heptansäure, 6-Hydroxy-heptansäure, 7-Hydroxy-heptansäure, 3-Hydroxy-octansäure, 4-Hydroxy-octansäure, 5-Hydroxy-octansäure, 6-Hydroxy-octansäure, 7-Hydroxy-octansäure, 8-Hydroxy-octansäure, 3-Hydroxy-nonansäure, 4-Hydroxy-nonansäure, 5-Hydroxy-nonansäure, 6-Hydroxy-nonansäure, 7-Hydroxy-nonansäure, 8-Hydroxy-nonansäure, 9-Hydroxy-nonansäure, 3-Hydroxy-decansäure, 4-Hydroxy-decansäure, 5-Hydroxy-decansäure, 6-Hydroxy-decansäure, 7-Hydroxy-decansäure, 8-Hydroxy-decansäure, 9-Hydroxy-decansäure, 10-Hydroxy-decansäure, 3-Hydroxy-undecansäure, 4-Hydroxy-undecansäure, 5-Hydroxy-undecansäure, 6-Hydroxy-undecansäure, 7-Hydroxy-undecansäure, 8-Hydroxy-undecansäure, 9-Hydroxy-undecansäure, 10-Hydroxy-undecansäure, 11-Hydroxy-undecansäure, 3-Hydroxy-dodecansäure, 4-Hydroxy-dodecansäure, 5-Hydroxy-dodecansäure, 6-Hydroxy-dodecansäure, 7-Hydroxy-dodecansäure, 8-Hydroxy-dodecansäure, 9-Hydroxy-dodecansäure, 10-Hydroxy-dodecansäure, 11-Hydroxy-dodecansäure, 12-Hydroxy-dodecansäure, 3-Hydroxy-tridecansäure, 4-Hydroxy-tridecansäure, 5-Hydroxy-tridecansäure, 6-Hydroxy-tridecansäure, 7-Hydroxy-tridecansäure, 8-Hydroxy-tridecansäure, 9-Hydroxy-tridecansäure, 10-Hydroxy-tridecansäure, 11-Hydroxy-tridecansäure, 12-Hydroxy-tridecansäure, 13-Hydroxy-tridecansäure, 3-Hydroxy-tetradecansäure, 4-Hydroxy-tetradecansäure, 5-Hydroxy-tetradecansäure, 6-Hydroxy-tetradecansäure, 7-Hydroxy-tetradecansäure, 8-Hydroxy-tetradecansäure, 9-Hydroxy-tetradecansäure, 10-Hydroxy-tetradecansäure, 11-Hydroxy-tetradecansäure, 12-Hydroxy-tetradecansäure, 13-Hydroxy-tetradecansäure, 14-Hydroxy-tetradecansäure, 3-Hydroxy-pentadecansäure, 4-Hydroxy-pentadecansäure, 5-Hydroxy-pentadecansäure, 6-Hydroxy-pentadecansäure, 7-Hydroxy-pentadecansäure, 8-Hydroxy-pentadecansäure, 9-Hydroxy-pentadecansäure, 10-Hydroxy-pentadecansäure, 11-Hydroxy-pentadecansäure, 12-Hydroxy-pentadecansäure, 13-Hydroxy-pentadecansäure, 14-Hydroxy-pentadecansäure, 15-Hydroxy-pentadecansäure, 3-Hydroxy-hexadecansäure, 4-Hydroxy-hexadecansäure, 5-Hydroxy-hexadecansäure, 6-Hydroxy-hexadecansäure, 7-Hydroxy-hexadecansäure, 8-Hydroxy-hexadecansäure, 9-Hydroxy-hexadecansäure, 10-Hydroxy-hexadecansäure, 11-Hydroxy-hexadecansäure, 12-Hydroxy-hexadecansäure, 13-Hydroxy-hexadecansäure, 14-Hydroxy-hexadecansäure, 15-Hydroxy-hexadecansäure, 16-Hydroxy-hexadecansäure, 3-Hydroxy-heptadecansäure, 4-Hydroxy-heptadecansäure, 5-Hydroxy-heptadecansäure, 6-Hydroxy-heptadecansäure, 7-Hydroxy-heptadecansäure, 8-Hydroxy-heptadecansäure, 9-Hydroxy-heptadecansäure, 10-Hydroxy-heptadecansäure, 11-Hydroxy-heptadecansäure, 12-Hydroxy-heptadecansäure, 13-Hydroxy-heptadecansäure, 14-Hydroxy-heptadecansäure, 15-Hydroxy-heptadecansäure, 16-Hydroxy-heptadecansäure, 17-Hydroxy-heptadecansäure, 3-Hydroxy-octadecansäure, 4-Hydroxy-octadecansäure, 5-Hydroxyoctadecansäure, 6-Hydroxy-octadecansäure, 7-Hydroxy-octadecansäure, 8-Hydroxyoctadecansäure, 9-Hydroxy-octadecansäure, 10-Hydroxy-octadecansäure, 11-Hydroxyoctadecansäure, 12-Hydroxy-octadecansäure, 13-Hydroxy-octadecansäure, 14-Hydroxyoctadecansäure, 15-Hydroxy-octadecansäure, 16-Hydroxy-octadecansäure, 17-Hydroxyoctadecansäure, 18-Hydroxy-octadecansäure, 3-Hydroxy-nonadecansäure, 4-Hydroxy-nonadecansäure, 5-Hydroxy-nonadecansäure, 6-Hydroxy-nonadecansäure, 7-Hydroxy-nonadecansäure, 8-Hydroxy-nonadecansäure, 9-Hydroxy-nonadecansäure, 10-Hydroxy-nonadecansäure, 11-Hydroxy-nonadecansäure, 12-Hydroxy-nonadecansäure, 13-Hydroxy-nonadecansäure, 14-Hydroxy-nonadecansäure, 15-Hydroxy-nonadecansäure, 16-Hydroxy-nonadecansäure, 17-Hydroxy-nonadecansäure, 18-Hydroxy-nonadecansäure, 19-Hydroxy-nonadecansäure, 3-Hydroxy-icosanansäure, 4-Hydroxy-icosanansäure, 5-Hydroxy-icosanansäure, 6-Hydroxy-icosanansäure, 7-Hydroxy-icosanansäure, 8-Hydroxy-icosanansäure, 9-Hydroxy-icosanansäure, 10-Hydroxy-icosanansäure, 11-Hydroxy-icosanansäure, 12-Hydroxy-icosanansäure, 13-Hydroxy-icosanansäure, 14-Hydroxy-icosanansäure, 15-Hydroxy-icosanansäure, 16-Hydroxy-icosanansäure, 17-Hydroxy-icosanansäure, 18-Hydroxy-icosanansäure, 19-Hydroxy-icosanansäure, 20-Hydroxy-icosanansäure, 3-Hydroxy-heneicosansäure, 4-Hydroxy-heneicosansäure, 5-Hydroxy-heneicosansäure, 6-Hydroxy-heneicosansäure, 7-Hydroxy-heneicosansäure, 8-Hydroxy-heneicosansäure, 9-Hydroxy-heneicosansäure, 10-Hydroxy-heneicosansäure, 11-Hydroxy-heneicosansäure, 12-Hydroxy-heneicosansäure, 13-Hydroxy-heneicosansäure, 14-Hydroxy-heneicosansäure, 15-Hydroxy-heneicosansäure, 16-Hydroxy-heneicosansäure, 17-Hydroxy-heneicosansäure, 18-Hydroxy-heneicosansäure, 19-Hydroxy-heneicosansäure, 20-Hydroxy-heneicosansäure, 21-Hydroxy-heneicosansäure, 3-Hydroxy-docosansäure, 4-Hydroxy-docosansäure, 5-Hydroxy-docosansäure, 6-Hydroxy-docosansäure, 7-Hydroxy-docosansäure, 8-Hydroxy-docosansäure, 9-Hydroxy-docosansäure, 10-Hydroxy-docosansäure, 11-Hydroxy-docosansäure, 12-Hydroxy-docosansäure, 13-Hydroxy-docosansäure, 14-Hydroxy-docosansäure, 15-Hydroxy-docosansäure, 16-Hydroxy-docosansäure, 17-Hydroxy-docosansäure, 18-Hydroxy-docosansäure, 19-Hydroxy-docosansäure, 20-Hydroxy-docosansäure, 21-Hydroxy-docosansäure, 22-Hydroxy-docosansäure, 3-Hydroxy-tricosansäure, 4-Hydroxy-tricosansäure, 5-Hydroxy-tricosansäure, 6-Hydroxy-tricosansäure, 7-Hydroxy-tricosansäure, 8-Hydroxy-tricosansäure, 9-Hydroxy-tricosansäure, 10-Hydroxy-tricosansäure, 11-Hydroxy-tricosansäure, 12-Hydroxy-tricosansäure, 13-Hydroxy-tricosansäure, 14-Hydroxy-tricosansäure, 15-Hydroxy-tricosansäure, 16-Hydroxy-tricosansäure, 17-Hydroxy-tricosansäure, 18-Hydroxy-tricosansäure, 19-Hydroxy-tricosansäure, 20-Hydroxy-tricosansäure, 21-Hydroxy-tricosansäure, 22-Hydroxy-tricosansäure, 23-Hydroxy-tricosansäure, 3-Hydroxy-tetracosansäure, 4-Hydroxy-tetracosansäure, 5-Hydroxy-tetracosansäure, 6-Hydroxy-tetracosansäure, 7-Hydroxy-tetracosansäure, 8-Hydroxy-tetracosansäure, 9-Hydroxy-tetracosansäure, 10-Hydroxy-tetracosansäure, 11-Hydroxy-tetracosansäure, 12-Hydroxy-tetracosansäure, 13-Hydroxy-tetracosansäure, 14-Hydroxy-tetracosansäure, 15-Hydroxy-tetracosansäure, 16-Hydroxy-tetracosansäure, 17-Hydroxy-tetracosansäure, 18-Hydroxy-tetracosansäure, 19-Hydroxy-tetracosansäure, 20-Hydroxy-tetracosansäure, 21-Hydroxy-tetracosansäure, 22-Hydroxy-tetracosansäure, 23-Hydroxy-tetracosansäure, 24-Hydroxy-tetracosansäure, 3-Hydroxy-pentacosansäure, 4-Hydroxy-pentacosansäure, 5-Hydroxy-pentacosansäure, 6-Hydroxy-pentacosansäure, 7-Hydroxy-pentacosansäure, 8-Hydroxy-pentacosansäure, 9-Hydroxy-pentacosansäure, 10-Hydroxy-pentacosansäure, 11-Hydroxy-pentacosansäure, 12-Hydroxy-pentacosansäure, 13-Hydroxy-pentacosansäure, 14-Hydroxy-pentacosansäure, 15-Hydroxy-pentacosansäure, 16-Hydroxy-pentacosansäure, 17-Hydroxy-pentacosansäure, 18-Hydroxy-pentacosansäure, 19-Hydroxy-pentacosansäure, 20-Hydroxy-pentacosansäure, 21-Hydroxy-pentacosansäure, 22-Hydroxy-pentacosansäure, 23-Hydroxy-pentacosansäure, 24-Hydroxy-pentacosansäure, 25-Hydroxy-pentacosansäure, 3-Hydroxy-hexacosansäure, 4-Hydroxy-hexacosansäure, 5-Hydroxy-hexacosansäure, 6-Hydroxy-hexacosansäure, 7-Hydroxy-hexacosansäure, 8-Hydroxy-hexacosansäure, 9-Hydroxy-hexacosansäure, 10-Hydroxy-hexacosansäure, 11-Hydroxy-hexacosansäure, 12-Hydroxy-hexacosansäure, 13-Hydroxy-hexacosansäure, 14-Hydroxy-hexacosansäure, 15-Hydroxy-hexacosansäure, 16-Hydroxy-hexacosansäure, 17-Hydroxy-hexacosansäure, 18-Hydroxy-hexacosansäure, 19-Hydroxy-hexacosansäure, 20-Hydroxy-hexacosansäure, 21-Hydroxy-hexacosansäure, 22-Hydroxy-hexacosansäure, 23-Hydroxy-hexacosansäure, 24-Hydroxy-hexacosansäure, 25-Hydroxy-hexacosansäure, 26-Hydroxy-hexacosansäure, 3-Hydroxy-heptacosansäure, 4-Hydroxy-heptacosansäure, 5-Hydroxy-heptacosansäure, 6-Hydroxy-heptacosansäure, 7-Hydroxy-heptacosansäure, 8-Hydroxy-heptacosansäure, 9-Hydroxy-heptacosansäure, 10-Hydroxy-heptacosansäure, 11-Hydroxy-heptacosansäure, 12-Hydroxy-heptacosansäure, 13-Hydroxy-heptacosansäure, 14-Hydroxy-heptacosansäure, 15-Hydroxy-heptacosansäure, 16-Hydroxy-heptacosansäure, 17-Hydroxy-heptacosansäure, 18-Hydroxy-heptacosansäure, 19-Hydroxy-heptacosansäure, 20-Hydroxy-heptacosansäure, 21-Hydroxy-heptacosansäure, 22-Hydroxy-heptacosansäure, 23-Hydroxy-heptacosansäure, 24-Hydroxy-heptacosansäure, 25-Hydroxy-heptacosansäure, 26-Hydroxy-heptacosansäure, 27-Hydroxy-heptacosansäure, 3-Hydroxy-octacosansäure, 4-Hydroxy-octacosansäure, 5-Hydroxy-octacosansäure, 6-Hydroxy-octacosansäure, 7-Hydroxy-octacosansäure, 8-Hydroxy-octacosansäure, 9-Hydroxy-octacosansäure, 10-Hydroxy-octacosansäure, 11-Hydroxy-octacosansäure, 12-Hydroxy-octacosansäure, 13-Hydroxy-octacosansäure, 14-Hydroxy-octacosansäure, 15-Hydroxy-octacosansäure, 16-Hydroxy-octacosansäure, 17-Hydroxy-octacosansäure, 18-Hydroxy-octacosansäure, 19-Hydroxy-octacosansäure, 20-Hydroxy-octacosansäure, 21-Hydroxy-octacosansäure, 22-Hydroxy-octacosansäure, 23-Hydroxy-octacosansäure, 24-Hydroxy-octacosansäure, 25-Hydroxy-octacosansäure, 26-Hydroxy-octacosansäure, 27-Hydroxy-octacosansäure, 28-Hydroxy-octacosansäure, 3-Hydroxy-nonacosansäure, 4-Hydroxy-nonacosansäure, 5-Hydroxy-nonacosansäure, 6-Hydroxy-nonacosansäure, 7-Hydroxy-nonacosansäure, 8-Hydroxy-nonacosansäure, 9-Hydroxy-nonacosansäure, 10-Hydroxy-nonacosansäure, 11-Hydroxy-nonacosansäure, 12-Hydroxy-nonacosansäure, 13-Hydroxy-nonacosansäure, 14-Hydroxy-nonacosansäure, 15-Hydroxy-nonacosansäure, 16-Hydroxy-nonacosansäure, 17-Hydroxy-nonacosansäure, 18-Hydroxy-nonacosansäure, 19-Hydroxy-nonacosansäure, 20-Hydroxy-nonacosansäure, 21-Hydroxy-nonacosansäure, 22-Hydroxy-nonacosansäure, 23-Hydroxy-nonacosansäure, 24-Hydroxy-nonacosansäure, 25-Hydroxy-nonacosansäure, 26-Hydroxy-nonacosansäure, 27-Hydroxy-nonacosansäure, 28-Hydroxy-nonacosansäure, 29-Hydroxy-nonacosansäure, 3-Hydroxy-triacontansäure, 4-Hydroxy-triacontansäure, 5-Hydroxy-triacontansäure, 6-Hydroxy-triacontansäure, 7-Hydroxy-triacontansäure, 8-Hydroxy-triacontansäure, 9-Hydroxy-triacontansäure, 10-Hydroxy-triacontansäure, 11-Hydroxy-triacontansäure, 12-Hydroxy-triacontansäure, 13-Hydroxy-triacontansäure, 14-Hydroxy-triacontansäure, 15-Hydroxy-triacontansäure, 16-Hydroxy-triacontansäure, 17-Hydroxy-triacontansäure, 18-Hydroxy-triacontansäure, 19-Hydroxy-triacontansäure, 20-Hydroxy-triacontansäure, 21-Hydroxy-triacontansäure, 22-Hydroxy-triacontansäure, 23-Hydroxy-triacontansäure, 24-Hydroxy-triacontansäure, 25-Hydroxy-triacontansäure, 26-Hydroxy-triacontansäure, 27-Hydroxy-triacontansäure, 28-Hydroxy-triacontansäure, 29-Hydroxy-triacontansäure, 30-Hydroxy-triacontansäure. In diesen kann die Hydroxygruppe jeweils *R* oder *S* Konfiguration aufweisen, bevorzugt *R*. Die obigen Alkansäuren sind bevorzugt lineare Alkansäuren (n-Alkansäuren).

Bevorzugte Beispiele von Fettsäuren mit zwei Hydroxygruppen beinhalten Hydroxyfettsäuren wie oben aufgeführt, in denen sich eine zusätzliche Hydroxygruppe befindet.

Es ist bevorzugt, dass sich die erste (oder einzige Hydroxygruppe) an C10 (oder weiter entfernt von der Carboxylgruppe) befindet. Die der Carboxylgruppe am weitesten entfernte Hydroxygruppe ist keine terminale Hydroxygruppe. Entsprechend sind insbesondere bevorzugt die Hydroxyfettsäuren mit einer oder zwei Hydroxygruppe(n) ausgewählt aus 10-Hydroxy-undecansäure, 10-Hydroxy-dodecansäure, 11-Hydroxy-dodecansäure, 10-Hydroxy-tridecansäure, 11-Hydroxy-tridecansäure, 12-Hydroxy-tridecansäure, 10-Hydroxy-tetradecansäure, 11-Hydroxy-tetradecansäure, 12-Hydroxy-tetradecansäure, 13-Hydroxy-tetradecansäure, 10-Hydroxy-pentadecansäure, 11-Hydroxy-pentadecansäure, 12-Hydroxy-pentadecansäure, 13-Hydroxy-pentadecansäure, 14-Hydroxy-pentadecansäure, 10-Hydroxy-hexadecansäure, 11-Hydroxy-hexadecansäure, 12-Hydroxy-hexadecansäure, 13-Hydroxy-hexadecansäure, 14-Hydroxy-hexadecansäure, 15-Hydroxy-hexadecansäure, 10-Hydroxy-heptadecansäure, 11-Hydroxy-heptadecansäure, 12-Hydroxy-heptadecansäure, 13-Hydroxy-heptadecansäure, 14-Hydroxy-heptadecansäure, 15-Hydroxy-heptadecansäure, 16-Hydroxy-heptadecansäure, 10-Hydroxy-octadecansäure, 11-Hydroxy-octadecansäure, 12-Hydroxyoctadecansäure, 13-Hydroxy-octadecansäure, 14-Hydroxy-octadecansäure, 15-Hydroxyoctadecansäure, 16-Hydroxy-octadecansäure, 17-Hydroxy-octadecansäure, 10-Hydroxy-nonadecansäure, 11-Hydroxy-nonadecansäure, 12-Hydroxy-nonadecansäure, 13-Hydroxy-nonadecansäure, 14-Hydroxy-nonadecansäure, 15-Hydroxy-nonadecansäure, 16-Hydroxy-nonadecansäure, 17-Hydroxy-nonadecansäure, 18-Hydroxy-nonadecansäure, 10-Hydroxy-icosanansäure, 11-Hydroxy-icosanansäure, 12-Hydroxy-icosanansäure, 13-Hydroxy-icosanansäure, 14-Hydroxy-icosanansäure, 15-Hydroxy-icosanansäure, 16-Hydroxy-icosanansäure, 17-Hydroxy-icosanansäure, 18-Hydroxy-icosanansäure, 19-Hydroxy-icosanansäure, 10-Hydroxy-heneicosansäure, 11-Hydroxy-heneicosansäure, 12-Hydroxy-heneicosansäure, 13-Hydroxy-heneicosansäure, 14-Hydroxy-heneicosansäure, 15-Hydroxy-heneicosansäure, 16-Hydroxy-heneicosansäure, 17-Hydroxy-heneicosansäure, 18-Hydroxy-heneicosansäure, 19-Hydroxy-heneicosansäure, 20-Hydroxy-heneicosansäure, 10-Hydroxy-docosansäure, 11-Hydroxy-docosansäure, 12-Hydroxy-docosansäure, 13-Hydroxy-docosansäure, 14-Hydroxy-docosansäure, 15-Hydroxy-docosansäure, 16-Hydroxy-docosansäure, 17-Hydroxy-docosansäure, 18-Hydroxy-docosansäure, 19-Hydroxy-docosansäure, 20-Hydroxy-docosansäure, 21-Hydroxy-docosansäure, 10-Hydroxy-tricosansäure, 11-Hydroxy-tricosansäure, 12-Hydroxy-tricosansäure, 13-Hydroxy-tricosansäure, 14-Hydroxy-tricosansäure, 15-Hydroxy-tricosansäure, 16-Hydroxy-tricosansäure, 17-Hydroxy-tricosansäure, 18-Hydroxy-tricosansäure, 19-Hydroxy-tricosansäure, 20-Hydroxy-tricosansäure, 21-Hydroxy-tricosansäure, 22-Hydroxy-tricosansäure, 10-Hydroxy-tetracosansäure, 11-Hydroxy-tetracosansäure, 12-Hydroxy-tetracosansäure, 13-Hydroxy-tetracosansäure, 14-Hydroxy-tetracosansäure, 15-Hydroxy-tetracosansäure, 16-Hydroxy-tetracosansäure, 17-Hydroxy-tetracosansäure, 18-Hydroxy-tetracosansäure, 19-Hydroxy-tetracosansäure, 20-Hydroxy-tetracosansäure, 21-Hydroxy-tetracosansäure, 22-Hydroxy-tetracosansäure, 23-Hydroxy-tetracosansäure, 10-Hydroxy-pentacosansäure, 11-Hydroxy-pentacosansäure, 12-Hydroxy-pentacosansäure, 13-Hydroxy-pentacosansäure, 14-Hydroxy-pentacosansäure, 15-Hydroxy-pentacosansäure, 16-Hydroxy-pentacosansäure, 17-Hydroxy-pentacosansäure, 18-Hydroxy-pentacosansäure, 19-Hydroxy-pentacosansäure, 20-Hydroxy-pentacosansäure, 21-Hydroxy-pentacosansäure, 22-Hydroxy-pentacosansäure, 23-Hydroxy-pentacosansäure, 24-Hydroxy-pentacosansäure, 10-Hydroxy-hexacosansäure, 11-Hydroxy-hexacosansäure, 12-Hydroxy-hexacosansäure, 13-Hydroxy-hexacosansäure, 14-Hydroxy-hexacosansäure, 15-Hydroxy-hexacosansäure, 16-Hydroxy-hexacosansäure, 17-Hydroxy-hexacosansäure, 18-Hydroxy-hexacosansäure, 19-Hydroxy-hexacosansäure, 20-Hydroxy-hexacosansäure, 21-Hydroxy-hexacosansäure, 22-Hydroxy-hexacosansäure, 23-Hydroxy-hexacosansäure, 24-Hydroxy-hexacosansäure, 25-Hydroxy-hexacosansäure, 10-Hydroxy-heptacosansäure, 11-Hydroxy-heptacosansäure, 12-Hydroxy-heptacosansäure, 13-Hydroxy-heptacosansäure, 14-Hydroxy-heptacosansäure, 15-Hydroxy-heptacosansäure, 16-Hydroxy-heptacosansäure, 17-Hydroxy-heptacosansäure, 18-Hydroxy-heptacosansäure, 19-Hydroxy-heptacosansäure, 20-Hydroxy-heptacosansäure, 21-Hydroxy-heptacosansäure, 22-Hydroxy-heptacosansäure, 23-Hydroxy-heptacosansäure, 24-Hydroxy-heptacosansäure, 25-Hydroxy-heptacosansäure, 26-Hydroxy-heptacosansäure, 10-Hydroxy-octacosansäure, 11-Hydroxy-octacosansäure, 12-Hydroxy-octacosansäure, 13-Hydroxy-octacosansäure, 14-Hydroxy-octacosansäure, 15-Hydroxy-octacosansäure, 16-Hydroxy-octacosansäure, 17-Hydroxy-octacosansäure, 18-Hydroxy-octacosansäure, 19-Hydroxy-octacosansäure, 20-Hydroxy-octacosansäure, 21-Hydroxy-octacosansäure, 22-Hydroxy-octacosansäure, 23-Hydroxy-octacosansäure, 24-Hydroxy-octacosansäure, 25-Hydroxy-octacosansäure, 26-Hydroxy-octacosansäure, 27-Hydroxy-octacosansäure, 10-Hydroxy-nonacosansäure, 11-Hydroxy-nonacosansäure, 12-Hydroxy-nonacosansäure, 13-Hydroxy-nonacosansäure, 14-Hydroxy-nonacosansäure, 15-Hydroxy-nonacosansäure, 16-Hydroxy-nonacosansäure, 17-Hydroxy-nonacosansäure, 18-Hydroxy-nonacosansäure, 19-Hydroxy-nonacosansäure, 20-Hydroxy-nonacosansäure, 21-Hydroxy-nonacosansäure, 22-Hydroxy-nonacosansäure, 23-Hydroxy-nonacosansäure, 24-Hydroxy-nonacosansäure, 25-Hydroxy-nonacosansäure, 26-Hydroxy-nonacosansäure, 27-Hydroxy-nonacosansäure, 28-Hydroxy-nonacosansäure, 10-Hydroxy-triacontansäure, 11-Hydroxy-triacontansäure, 12-Hydroxy-triacontansäure, 13-Hydroxy-triacontansäure, 14-Hydroxy-triacontansäure, 15-Hydroxy-triacontansäure, 16-Hydroxy-triacontansäure, 17-Hydroxy-triacontansäure, 18-Hydroxy-triacontansäure, 19-Hydroxy-triacontansäure, 20-Hydroxy-triacontansäure, 21-Hydroxy-triacontansäure, 22-Hydroxy-triacontansäure, 23-Hydroxy-triacontansäure, 24-Hydroxy-triacontansäure, 25-Hydroxy-triacontansäure, 26-Hydroxy-triacontansäure, 27-Hydroxy-triacontansäure, 28-Hydroxy-triacontansäure, 29-Hydroxy-triacontansäure, 10,13-Dihydroxytetradecansäure, 10,13 -Dihydroxypentadecansäure, 10,13-Dihydroxyhexadecansäure, 11,14-Dihydroxyhexadecansäure, 10,13-Dihydroxyheptadecansäure, 11,14-Dihydroxyheptadecansäure, 12,15-Dihydroxyheptadecansäure, 10,13 -Dihydroxyoctadecansäure, 11,14-Dihydroxyoctadecansäure, 12,15-Dihydroxyoctadecansäure, 13,16-Dihydroxyoctadecansäure, 10,13 -Dihydroxynonadecansäure, 11,14-Dihydroxynonadecansäure, 12,15-Dihydroxynonadecansäure, 13,16-Dihydroxynonadecansäure, 14,17-Dihydroxynonadecansäure, 10,13-Dihydroxyicosanansäure, 11,14-Dihydroxyicosanansäure, 12,15-Dihydroxyicosanansäure, 13,16-Dihydroxyicosanansäure, 14,17-Dihydroxyicosanansäure, 15,18-Dihydroxyicosanansäure, 10,13-Dihydroxyheneicosansäure, 11,14-Dihydroxyheneicosansäure, 12,15-Dihydroxyheneicosansäure, 13,16-Dihydroxyheneicosansäure, 14,17-Dihydroxyheneicosansäure, 15,18-Dihydroxyheneicosansäure, 16,19-Dihydroxyheneicosansäure, 10,13-Dihydroxydocosansäure, 11,14-Dihydroxydocosansäure, 12,15-Dihydroxydocosansäure, 13,16-Dihydroxydocosansäure, 14,17-Dihydroxydocosansäure, 15,18-Dihydroxydocosansäure, 16,19-Dihydroxydocosansäure, 17,20-Dihydroxydocosansäure, 10,13-Dihydroxytricosansäure, 11,14-Dihydroxytricosansäure, 12,15-Dihydroxytricosansäure, 13,16-Dihydroxytricosansäure, 14,17-Dihydroxytricosansäure, 15,18-Dihydroxytricosansäure, 16,19-Dihydroxytricosansäure, 17,20-Dihydroxytricosansäure, 18,21-Dihydroxytricosansäure, 10,13-Dihydroxytetracosansäure, 11,14-Dihydroxytetracosansäure, 12,15-Dihydroxytetracosansäure, 13,16-Dihydroxytetracosansäure, 14,17-Dihydroxytetracosansäure, 15,18-Dihydroxytetracosansäure, 16,19-Dihydroxytetracosansäure, 17,20-Dihydroxytetracosansäure, 18,21-Dihydroxytetracosansäure, 19,22-Dihydroxytetracosansäure, 10,13 -Dihydroxypentacosansäure, 11,14-Dihydroxypentacosansäure, 12,15-Dihydroxypentacosansäure, 13,16-Dihydroxypentacosansäure, 14,17-Dihydroxypentacosansäure, 15,18-Dihydroxypentacosansäure, 16,19-Dihydroxypentacosansäure, 17,20-Dihydroxypentacosansäure, 18,21-Dihydroxypentacosansäure, 19,22-Dihydroxypentacosansäure, 20,23-Dihydroxypentacosansäure, 10,13-Dihydroxyhexacosansäure, 11,14-Dihydroxyhexacosansäure, 12,15-Dihydroxyhexacosansäure, 13,16-Dihydroxyhexacosansäure, 14,17-Dihydroxyhexacosansäure, 15,18-Dihydroxyhexacosansäure, 16,19-Dihydroxyhexacosansäure, 17,20-Dihydroxyhexacosansäure, 18,21-Dihydroxyhexacosansäure, 19,22-Dihydroxyhexacosansäure, 20,23-Dihydroxyhexacosansäure, 21,24-Dihydroxyhexacosansäure, 10,13-Dihydroxyheptacosansäure, 11,14-Dihydroxyheptacosansäure, 12,15-Dihydroxyheptacosansäure, 13,16-Dihydroxyheptacosansäure, 14,17-Dihydroxyheptacosansäure, 15,18-Dihydroxyheptacosansäure, 16,19-Dihydroxyheptacosansäure, 17,20-Dihydroxyheptacosansäure, 18,21-Dihydroxyheptacosansäure, 19,22-Dihydroxyheptacosansäure, 20,23-Dihydroxyheptacosansäure, 21,24-Dihydroxyheptacosansäure, 22,25-Dihydroxyheptacosansäure, 10,13 -Dihydroxyoctacosansäure, 11,14-Dihydroxyoctacosansäure, 12,15-Dihydroxyoctacosansäure, 13,16-Dihydroxyoctacosansäure, 14,17-Dihydroxyoctacosansäure, 15,18-Dihydroxyoctacosansäure, 16,19-Dihydroxyoctacosansäure, 17,20-Dihydroxyoctacosansäure, 18,21-Dihydroxyoctacosansäure, 19,22-Dihydroxyoctacosansäure, 20,23-Dihydroxyoctacosansäure, 21,24-Dihydroxyoctacosansäure, 22,25-Dihydroxyoctacosansäure, 23,26-Dihydroxyoctacosansäure, 10,13 -Dihydroxynonacosansäure, 11,14-Dihydroxynonacosansäure, 12,15-Dihydroxynonacosansäure, 13,16-Dihydroxynonacosansäure, 14,17-Dihydroxynonacosansäure, 15,18-Dihydroxynonacosansäure, 16,19-Dihydroxynonacosansäure, 17,20-Dihydroxynonacosansäure, 18,21-Dihydroxynonacosansäure, 19,22-Dihydroxynonacosansäure, 20,23-Dihydroxynonacosansäure, 21,24-Dihydroxynonacosansäure, 22,25-Dihydroxynonacosansäure, 23,26-Dihydroxynonacosansäure, 24,27-Dihydroxynonacosansäure, 10,13 -Dihydroxytriacontansäure, 11,14-Dihydroxytriacontansäure, 12,15-Dihydroxytriacontansäure, 13,16-Dihydroxytriacontansäure, 14,17-Dihydroxytriacontansäure, 15,18-Dihydroxytriacontansäure, 16,19-Dihydroxytriacontansäure, 17,20-Dihydroxytriacontansäure, 18,21-Dihydroxytriacontansäure, 19,22-Dihydroxytriacontansäure, 20,23-Dihydroxytriacontansäure, 21,24-Dihydroxytriacontansäure, 22,25-Dihydroxytriacontansäure, 23,26-Dihydroxytriacontansäure, 24,27-Dihydroxytriacontansäure und 25,28-Dihydroxytriacontansäure. Dabei hat vorzugsweise zumindest die der Carbonsäurefunktion am nächsten befindliche Hydroxygruppe, bevorzugt aber alle Hydroxygruppen, R-Konformation. Verbleibende C=C-Doppelbindungen haben bevorzugt cis-Konformation.

Unter diesen sind stärker bevorzugt die Monohydroxyfettsäuren z.B. 10-Hydroxy-undecansäure, 10-Hydroxy-dodecansäure, 11-Hydroxy-dodecansäure, 10-Hydroxy-tridecansäure, 11-Hydroxy-tridecansäure, 12-Hydroxy-tridecansäure, 10-Hydroxy-tetradecansäure, 11-Hydroxy-tetradecansäure, 12-Hydroxy-tetradecansäure, 13-Hydroxy-tetradecansäure, 10-Hydroxy-pentadecansäure, 11-Hydroxy-pentadecansäure, 12-Hydroxy-pentadecansäure, 13-Hydroxy-pentadecansäure, 14-Hydroxy-pentadecansäure, 10-Hydroxy-hexadecansäure, 11-Hydroxy-hexadecansäure, 12-Hydroxy-hexadecansäure, 13-Hydroxy-hexadecansäure, 14-Hydroxy-hexadecansäure, 15-Hydroxy-hexadecansäure, 10-Hydroxy-heptadecansäure, 11-Hydroxy-heptadecansäure, 12-Hydroxy-heptadecansäure, 13-Hydroxy-heptadecansäure, 14-Hydroxy-heptadecansäure, 15-Hydroxy-heptadecansäure, 16-Hydroxy-heptadecansäure, 10-Hydroxy-octadecansäure, 11-Hydroxy-octadecansäure, 12-Hydroxyoctadecansäure, 13-Hydroxy-octadecansäure, 14-Hydroxy-octadecansäure, 15-Hydroxyoctadecansäure, 16-Hydroxy-octadecansäure, 17-Hydroxy-octadecansäure, 10-Hydroxy-nonadecansäure, 11-Hydroxy-nonadecansäure, 12-Hydroxy-nonadecansäure, 13-Hydroxy-nonadecansäure, 14-Hydroxy-nonadecansäure, 15-Hydroxy-nonadecansäure, 16-Hydroxy-nonadecansäure, 17-Hydroxy-nonadecansäure, 18-Hydroxy-nonadecansäure, 10-Hydroxy-icosanansäure, 11-Hydroxy-icosanansäure, 12-Hydroxy-icosanansäure, 13-Hydroxy-icosanansäure, 14-Hydroxy-icosanansäure, 15-Hydroxy-icosanansäure, 16-Hydroxy-icosanansäure, 17-Hydroxy-icosanansäure, 18-Hydroxy-icosanansäure, 19-Hydroxy-icosanansäure, 10-Hydroxy-heneicosansäure, 11-Hydroxy-heneicosansäure, 12-Hydroxy-heneicosansäure, 13-Hydroxy-heneicosansäure, 14-Hydroxy-heneicosansäure, 15-Hydroxy-heneicosansäure, 16-Hydroxy-heneicosansäure, 17-Hydroxy-heneicosansäure, 18-Hydroxy-heneicosansäure, 19-Hydroxy-heneicosansäure, 20-Hydroxy-heneicosansäure, 10-Hydroxy-docosansäure, 11-Hydroxy-docosansäure, 12-Hydroxy-docosansäure, 13-Hydroxy-docosansäure, 14-Hydroxy-docosansäure, 15-Hydroxy-docosansäure, 16-Hydroxy-docosansäure, 17-Hydroxy-docosansäure, 18-Hydroxy-docosansäure, 19-Hydroxy-docosansäure, 20-Hydroxy-docosansäure, 21-Hydroxy-docosansäure, 10-Hydroxy-tricosansäure, 11-Hydroxy-tricosansäure, 12-Hydroxy-tricosansäure, 13-Hydroxy-tricosansäure, 14-Hydroxy-tricosansäure, 15-Hydroxy-tricosansäure, 16-Hydroxy-tricosansäure, 17-Hydroxy-tricosansäure, 18-Hydroxy-tricosansäure, 19-Hydroxy-tricosansäure, 20-Hydroxy-tricosansäure, 21-Hydroxy-tricosansäure, 22-Hydroxy-tricosansäure, 10-Hydroxy-tetracosansäure, 11-Hydroxy-tetracosansäure, 12-Hydroxy-tetracosansäure, 13-Hydroxy-tetracosansäure, 14-Hydroxy-tetracosansäure, 15-Hydroxy-tetracosansäure, 16-Hydroxy-tetracosansäure, 17-Hydroxy-tetracosansäure, 18-Hydroxy-tetracosansäure, 19-Hydroxy-tetracosansäure, 20-Hydroxy-tetracosansäure, 21-Hydroxy-tetracosansäure, 22-Hydroxy-tetracosansäure, 23-Hydroxy-tetracosansäure, 10-Hydroxy-pentacosansäure, 11-Hydroxy-pentacosansäure, 12-Hydroxy-pentacosansäure, 13-Hydroxy-pentacosansäure, 14-Hydroxy-pentacosansäure, 15-Hydroxy-pentacosansäure, 16-Hydroxy-pentacosansäure, 17-Hydroxy-pentacosansäure, 18-Hydroxy-pentacosansäure, 19-Hydroxy-pentacosansäure, 20-Hydroxy-pentacosansäure, 21-Hydroxy-pentacosansäure, 22-Hydroxy-pentacosansäure, 23-Hydroxy-pentacosansäure, 24-Hydroxy-pentacosansäure, 10-Hydroxy-hexacosansäure, 11-Hydroxy-hexacosansäure, 12-Hydroxy-hexacosansäure, 13-Hydroxy-hexacosansäure, 14-Hydroxy-hexacosansäure, 15-Hydroxy-hexacosansäure, 16-Hydroxy-hexacosansäure, 17-Hydroxy-hexacosansäure, 18-Hydroxy-hexacosansäure, 19-Hydroxy-hexacosansäure, 20-Hydroxy-hexacosansäure, 21-Hydroxy-hexacosansäure, 22-Hydroxy-hexacosansäure, 23-Hydroxy-hexacosansäure, 24-Hydroxy-hexacosansäure, 25-Hydroxy-hexacosansäure, 10-Hydroxy-heptacosansäure, 11-Hydroxy-heptacosansäure, 12-Hydroxy-heptacosansäure, 13-Hydroxy-heptacosansäure, 14-Hydroxy-heptacosansäure, 15-Hydroxy-heptacosansäure, 16-Hydroxy-heptacosansäure, 17-Hydroxy-heptacosansäure, 18-Hydroxy-heptacosansäure, 19-Hydroxy-heptacosansäure, 20-Hydroxy-heptacosansäure, 21-Hydroxy-heptacosansäure, 22-Hydroxy-heptacosansäure, 23-Hydroxy-heptacosansäure, 24-Hydroxy-heptacosansäure, 25-Hydroxy-heptacosansäure, 26-Hydroxy-heptacosansäure, 10-Hydroxy-octacosansäure, 11-Hydroxy-octacosansäure, 12-Hydroxy-octacosansäure, 13-Hydroxy-octacosansäure, 14-Hydroxy-octacosansäure, 15-Hydroxy-octacosansäure, 16-Hydroxy-octacosansäure, 17-Hydroxy-octacosansäure, 18-Hydroxy-octacosansäure, 19-Hydroxy-octacosansäure, 20-Hydroxy-octacosansäure, 21-Hydroxy-octacosansäure, 22-Hydroxy-octacosansäure, 23-Hydroxy-octacosansäure, 24-Hydroxy-octacosansäure, 25-Hydroxy-octacosansäure, 26-Hydroxy-octacosansäure, 27-Hydroxy-octacosansäure, 10-Hydroxy-nonacosansäure, 11-Hydroxy-nonacosansäure, 12-Hydroxy-nonacosansäure, 13-Hydroxy-nonacosansäure, 14-Hydroxy-nonacosansäure, 15-Hydroxy-nonacosansäure, 16-Hydroxy-nonacosansäure, 17-Hydroxy-nonacosansäure, 18-Hydroxy-nonacosansäure, 19-Hydroxy-nonacosansäure, 20-Hydroxy-nonacosansäure, 21-Hydroxy-nonacosansäure, 22-Hydroxy-nonacosansäure, 23-Hydroxy-nonacosansäure, 24-Hydroxy-nonacosansäure, 25-Hydroxy-nonacosansäure, 26-Hydroxy-nonacosansäure, 27-Hydroxy-nonacosansäure, 28-Hydroxy-nonacosansäure, 10-Hydroxy-triacontansäure, 11-Hydroxy-triacontansäure, 12-Hydroxy-triacontansäure, 13-Hydroxy-triacontansäure, 14-Hydroxy-triacontansäure, 15-Hydroxy-triacontansäure, 16-Hydroxy-triacontansäure, 17-Hydroxy-triacontansäure, 18-Hydroxy-triacontansäure, 19-Hydroxy-triacontansäure, 20-Hydroxy-triacontansäure, 21-Hydroxy-triacontansäure, 22-Hydroxy-triacontansäure, 23-Hydroxy-triacontansäure, 24-Hydroxy-triacontansäure, 25-Hydroxy-triacontansäure, 26-Hydroxy-triacontansäure, 27-Hydroxy-triacontansäure, 28-Hydroxy-triacontansäure und 29-Hydroxy-triacontansäure. Stärker bevorzugt sind 10-Hydroxy-hexadecansäure, 11-Hydroxy-hexadecansäure, 12-Hydroxy-hexadecansäure, 13-Hydroxy-hexadecansäure, 14-Hydroxy-hexadecansäure, 10-Hydroxy-heptadecansäure, 11-Hydroxy-heptadecansäure, 12-Hydroxy-heptadecansäure, 13-Hydroxy-heptadecansäure, 14-Hydroxy-heptadecansäure, 10-Hydroxy-octadecansäure, 11-Hydroxyoctadecansäure, 12-Hydroxy-octadecansäure, 13-Hydroxy-octadecansäure, 14-Hydroxyoctadecansäure, 10-Hydroxy-nonadecansäure, 11-Hydroxy-nonadecansäure, 12-Hydroxy-nonadecansäure, 13-Hydroxy-nonadecansäure, 14-Hydroxy-nonadecansäure, 10-Hydroxy-icosanansäure, 11-Hydroxy-icosanansäure, 12-Hydroxy-icosanansäure, 13-Hydroxy-icosanansäure und 14-Hydroxy-icosanansäure. Weiter bevorzugt sind die Monohydroxyfettsäuren ausgewählt aus 10-Hydroxy-hexadecansäure, 11-Hydroxy-hexadecansäure, 12-Hydroxy-hexadecansäure, 13-Hydroxy-hexadecansäure, 14-Hydroxy-hexadecansäure, 10-Hydroxy-heptadecansäure, 11-Hydroxy-heptadecansäure, 12-Hydroxy-heptadecansäure, 13-Hydroxy-heptadecansäure, 14-Hydroxy-heptadecansäure, 10-Hydroxyoctadecansäure, 11-Hydroxy-octadecansäure, 12-Hydroxy-octadecansäure, 13-Hydroxyoctadecansäure, 14-Hydroxy-octadecansäure, 10-Hydroxy-nonadecansäure, 11-Hydroxy-nonadecansäure, 12-Hydroxy-nonadecansäure, 13-Hydroxy-nonadecansäure, 14-Hydroxy-nonadecansäure, 10-Hydroxy-icosanansäure, 11-Hydroxy-icosanansäure, 12-Hydroxy-icosanansäure, 13-Hydroxy-icosanansäure und 14-Hydroxy-icosanansäure. Dabei hat die Hydroxygruppe bevorzugt *R*-Konformation.

Um die Verarbeitbarkeit der Hydroxyfettsäuren zu verbessern, sind insbesondere bei Raumtemperatur (25°C, 1 atm) flüssige Hydroxyfettsäuren bevorzugt. Unter diesem Gesichtspunkt ist es bevorzugt, dass die Hydroxyfettsäuren eine oder zwei C=C Doppelbindungen enthalten. Bevorzugt wird die der Carboxylgruppe der Fettsäure am nächsten befindliche C=C Doppelbindung hydratisiert, während die eine oder zwei weiteren C=C Doppelbindungen erhalten bleiben. Darüber hinaus ist es auch möglich, dass die beiden der Carboxylgruppe der Fettsäure am nächsten befindlichen C=C Doppelbindungen hydratisiert werden, während die eine weitere C=C Doppelbindung erhalten bleibt.

Bevorzugte Hydroxyfettsäuren, die eine C=C Doppelbindung enthalten, sind 10-Hydroxytetradec-12-ensäure, 10-Hydroxy-pentadec-12-ensäure, 11-Hydroxy-pentadec-13-ensäure, 10-Hydroxy-hexadec-12-ensäure, 11-Hydroxy-hexadec-13-ensäure, 12-Hydroxy-hexadec-14-ensäure, 10-Hydroxy-heptadec-12-ensäure, 11-Hydroxy-heptadec-13-ensäure, 12-Hydroxyheptadec-14-ensäure, 13-Hydroxy-heptadec-15-ensäure, 10-Hydroxy-octadec-12-ensäure, 11-Hydroxy-octadec-13-ensäure, 12-Hydroxy-octadec-14-ensäure, 13-Hydroxy-octadec-15-ensäure, 14-Hydroxy-octadec-16-ensäure, 10-Hydroxy-nonadec-12-ensäure, 11-Hydroxynonadec-13-ensäure, 12-Hydroxy-nonadec-14-ensäure, 13-Hydroxy-nonadec-15-ensäure, 14-Hydroxy-nonadec-16-ensäure, 15-Hydroxy-nonadec-17-ensäure, 10-Hydroxy-icosan-12-ensäure, 11-Hydroxy-icosan-13-ensäure, 12-Hydroxy-icosan-14-ensäure, 13-Hydroxy-icosan-15-ensäure, 14-Hydroxy-icosan-16-ensäure, 15-Hydroxy-icosan-17-ensäure, 16-Hydroxyicosan-18-ensäure, 10-Hydroxy-heneicos-12-ensäure, 11-Hydroxy-heneicos-13-ensäure, 12-Hydroxy-heneicos-14-ensäure, 13-Hydroxy-heneicos-15-ensäure, 14-Hydroxy-heneicos-16-ensäure, 15-Hydroxy-heneicos-17-ensäure, 16-Hydroxy-heneicos-18-ensäure, 17-Hydroxyheneicos-19-ensäure, 10-Hydroxy-docos-12-ensäure, 11-Hydroxy-docos-13-ensäure, 12-Hydroxy-docos-14-ensäure, 13-Hydroxy-docos-15-ensäure, 14-Hydroxy-docos-16-ensäure, 15-Hydroxy-docos-17-ensäure, 16-Hydroxy-docos-18-ensäure, 17-Hydroxy-docos-19-ensäure, 18-Hydroxy-docos-20-ensäure, 10-Hydroxy-tricos-12-ensäure, 11-Hydroxy-tricos-13-ensäure, 12-Hydroxy-tricos-14-ensäure, 13-Hydroxy-tricos-15-ensäure, 14-Hydroxytricos-16-ensäure, 15-Hydroxy-tricos-17-ensäure, 16-Hydroxy-tricos-18-ensäure, 17-Hydroxy-tricos-19-ensäure, 18-Hydroxy-tricos-20-ensäure, 19-Hydroxy-tricos-21-ensäure, 10-Hydroxy-tetracos-12-ensäure, 11-Hydroxy-tetracos-13-ensäure, 12-Hydroxy-tetracos-14-ensäure, 13-Hydroxy-tetracos-15-ensäure, 14-Hydroxy-tetracos-16-ensäure, 15-Hydroxytetracos-17-ensäure, 16-Hydroxy-tetracos-18-ensäure, 17-Hydroxy-tetracos-19-ensäure, 18-Hydroxy-tetracos-20-ensäure, 19-Hydroxy-tetracos-21-ensäure und 20-Hydroxy-tetracos-22-ensäure.

Zur Verbesserung der Verarbeitbarkeit sind weitere Modifikationen, die die Kristallisation bzw. parallele Ausrichtung der FS-Reste stören und auch Abmischungen mit entsprechenden Produkten zur Kristallisationsverhinderung geeignet. Dazu zählen ungesättigte Fettsäuren, insbesondere in cis-Konfiguration, Kettenverzweigungen wie z.B. durch Methylgruppen oder Alkoxygruppen, die durch Alkoholyse von epoxidierten Ketten erhältlich sind, Epoxidgruppen sowie Mischungen verschiedener Kettenlängen und sonstige Abmischungen mit Komponenten, die als Lösemittel oder Weichmacher wirken.

Bevorzugte Hydroxyfettsäuren, die zwei C=C Doppelbindungen enthalten sind 10-Hydroxyheptadeca-12,15-diensäure, 10-Hydroxy-octadeca-12,15-diensäure, 11-Hydroxy-octadeca-13,16-diensäure, 10-Hydroxy-nonadeca-12,15-diensäure, 11-Hydroxy-nonadeca-13,16-diensäure, 12-Hydroxy-nonadeca-14,17-diensäure, 10-Hydroxy-icosana-12,15-diensäure, 11-Hydroxy-icosana-13,16-diensäure, 12-Hydroxy-icosana-14,17-diensäure, 13-Hydroxyicosana-15,18-diensäure, 10-Hydroxy-heneicosa-12,15-diensäure, 11-Hydroxy-heneicosa-13,16-diensäure, 12-Hydroxy-heneicosa-14,17-diensäure, 13-Hydroxy-heneicosa-15,18-diensäure, 14-Hydroxy-heneicosa-16,19-diensäure, 10-Hydroxy-docosa-12,15-diensäure, 11-Hydroxy-docosa-13,16-diensäure, 12-Hydroxy-docosa-14,17-diensäure, 13-Hydroxy-docosa-15,18-diensäure, 14-Hydroxy-docosa-16,19-diensäure, 15-Hydroxy-docosa-17,20-diensäure, 10-Hydroxy-tricosa-12,15-diensäure, 11-Hydroxy-tricosa-13,16-diensäure, 12-Hydroxytricosa-14,17-diensäure, 13-Hydroxy-tricosa-15,18-diensäure, 14-Hydroxy-tricosa-16,19-diensäure, 15-Hydroxy-tricosa-17,20-diensäure, 16-Hydroxy-tricosa-18,21-diensäure, 10-Hydroxy-tetracosa-12,15-diensäure, 11-Hydroxy-tetracosa-13,16-diensäure, 12-Hydroxytetracosa-14,17-diensäure, 13-Hydroxy-tetracosa-15,18-diensäure, 14-Hydroxy-tetracosa-16,19-diensäure, 15-Hydroxy-tetracosa-17,20-diensäure, 16-Hydroxy-tetracosa-18,21-diensäure, 17-Hydroxy-tetracosa-19,22-diensäure, 10-Hydroxy-pentacosa-12,15-diensäure, 11-Hydroxy-pentacosa-13, 16-diensäure, 12-Hydroxy-pentacosa-14,17-diensäure, 13-Hydroxy-pentacosa-15,18-diensäure, 14-Hydroxy-pentacosa-16,19-diensäure, 15-Hydroxy-pentacosa-17,20-diensäure, 16-Hydroxy-pentacosa-18,21-diensäure, 17-Hydroxy-pentacosa-19,22-diensäure, 18-Hydroxy-pentacosa-20,23-diensäure, 10-Hydroxy-hexacosa-12,15-diensäure, 11-Hydroxy-hexacosa-13,16-diensäure, 12-Hydroxy-hexacosa-14,17-diensäure, 13-Hydroxy-hexacosa-15,18-diensäure, 14-Hydroxy-hexacosa-16,19-diensäure, 15-Hydroxyhexacosa-17,20-diensäure, 16-Hydroxy-hexacosa-18,21-diensäure, 17-Hydroxy-hexacosa-19,22-diensäure, 18-Hydroxy-hexacosa-20,23-diensäure, 19-Hydroxy-hexacosa-21,24-diensäure, 10-Hydroxy-heptacosa-12,15-diensäure, 11-Hydroxy-heptacosa-13,16-diensäure, 12-Hydroxy-heptacosa-14,17-diensäure, 13-Hydroxy-heptacosa-15,18-diensäure, 14-Hydroxy-heptacosa-16,19-diensäure, 15-Hydroxy-heptacosa-17,20-diensäure, 16-Hydroxyheptacosa-18,21-diensäure, 17-Hydroxy-heptacosa-19,22-diensäure, 18-Hydroxy-heptacosa-20,23-diensäure, 19-Hydroxy-heptacosa-21,24-diensäure, 20-Hydroxy-heptacosa-22,25-diensäure, 10-Hydroxy-octacosa-12,15-diensäure, 11-Hydroxy-octacosa-13,16-diensäure, 12-Hydroxy-octacosa-14,17-diensäure, 13-Hydroxy-octacosa-15,18-diensäure, 14-Hydroxyoctacosa-16,19-diensäure, 15-Hydroxy-octacosa-17,20-diensäure, 16-Hydroxy-octacosa-18,21-diensäure, 17-Hydroxy-octacosa-19,22-diensäure, 18-Hydroxy-octacosa-20,23-diensäure, 19-Hydroxy-octacosa-21,24-diensäure, 20-Hydroxy-octacosa-22,25-diensäure, 21-Hydroxy-octacosa-23,26-diensäure, 10-Hydroxy-nonacosa-12,15-diensäure, 11-Hydroxynonacosa-13,16-diensäure, 12-Hydroxy-nonacosa-14,17-diensäure, 13-Hydroxy-nonacosa-15,18-diensäure, 14-Hydroxy-nonacosa-16,19-diensäure, 15-Hydroxy-nonacosa-17,20-diensäure, 16-Hydroxy-nonacosa-18,21-diensäure, 17-Hydroxy-nonacosa-19,22-diensäure, 18-Hydroxy-nonacosa-20,23-diensäure, 19-Hydroxy-nonacosa-21,24-diensäure, 20-Hydroxynonacosa-22,25-diensäure, 21-Hydroxy-nonacosa-23,26-diensäure, 22-Hydroxy-nonacosa-24,27-diensäure, 10-Hydroxy-triaconta-12,15-diensäure, 11-Hydroxy-triaconta-13,16-diensäure, 12-Hydroxy-triaconta-14,17-diensäure, 13-Hydroxy-triaconta-15,18-diensäure, 14-Hydroxy-triaconta-16,19-diensäure, 15-Hydroxy-triaconta-17,20-diensäure, 16-Hydroxytriaconta-18,21-diensäure, 17-Hydroxy-triaconta-19,22-diensäure, 18-Hydroxy-triaconta-20,23-diensäure, 19-Hydroxy-triaconta-21,24-diensäure, 20-Hydroxy-triaconta-22,25-diensäure, 21-Hydroxy-triaconta-23,26-diensäure, 22-Hydroxy-triaconta-24,27-diensäure und 23-Hydroxy-triaconta-25,28-diensäure.

Bevorzugte Dihydroxyfettsäuren, die eine C=C Doppelbindungen enthalten, sind 10,13-Dihydroxy-heptadec-15-ensäure, 10,13-Dihydroxy-octadec-15-ensäure, 11,14-Dihydroxyoctadec-16-ensäure, 10,13-Dihydroxy-nonadec-15-ensäure, 11,14-Dihydroxy-nonadec-16-ensäure, 12,15-Dihydroxy-nonadec-17-ensäure, 10,13-Dihydroxy-icosan-15-ensäure, 11,14-Dihydroxy-icosan-16-ensäure, 12,15-Dihydroxy-icosan-17-ensäure, 13,16-Dihydroxy-icosan-18-ensäure, 10,13-Dihydroxy-heneicos-15-ensäure, 11,14-Dihydroxy-heneicos-16-ensäure, 12,15-Dihydroxy-heneicos-17-ensäure, 13,16-Dihydroxy-heneicos-18-ensäure, 14,17-Dihydroxy-heneicos-19-ensäure, 10,13-Dihydroxy-docos-15-ensäure, 11,14-Dihydroxydocos-16-ensäure, 12,15-Dihydroxy-docos-17-ensäure, 13,16-Dihydroxy-docos-18-ensäure, 14,17-Dihydroxy-docos-19-ensäure, 15,18-Dihydroxy-docos-20-ensäure, 10,13-Dihydroxytricos-15-ensäure, 11,14-Dihydroxy-tricos-16-ensäure, 12,15-Dihydroxy-tricos-17-ensäure, 13,16-Dihydroxy-tricos-18-ensäure, 14,17-Dihydroxy-tricos-19-ensäure, 15,18-Dihydroxytricos-20-ensäure, 16,19-Dihydroxy-tricos-21-ensäure, 10,13-Dihydroxy-tetracos-15-ensäure, 11,14-Dihydroxy-tetracos-16-ensäure, 12,15-Dihydroxy-tetracos-17-ensäure, 13,16-Dihydroxy-tetracos-18-ensäure, 14,17-Dihydroxy-tetracos-19-ensäure, 15,18-Dihydroxytetracos-20-ensäure, 16,19-Dihydroxy-tetracos-21-ensäure und 17,20-Dihydroxy-tetracos-22-ensäure.

Unter den hierin genannten Hydroxyfettsäuren sind bevorzugt die C₁₄₋₂₄ Hydroxyfettsäuren, stärker bevorzugt die C₁₆₋₂₀ Hydroxyfettsäuren, noch stärker bevorzugt die C₁₆ oder C₁₈ Hydroxyfettsäuren, am stärksten bevorzugt die C₁₈ Hydroxyfettsäuren.

Unter den hierin genannten Hydroxyfettsäuren sind außerdem bevorzugt die C₁₄₋₂₄ Hydroxyfettsäuren, stärker bevorzugt die C₁₆₋₂₀ Hydroxyfettsäuren, noch stärker bevorzugt die C₁₆ oder C₁₈ Hydroxyfettsäuren, am stärksten bevorzugt C₁₈ Hydroxyfettsäuren, in denen die erste Hydroxygruppe an C₁₀ bis C₁₈, C₁₀ bis C₁₆, C₁₀ bis C₁₄, weiter bevorzugt an C₁₀ bis C₁₃ ist.

Bevorzugt kann beispielsweise Linolsäure ((*cis*,*cis*)-Octadeca-9,12-diensäure) im Verfahren der vorliegenden Erfindung zu (*cis*)-10-Hydroxyoctadec-12-ensäure umgesetzt werden. Dabei wird eine der beiden Doppelbindungen hydratisiert, während die andere Doppelbindung erhalten bleibt.

Außerdem kann bevorzugt beispielsweise Linolensäure ((*cis,cis,cis*)-Octadeca-9,12,15-triensäure) im Verfahren der vorliegenden Erfindung zu (*cis*,*cis*)-10-Hydroxyoctadeca-12,15-diensäure umgesetzt werden. Dabei wird eine der drei Doppelbindungen hydratisiert, während die anderen beiden Doppelbindungen erhalten bleiben.

Um Produkte zu erhalten, die bei Raumtemperatur flüssig sind, ist es bevorzugt, dass die eine oder mehreren Hydroxyfettsäuren ein Gemisch von zwei oder mehr Hydroxyfettsäuren, bevorzugt drei oder mehr, vier oder mehr, oder sogar fünf oder mehr Hydroxyfettsäuren sind. In diesem Gemisch sind bevorzugt mindestens 50 mol-%, 60 mol-%, 70 mol-%, 80 mol-%, 90 mol-%, der Hydroxyfettsäuren ausgewählt aus 10-Hydroxystearinsäure, (*cis*)-10-Hydroxyoctadec-12-ensäure und (*cis*,*cis*)-10-Hydroxyoctadeca-12,15-diensäure.

Es ist bevorzugt, dass das Gemisch, bezogen auf alle Hydroxyfettsäuren, mindestens 40 mol-%, mindestens 50 mol-% oder sogar mindestens 60 mol-%, jedoch 90 mol-% oder weniger, bevorzugt 80 mol-% oder weniger an 10-Hydroxystearinsäure enthält.

Bevorzugt kann das Gemisch von Hydroxyfettsäuren darüber hinaus 10,13-Dihydroxystearinsäure enthalten.

Bevorzugt sind Hydroxyfettsäuren, die 24 oder weniger, bevorzugt 22 oder weniger, Kohlenstoffatome enthalten, wobei sich vorzugsweise die (einzige bzw. der Carboxylgruppe am weitesten entfernteste) Hydroxygruppe an C18 oder näher an der Carboxylgruppe befindet (jedoch vorzugsweise nicht näher an der Carboxylgruppe als C10) und vorzugsweise keine terminale Hydroxygruppe ist.

Demzufolge sind die einen oder mehreren Hydroxyfettsäuren bevorzugt ausgewählt aus hydratiserter Undecylensäure, Myristoleinsäure, Palmitoleinsäure, Margaroleinsäure, Petroselinsäure, Ölsäure, Elaidinsäure, Vaccensäure, Gadoleinsäure, Gondosäure, Cetoleinsäure, Erucasäure, Vernolsäure, cis-5-Eicosensäure, Brassidinsäure und Nervonsäure, und/oder aus der Gruppe bestehend aus hydratiserter Linolsäure, Linolensäure, Calendulasäure, Punicinsäure, Elaeostearinsäure, Arachidonsäure, Timnodonsäure, Clupanodonsäure, Lesquerolsäure, Licansäure und Cervonsäure.

Unter diesen sind die einen oder mehreren Hydroxyfettsäuren bevorzugt ausgewählt aus hydratisierter Undecylensäure, Myristoleinsäure, Palmitoleinsäure, Margaroleinsäure, Petroselinsäure, Ölsäure, Elaidinsäure, Vaccensäure, Gadoleinsäure, Gondosäure, Cetoleinsäure, Erucasäure, Vernolsäure, cis-5-Eicosensäure, Brassidinsäure und Nervonsäure.

Besonders bevorzugt sind Hydroxyfettsäuren, die durch Hydratisierung einer einfach ungesättigten Fettsäure erhalten werden.

Noch stärker bevorzugt sind die einen oder mehreren Hydroxyfettsäuren bevorzugt ausgewählt aus hydratisierter Ölsäure, Linolsäure, Linolensäure und Palmitoleinsäure, wobei die Hydroxyfettsäure bevorzugt ausgewählt ist aus hydratisierter Ölsäure, Linolsäure, Linolensäure und Kombinationen davon, und stärker bevorzugt hydratisierte Ölsäure ist.

Bevorzugt sind die einen oder mehreren Hydroxyfettsäuren ausgewählt aus der Gruppe von 7-Hydroxy-, 8-Hydroxy-, 9-Hydroxy-, 10-Hydroxy-, 11-Hydroxy-, 12-Hydroxy- und 13-Hydroxy-Fettsäuren, wobei die Fettsäure bevorzugt eine C₆₋₄₀-Alkansäure, stärker bevorzugt eine C₁₀₋₃₀-Alkansäure, stärker bevorzugt eine C₁₂₋₂₄-Alkansäure ist. Stärker bevorzugt sind die einen oder mehreren Hydroxyfettsäuren ausgewählt aus der Gruppe von 9-Hydroxy-, 10-Hydroxy-, 11-Hydroxy-, 12-Hydroxy- und 13-Hydroxy-Fettsäuren, wobei die Fettsäure bevorzugt eine C₆₋₄₀-Alkansäure, stärker bevorzugt eine C₁₀₋₃₀-Alkansäure, stärker bevorzugt eine C₁₂₋₂₄-Alkansäure ist. Stärker bevorzugt sind die einen oder mehreren Hydroxyfettsäuren ausgewählt aus der Gruppe von 10-Hydroxy- und 13-Hydroxy-Fettsäuren (insbesondere bevorzugt 10-Hydroxy-Fettsäuren oder 10,13-Dihydroxyfettsäuren), wobei die Fettsäure bevorzugt eine C₆₋₄₀-Alkansäure, stärker bevorzugt eine C₁₀₋₃₀-Alkansäure, stärker bevorzugt eine C₁₂₋₂₄-Alkansäure ist. Die Hydroxyfettsäure enthält bevorzugt eine oder zwei (stärker bevorzugt eine) Hydroxygruppe(n). Optional kann die Hydroxyfettsäure eine oder zwei C=C Doppelbindungen aufweisen. Es ist bevorzugt, dass die Hydroxyfettsäure eine Hydroxygruppe und keine C=C-Doppelbindung aufweist.

Es ist bevorzugt, dass die eine oder mehreren Hydroxyfettsäuren ein Gemisch von zwei oder mehr Hydroxyfettsäuren, bevorzugt drei oder mehr, vier oder mehr, oder sogar fünf oder mehr Hydroxyfettsäuren sind, wobei das Gemisch vorzugsweise, bezogen auf alle Hydroxyfettsäuren, mindestens 40 mol-%, mindestens 50 mol-% oder sogar mindestens 60 mol-%, jedoch 90 mol-% oder weniger, bevorzugt 80 mol-% oder weniger an einer oder mehreren gesättigten Hydroxyfettsäuren, bevorzugt 10-Hydroxystearinsäure, enthält. Das Gemisch enthält vorzugsweise, bezogen auf alle Hydroxyfettsäuren, mindestens 5 mol-%, mindestens 7 mol-% oder sogar mindestens 10 mol-%, jedoch 70 mol-% oder weniger, bevorzugt 50 mol-% oder weniger, stärker bevorzugt 30 mol-% oder weniger, noch stärker bevorzugt 20 mol-% oder weniger, an einer oder mehreren einfach ungesättigten Hydroxyfettsäuren, bevorzugt (*cis*)-10-Hydroxyoctadec-12-ensäure. (*cis*)-10-Hydroxyoctadec-12-ensäure. Das Gemisch enthält vorzugsweise, bezogen auf alle Hydroxyfettsäuren mindestens 0,1 mol-%, mindestens 0,5 mol-% oder sogar mindestens 1 mol-%, jedoch 20 mol-% oder weniger, bevorzugt 15 mol-% oder weniger, stärker bevorzugt 10 mol-% oder weniger, noch stärker bevorzugt 5 mol-% oder weniger, an einer oder mehreren zweifach ungesättigten Hydroxyfettsäuren, bevorzugt (*cis,cis*)-10-Hydroxyoctadeca-12,15-diensäure.

### Umsetzung der einen oder mehreren Hydroxyfettsäuren

Die Umsetzung der einen oder mehreren Hydroxyfettsäuren mit einer oder mehreren mindestens zweiwertigen Linkergruppen erfolgt biotechnologisch.

Die biotechnologische Umsetzung der einen oder mehreren Hydroxyfettsäuren mit einer oder mehreren mindestens zweiwertigen Linkergruppen im Sinne der vorliegenden Erfindung bezieht sich bevorzugt auf die Veresterung und/oder Amidierung einer oder mehrerer Hydroxyfettsäuren mit einer oder mehreren mindestens zweiwertigen Linkergruppen unter Vermittlung (insbesondere Katalyse) eines Proteins.

Insbesondere wenn die eine oder mehreren mindestens zweiwertigen Linkergruppen (Poly)amino(poly)alkohole oder Polyamine enthalten/sind, kann die Umsetzung jedoch selbstverständlich nach beliebigen herkömmlichen Amidierungsverfahren, die dem Fachmann hinlänglich bekannt sind, durchgeführt werden.

Die biotechnologische Umsetzung der einen oder mehreren Hydroxyfettsäuren mit einer oder mehreren mindestens zweiwertigen Linkergruppen im Sinne der vorliegenden Erfindung bezieht sich stärker bevorzugt auf die Veresterung einer oder mehrerer Hydroxyfettsäuren mit einer oder mehreren mindestens zweiwertigen Linkergruppen unter Vermittlung eines Enzyms ausgewählt aus der Gruppe bestehend aus Hydroxylasen, bevorzugt Esterasen, Lipasen und/oder Proteasen, stärker bevorzugt Lipasen. Wenn die eine oder mehreren mindestens zweiwertigen Linkergruppen (Poly)amino(poly)alkohole und/oder Polyamine enthalten, erfolgt die Umsetzung vorzugsweise unter Vermittlung eines Enzyms ausgewählt aus der Gruppe bestehend aus Proteasen und Amidasen.

Das Enzym für die biotechnologische Umsetzung der einen oder mehreren Hydroxyfettsäuren mit einer oder mehreren mindestens zweiwertigen Linkergruppen ist bevorzugt eine Lipase, stärker bevorzugt eine Lipase von *Candida antarctica,* besonders bevorzugt Lipase B von *Candida antarctica.*

Es ist insbesondere bevorzugt, dass die Umsetzung der einen oder mehreren Hydroxyfettsäuren mit einer oder mehreren mindestens zweiwertigen Linkergruppen mittels einer Lipase von *Candida antarctica,* besonders bevorzugt Lipase B von *Candida antarctica,* durchgeführt wird, wobei die eine oder mehreren mindestens zweiwertigen Linkergruppen aus Alkandiolen, Alkantriolen, Alkantetraolen und deren Kombinationen ausgewählt sind. Dabei ist weiterhin bevorzugt, dass die eine oder mehreren mindestens zweiwertigen Linkergruppen aus Alkandiolen, Alkantriolen und deren Kombinationen ausgewählt sind. Noch stärker bevorzugt sind hierbei die eine oder mehreren mindestens zweiwertigen Linkergruppen aus 2-Butyl-2-ethyl-1,3-propandiol, 2-Ethyl-2-(hydroxymethyl)1,3-propandiol, 1,2-Propandiol, 1,3-Propandiol, 1,4-Butandiol, 1,3-Butandiol, 2-Methyl-1,3-propandiol, 1,5-Pentandiol und deren Kombinationen ausgewählt. Die Lipase ist bevorzugt geträgert und besonders bevorzugt ausgewählt aus Addzyme CalB 165G, CalB immo 8285 und CalB immo 8806.

Bei der Umsetzung der einen oder mehreren Hydroxyfettsäuren mit einer oder mehreren mindestens zweiwertigen Linkergruppen werden mindestens zwei, bevorzugt zwei, drei oder vier, der Hydroxy- oder Aminogruppen der mindestens zweiwertigen Linkergruppe mit Hydroxyfettsäure kondensiert.

Wie für den Fachmann ersichtlich enthalten die Hydroxyfettsäuren ebenfalls Hydroxygruppen, die mit Carbonsäuren reagieren könnten. Bei einer herkömmlichen chemischen Veresterung wäre folglich zu erwarten, dass zwischen dem sekundären Alkohol der Hydroxygruppe und der Hydroxygruppe des Polyols nicht, oder nur in begrenzten Umfang, unterschieden werden könnte und sich daher ein Gemisch von zahlreichen verschiedenen Estern bilden würde. Durch die biotechnologische Umsetzung, insbesondere mittels einer Lipase, lassen sich hingegen gezielt die Esterbindungen zwischen den Alkoholgruppen der Polyole und den Carbonsäuregruppen der Hydroxyfettsäuren bilden. 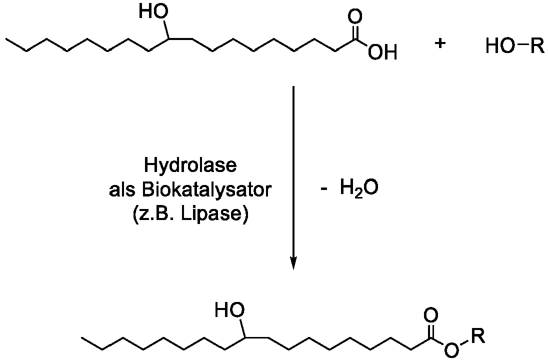 wobei R ein Polyolrest ist, mit dem die Funktionalität, Verzweigung das Molekulargewicht und weitere Eigenschaften des Endproduktes eingestellt werden. Entsprechende Technologien und Zusammenhänge sind dem Fachmann bekannt.

Bevorzugt werden 1 bis 100 mg der Hydroxylase(n), stärker bevorzugt 2 bis 70 mg, noch stärker bevorzugt 5 bis 50 mg pro mmol Substrat (d.h. Gesamtheit der zu hydratisierenden Fettsäuren) verwendet.

Als Medium wird üblicherweise ein Lösungsmittelgemisch eines oder mehrerer C₁₋₆-Nitrile, C₁₋₆-Alholole und/oder C₁₋₆-Ether, wie MTBE, mit einem oder mehreren aliphatischen und/oder aromatischen Kohlenwasserstoffen, wie n-Hexan oder Toluol, verwendet. Dabei beträgt das Volumenverhältnis der einen oder mehreren C₁₋₆-Nitrile, C₁₋₆-Alholole und/oder C₁₋₆-Ether zu den ein oder mehreren aliphatischen und/oder aromatischen Kohlenwasserstoffen bevorzugt 5:1 bis 1:5, stärker bevorzugt 2:1 bis 1:2, insbesondere etwa 1:1. Außerdem wird üblicherweise Molsieb (0,3 bis 0,5 nm, bevorzugt 4 nm) in einer Menge von 0,1 bis 10 g/mmol Substrat, bevorzugt 0,2 bis 1 g/mmol Substrat, stärker bevorzugt 0,4 g/mmol Substrat, verwendet.

Bevorzugt wird/werden die Hydroxylase(n) auf einem Träger immobilisiert und die Umsetzung bei einer Temperatur im Bereich von 30 bis 90°C, stärker bevorzugt 50 bis 85, noch stärker bevorzugt 60 bis 80°C durchgeführt. Die für die jeweilige(n) Hydroxylase(n) geeigneten Bedingungen können von einem Fachmann basierend auf den in der Literatur bekannten Angaben entsprechend eingestellt werden.

Typische Reaktionsbedingungen sind CAL-B mit (30 mg/mmol Substrat) in einer 1:1 (*v*/*v*) Mischung aus MTBE und n-Hexan oder Toluol mit Molsieb (0,4 nm, 0,4 g/mmol Substrat) für 14 h bei 35 oder 60 °C gerührt. Die optimalen Reaktionsbedingungen für die CAL-B sind, wenn die Lipase immobilisiert ist, 60-80 °C. Die Verwendung von immobilisierten Enzymen wird oft wegen der einfachen Rückgewinnung des Enzyms und der Produkte bevorzugt. Darüber hinaus hat die Immobilisierung oft stabilisierende Wirkung auf das Enzym und ermöglicht die Verwendung des Katalysators in organischen Lösungsmitteln (apolar, mit hohem log P, wie Hexan und/oder iso-Octan). Auch polare Lösungsmittel (tert-Butanol, Acetonitril) werden akzeptiert (E.M. Anderson, K. M. Larsson, O. Kirk, One Biocatalyst-Many Applications: The Use of Candida Antarctica B-Lipase in Organic Synthesis, Biocatalysis and Biotransformation 1998, 16, 181-204).

### Die eine oder mehreren mindestens zweiwertigen Linkergruppen

Die eine oder mehreren mindestens zweiwertigen Linkergruppen beziehen sich auf eine oder mehrere Verbindungen, die jeweils zwei oder mehr Gruppen enthalten, die mit einer Carbonsäure unter Bildung einer Bindung reagieren können. Im Folgenden wird auf die mindestens zweiwertige Linkergruppe im Singular Bezug genommen, dies ist jedoch nicht dahingehend zu verstehen, dass die vorliegende Erfindung darauf beschränkt wäre. Vielmehr versteht es sich, dass eine oder mehrere mindestens zweiwertige Linkergruppen eingesetzt werden können. Insbesondere können Gemische von mehreren mindestens zweiwertigen Linkergruppen eingesetzt werden. In einer bevorzugten Ausführungsform enthält die mindestens zweiwertige Linkergruppe jedoch zu mindestens 90 Gew.-% eine einzelne mindestens zweiwertige Linkergruppe.

Die mindestens zweiwertige Linkergruppe ausgewählt aus Polyolen, Polyaminen und (Poly)amino(poly)alkoholen (wie z.B. Aminoalkohol, Polyaminoalkohol, Aminopolyol oder Polyaminopolyol). Bevorzugt ist die mindestens zweiwertige Linkergruppe ein Polyol, wobei das Polyol bevorzugt ein Diol, Triol, Tetraol, Pentaol oder Hexaol, stärker bevorzugt ein Diol, Triol oder Tetraol, ist. Es ist bevorzugt, dass die mindestens zweiwertige Linkergruppe aus nachwachsenden Rohstoffen erhalten ist, also biobasierte zweiwertige Linkergruppen, wie biobasierte Polyole, verwendet werden.

Im Folgenden wird auf das Polyol, das Polyamin oder den (Poly)amino(poly)alkohol im Singular Bezug genommen, dies ist jedoch nicht dahingehend zu verstehen, dass die vorliegende Erfindung darauf beschränkt wäre. Vielmehr versteht es sich, dass ein oder mehrere Polyole und/oder Polyamine und/oder (Poly)amino(poly)alkohole eingesetzt werden können, wie z.B. ein Gemisch von Polyolen und (Poly)amino(poly)alkoholen, ein Gemisch von Polyaminen und Polyolen, und/oder ein Gemisch von Polyaminen und (Poly)amino(poly)alkoholen. In einer bevorzugten Ausführungsform enthält die mindestens zweiwertige Linkergruppe jedoch zu mindestens 90 Gew.-% Polyole, bevorzugt mindestens 90 Gew.-% einer einzelnen Polyolverbindung.

Die mindestens zweiwertige Linkergruppe ist besonders bevorzugt ein Polyol. Es versteht sich, dass das Polyol entweder ein einzelnes oder ein Gemisch von Polyolen sein kann. Ebenso können bei der Umsetzung der mindestens zweiwertige Linkergruppe zwei oder mehr Arten von Linkergruppe selbstverständlich gleichzeitig oder auch nacheinander zugegeben werden. Das Polyol enthält bevorzugt mindestens zwei primäre OH-Gruppen, d.h. OH-Gruppen, die an ein Kohlenstoffatom gebunden sind, an das zwei Wasserstoffe gebunden sind. Mit anderen Worten enthält das Polyol bevorzugt mindestens zwei Teilstrukturen der folgenden Formel: 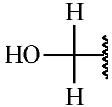 d.h. es enthält bevorzugt mindestens zwei -CH₂OH Gruppen.

Die mindestens zweiwertige Linkergruppe ist bevorzugt ein aliphatisches Polyol, bevorzugt ein aliphatisches C₂₋₄₀ Polyol, wobei das aliphatische C₂₋₄₀ Polyol bevorzugt ein aliphatisches Alkandiol, Alkantriol, Alkantetraol, Alkanpentaol oder Alkanhexaol ist.

Bevorzugte Beispiele von Linkergruppen beinhalten Ethandiol, Propandiole, Butandiole, Pentandiole, Hexandiole, Heptandiole, Octandiole, Nonandiole, Decandiole, Undecandiole, Dodecandiole, Tridecandiole, Tetradecandiole, Propantriole, Butantriole, Pentantriole, Hexantriole, Heptantriole, Octantriole, Nonantriole, Decantriole, Undecantriole, Dodecantriole, Tridecantriole, Tetradecantriole, Butantetrole, Pentantetrole, Hexantetrole, Heptantetrole, Octantetrole, Nonantetrole, Decantetrole, Undecantetrole, Dodecantetrole, Tridecantetrole, Tetradecantetrole, Pentanpentole, Hexanpentole, Heptanpentole, Octanpentole, Nonanpentole, Decanpentole, Undecanpentole, Dodecanpentole, Tridecanpentole, Tetradecanpentole, Hexanhexole, Heptanhexole, Octanhexole, Nonanhexole, Decanhexole, Undecanhexole, Dodecanhexole, Tridecanhexole, Tetradecanhexole, Heptanheptole, Octanheptole, Nonanheptole, Decanheptole, Undecanheptole, Dodecanheptole, Tridecanheptole, Tetradecanheptole, Octanoctole, Nonanoctole, Decanoctole, Undecanoctole, Dodecanoctole, Tridecanoctole, Tetradecanoctole, Nonannonole, Decannonole, Undecannonole, Dodecannonole, Tridecannonole, Tetradecannonole, Decandecole, Undecandecole, Dodecandecole, Tridecandecole, Tetradecandecole, Undecanundecole, Dodecanundecole, Tridecanundecole, Tetradecanundecole, Dodecandodecole, Tridecandodecole, Tetradecandodecole, Tridecantridecole, Tetradecantridecole und Tetradecantetradecole. Es versteht sich, dass die Kohlenstoffketten in diesen Polyolen linear oder verzweigt sein können. Optional können sie auch cyclisch sein, insbesondere bei Kohlenstoffketten mit mehr als 4 Kohlenstoffen.

Stärker bevorzugte Beispiele von Linkergruppen beinhalten Ethandiol, Propandiole, Butandiole, Pentandiole, Hexandiole, Heptandiole, Octandiole, Propantriole, Butantriole, Pentantriole, Hexantriole, Heptantriole, Octantriole, Butantetrole, Pentantetrole, Hexantetrole, Heptantetrole, Octantetrole, Pentanpentole, Hexanpentole, Heptanpentole und Octanpentole. Es versteht sich, dass die Kohlenstoffketten in diesen Polyolen linear oder verzweigt sein können. Optional können sie auch cyclisch sein, insbesondere bei Kohlenstoffketten mit mehr als 4 Kohlenstoffen.

Es versteht sich, dass beliebige Gemische der genannten Polyole verwendet werden können. Außerdem sind auch Polyole umfasst, die durch Veretherung einer oder mehrerer der obigen Polyole erhalten werden. Diese sind bevorzugt Verbindungen, in denen 2 bis 10, vorzugsweise 2 bis 6, der hierin genannten Polyole miteinander verethert sind. Beispielsweise können Diole durch Veretherung (kann auch als Oligomerisierung durch Veretherung bezeichnet werden) von 2 bis 6 Diolen, wie beispielsweise 2 bis 6 Molekülen Ethandiol (HOC₂H₄OH) unter Erhalt von HO(C₂H₄O)ₙH mit n als einer Zahl von 2 bis 6, erhalten werden.

In allen Beispielen von Linkergruppen sind Hydroxygruppen so angeordnet, dass nicht mehr als eine Hydroxygruppe pro Kohlenstoff vorhanden ist. Optional kann eine >CHOH Gruppe durch eine >C(=O) Gruppe ersetzt sein (d.h. beispielsweise die Alkoholgruppe zu einer Aldehyd- oder Ketogruppe oxidiert sein). In diesem Fall sind selbstverständlich auch die daraus durch Ringschluss gebildeten Acetale und Halbacetale mit umfasst. Darüber hinaus können eine oder mehrere Hydroxygruppen in den Linkergruppen z.B. mit C₁ bis C₃₀-Alkanol(en) verethert sein, vorausgesetzt, dass für die Reaktion mit der/den Hydroxycarbonsäure(n) mindestens zwei Hydroxygruppen verbleiben. Weiterhin können beliebige zwei Hydroxygruppen unter Bildung eines zyklischen Ethers verbunden sein. Außerdem können die Polyole bevorzugt sekundäre und/oder tertiäre Aminogruppen enthalten, da diese bei der Polyurethansynthese als Katalysatoren wirken können. Durch Verwendung von stickstoffhaltigen und/oder phosphorhaltigen Polyolen können darüber hinaus Polyurethane mit Flammschutzmitteleigenschaften erhalten werden. Darüber hinaus können auch Silangruppen enthaltende Polyole als sogenannte Hybride eingesetzt werden. Ebenfalls sind Polyole mit ein oder mehreren ionischen Gruppen wie NH₄⁺, PO₄³⁻, SO₄²⁻ pro Molekül interessant, da somit hydrophile und gut wassermischbare Produkte erhalten werden können.

Die mindestens zweiwertige Linkergruppe ist weiter bevorzugt ein aliphatisches α-ω-Alkandiol, α-ω-Alkantriol, α-ω-Alkantetraol, α-ω-Alkanpentaol oder α-ω-Alkanhexaol. Beispiele von aliphatischen α-ω-Alkandiolen, α-ω-Alkantriolen, α-ω-Alkantetraolen, α-ω-Alkanpentaolen und α-ω-Alkanhexaolen beinhalten insbesondere 1,2-Ethandiol, 1,3-Propandiol, 1,4-Butandiol, 1,5-Pentandiol, 1,6-Hexandiol, 1,7-Heptandiol, 1,8-Octandiol, 1,9-Nonandiol, 1,10-Decandiol, 1,11-Undecandiol, 1,12-Dodecandiol, 1,13-Tridecandiol, 1,14-Tetradecandiol, Glycerin, α-ω-Butantriole, α-ω-Pentantriole, α-ω-Hexantriole, α-ω-Heptantriole, α-ω-Octantriole, α-ω-Nonantriole, α-ω-Decantriole, α-ω-Undecantriole, α-ω-Dodecantriole, α-ω-Tridecantriole, α-ω-Tetradecantriole, α-ω-Butantetrole, α-ω-Pentantetrole, α-ω-Hexantetrole, α-ω-Heptantetrole, α-ω-Octantetrole, α-ω-Nonantetrole, α-ω-Decantetrole, α-ω-Undecantetrole, α-ω-Dodecantetrole, α-ω-Tridecantetrole, α-ω-Tetradecantetrole, α-ω-Pentanpentole, α-ω-Hexanpentole, α-ω-Heptanpentole, α-ω-Octanpentole, α-ω-Nonanpentole, α-ω-Decanpentole, α-ω-Undecanpentole, α-ω-Dodecanpentole, α-ω-Tridecanpentole, α-ω-Tetradecanpentole, α-ω-Hexanhexole, α-ω-Heptanhexole, α-ω-Octanhexole, α-ω-Nonanhexole, α-ω-Decanhexole, α-ω-Undecanhexole, α-ω-Dodecanhexole, α-ω-Tridecanhexole und α-ω-Tetradecanhexole. Dabei bedeutet, wie dem Fachmann bekannt ist, "α-ω", dass sich am Ende und am Anfang der längsten Kette von Kohlenstoffatomen jeweils eine OH-Gruppe befindet. Es versteht sich, dass die Kohlenstoffketten in diesen Polyolen linear oder verzweigt sein können. Optional können sie auch cyclisch sein, insbesondere bei Kohlenstoffketten mit mehr als 4 Kohlenstoffen.

Bevorzugte Beispiele von Polyolen beinhalten 1,2-Ethandiol, 1,3-Propandiol, 1,4-Butandiol, 1,5-Pentandiol, 1,6-Hexandiol, 1,7-Heptandiol, 1,8-Octandiol, 1,9-Nonandiol, 1,10-Decandiol, 1,11-Undecandiol, 1,12-Dodecandiol, 1,13-Tridecandiol, 1,14-Tetradecandiol, Glycerin, α-ω-Butantriole, α-ω-Pentantriole, α-ω-Hexantriole, α-ω-Heptantriole, α-ω-Octantriole, α-ω-Nonantriole, α-ω-Decantriole, α-ω-Undecantriole, α-ω-Dodecantriole, α-ω-Tridecantriole, α-ω-Tetradecantriole,

Weiter bevorzugte Beispiele von Polyolen beinhalten 1,2-Ethandiol, 1,3-Propandiol, 1,4-Butandiol, 1,5-Pentandiol, 1,6-Hexandiol, 1,7-Heptandiol, 1,8-Octandiol, 1,9-Nonandiol, 1,10-Decandiol, 1,11-Undecandiol, 1,12-Dodecandiol, 1,13-Tridecandiol, 1,14-Tetradecandiol, sowie α-ω-Pentantriole und α-ω-Hexantriole.

Noch weiter bevorzugte Beispiele von Polyolen beinhalten 1,3-Propandiol, 1,4-Butandiol, 1,5-Pentandiol, 1,6-Hexandiol, sowie α-ω-Pentantriole und α-ω-Hexantriole.

Es ist bevorzugt, dass die eine oder mehreren mindestens zweiwertigen Linkergruppen aus Alkandiolen, Alkantriolen, Alkantetraolen, und Kombinationen davon ausgewählt sind. Stärker bevorzugt sind die eine oder mehreren mindestens zweiwertigen Linkergruppen aus Alkandiolen, Alkantriolen und Kombinationen davon ausgewählt. Noch stärker bevorzugt sind hierbei die eine oder mehreren mindestens zweiwertigen Linkergruppen aus 2-Butyl-2-ethyl-1,3-propandiol, 2-Ethyl-2-(hydroxymethyl)1,3-propandiol, 1,2-Propandiol, 1,3-Propandiol, 1,4-Butandiol, 1,3-Butandiol, 2-Methyl-1,3-propandiol, 1,5-Pentandiol, sowie deren Kombinationen ausgewählt.

Beispiele von Polyaminen sind 1,2-Diaminocyclohexan, 4,4'-Diaminodiphenylsulfon, 1,5-Diamino-2-methylpentan, Diethylentriamin, Hexamethylendiamin, Isophorondiamin, Triethylentetramin und Trimethylhexamethylendiamin.

Beispiele von (Poly)amino(poly)alkoholen sind 2-Aminoethanol, Diethanolamin, Triethanolamin, 2-Amino-2-methylpropanol, 2-(Dimethylamino)ethanol, 2-(2-Aminoethoxy)ethanol, Methyldiethanolamin, 4-Amino-1-butanol, (S)-3-Amino-1,2-propandiol, 1-Aminopropan-2-ol, 2-Amino-2-methyl-1,3-propandiol und Polyaminosaccharide wie Chitin (N-Acetylierungsgrad bevorzugt geringer als 90%) und insbesondere Chitosan, sowie Aminosaccharide wie Glucosamin. Bevorzugte (Poly)amino(poly)alkohole aus nachwachsenden Quellen sind Polyaminosaccharide wie Chitosan und Aminosaccharide wie Glucosamin.

Besonders bevorzugt ist die mindestens zweiwertige Linkergruppe ausgewählt aus 1,3-Propandiol, 2-Methyl-1,3-propandiol, 2,2-Dimethyl-1,3-propandiol, 2-Ethyl-2-hydroxymethyl-1,3-propandiol, 1,6-Hexandiol, Ethanolamin und Diethanolamin. Noch stärker bevorzugt ist die mindestens zweiwertige Linkergruppe ausgewählt aus 1,3-Propandiol, 2-Methyl-1,3-propandiol, 2,2-Dimethyl-1,3-propandiol, 2-Ethyl-2-hydroxymethyl-1,3-propandiol und 1,6-Hexandiol.

Es ist bevorzugt, dass die mindestens zweiwertige Linkergruppe nicht Glycerin ist.

### Das Hydroxyfettsäurekondensat oder das Gemisch von Hydroxyfettsäurekondensaten

Hierin offenbart, jedoch nicht als solches beansprucht, ist ein Hydroxyfettsäurekondensat oder ein Gemisch von Hydroxyfettsäurekondensaten, das durch das Verfahren gemäß der Erfindung erhältlich ist. Der Begriff Hydroxyfettsäurekondensat wie hierin verwendet kann sich auf ein einzelnes oder eine Mehrzahl von Hydroxyfettsäurekondensaten beziehen.

Es versteht sich, dass diese Hydroxyfettsäurekondensate durch Veresterung bzw. Amidierung der oben genannten Polyole, Polyamine und/oder (Poly)amino(poly)alkohole mit den oben genannten Hydroxyfettsäuren gebildet werden. Die Hydroxyfettsäurekondensate weisen daher die sich daraus ergebenden chemischen Strukturen auf.

Vorzugsweise werden alle in dem Polyol enthaltenen primären Hydroxygruppen (auch primäre Alkohole, bzw. -CH₂OH-Gruppen genannt) mit jeweils einer Hydroxyfettsäure verestert. Stärker bevorzugt werden alle in dem Polyol enthaltenen Hydroxygruppen mit jeweils einer Hydroxyfettsäure verestert. Es ist bevorzugt, dass mindestens zwei der im Polyol enthaltenen Hydroxygruppen mit jeweils einer Hydroxyfettsäure verestert werden.

Infolgedessen weist ein Hydroxyfettsäurekondensat Molekül bevorzugt jeweils mindestens zwei Estergruppen (bzw. eine Ester- und eine Amidgruppe, bzw. zwei Amidgruppen) auf, über die die mindestens zwei Hydroxyfettsäurereste an das Polyol, das Polyamin und/oder den (Poly)amino(poly)alkohol gebunden sind. Alternativ können bevorzugte Hydroxyfettsäurekondensate nur eine Estergruppe oder eine Amidgruppe aufweisen, über die ein Hydroxyfettsäurerest an das Polyol, das Polyamin und/oder den (Poly)amino(poly)alkohol gebunden ist.

Schematisch lässt sich dies im Falle von zwei Hydroxyfettsäuren und einem Diol wie folgt darstellen: 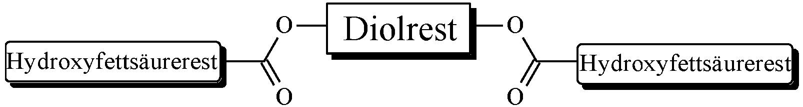 sowie im Falle von drei Hydroxyfettsäuren und einem Triol wie folgt darstellen: 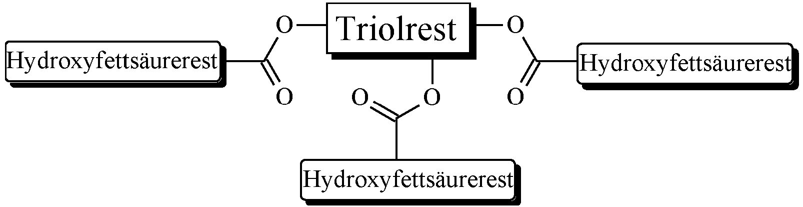 wobei "Hydroxyfettsäurerest" für eine Teilstruktur steht, die einer Hydroxyfettsäure (bevorzugt Monohydroxyfettsäure) ohne Carboxylgruppe entspricht, und wobei "Diolrest" für ein Diol ohne die beiden Hydroxygruppen steht, sowie "Triolrest" für ein Triol ohne die drei Hydroxygruppen steht.

Bevorzugt weisen die erhaltenen Hydroxyfettsäurekondensate eine durchschnittliche OH-Funktionalität pro Molekül von mindestens 1 auf. Hydroxyfettsäurekondensate mit einer OH-Funktionalität von exakt 1 bilden in einem verzweigten Polyurethan-Gerüst abschließende, Ketten beendende Bindungen aus. Solche, monofunktionalen Moleküle werden auch als Weichmacher oder Seitenketten-Weichteile bezeichnet. Höhere OH- Funktionalitäten erhöhen die Fähigkeit des jeweiligen Moleküls über zusätzliche Urethanbindungen ein Gerüst weiter auszubauen und/oder zu vernetzen.

Die durchschnittliche OH-Funktionalität pro Molekül lässt sich durch dem Fachmann bekannte Verfahren bestimmen, z.B. durch die Bestimmung der mit Essigsäureanhydrid veresterbaren OH-Gruppen, wobei das Molekulargewicht (Mₙ) z.B. durch Gelpermeationschromatographie ermittelt werden kann. Bevorzugt kann die durchschnittliche OH-Funktionalität pro Molekül gemäß DIN EN ISO 4629-2:2016-12 durchgeführt werden.

Die durchschnittliche OH-Funktionalität pro Molekül bezieht sich auf mol OH-Gruppen/mol Hydroxyfettsäurekondensat. Ein durch Veresterung eines Diols mit zwei Hydroxyfettsäuen erhaltenes Hydroxyfettsäurekondensat weist somit eine durchschnittliche OH-Funktionalität pro Molekül von exakt 2 auf.

Weiter bevorzugt weisen die erhaltenen Hydroxyfettsäurekondensate eine durchschnittliche OH-Funktionalität pro Molekül von 1,5 bis 2,5, bevorzugt 1,7 bis 2,3, stärker bevorzugt 1,8 bis 2,2, noch stärker bevorzugt 1,9 bis 2,1, noch stärker bevorzugt 1,95 bis 2,05 auf. Derartige Hydroxyfettsäurekondensate bilden mit Diisocyanaten schmelzbare Polymere, sog. TPU's, die in Heißklebern, extrudierbaren Thermoplasten und als thermoplastischer Anteil von Verbundwerkstoffen Verwendung finden. Die erfindungsgemäß erhaltenen TPUs weisen einen deutlich verringerten Anteil an gelartigen Oligomeren auf, die bei den etablierten Produkten dieser Art häufig das Schmelzverhalten problematisch verschlechtern.

Besonders vorteilhaft kommen präzise eingestellte OH-Funktionalitäten bei hoch schmelzenden TPU's zum Tragen: Extrem langkettige, unverzweigte und gleichmäßig in ihrer mittleren Kettenlänge aufgebaute, lineare Polymere ergeben einen Thermoplasten mit homogenen und eng eingestellten Verarbeitungseigenschaften. Die sonst üblichen Schwankungen in der Erweichungstemperatur und dem *Melt-Flow-Index* werden so vorteilhaft vermieden und erlauben eine präzise, einfachere und schnellere Verarbeitung, da eine komplexe Regelung zur Kompensation schwankender TPU-Eigenschaften nicht mehr notwendig ist.

Eine bevorzugte Schwankung bis zu ±0,1 in der durchschnittlichen OH-Funktionalität pro Molekül erlaubt das Einbauen der biobasierten Komponente in einfache, robuste und kostengünstige Polymerisationsprodukte. Eine bevorzugte Schwankung von bis zu ±0,05 in der durchschnittlichen OH-Funktionalität pro Molekül ermöglicht gleichmäßigere und besser einstellbare Volumeneigenschaften in darüber zugänglichen Polymerisations-Produkten. Eine bevorzugte Schwankung von bis zu ±0,011 in der durchschnittlichen OH-Funktionalität pro Molekül bietet durch eng eingestellte Eigenschaften der Komponente einen Zugang zu präzise regelbaren Volumeneigenschaften.

Bei anderen Anwendungen kann es jedoch auch erwünscht sein, dass ein gewisser Grad an Vernetzung auftritt. In diesem Fall weisen die erhaltenen Hydroxyfettsäurekondensate eine durchschnittliche OH-Funktionalität pro Molekül von 2,0 bis 7, besonders bevorzugt 2,5 bis 3, auf. Bei einer OH-Funktionalität von 2 und mehr wird die biobasierte Komponente in einem Polyurethan-Gerüst Vernetzungen weiterbilden und die mittlere Molekülmasse des so zugänglichen Polymerisats ansteigen lassen. Eine durchschnittliche OH-Funktionalität von deutlich über 2 wirkt quervernetzend und verfestigt ein Polyurethan-Polymerisat zunehmend. Bevorzugt weist die modifizierte, biobasierte Komponente der Polyurethan-Reaktivmasse eine OH-Funktionalität pro Molekül von 2,69 bis 2,71 auf. Eine so durch enzymatische H₂O-Addition eingestellte Hydroxy-Funktionalität bietet - vorteilhaft in Kombination mit einer mittleren Molmasse um 950 g/mol mit 10% Schwankung - eine Kombination von verlängernden und quervernetzenden OH-Gruppen, welche sehr stabile und gut belastbare Polyurethan-Polymere zugänglich macht. Besonders bevorzugt weisen die erfindungsgemäß eingesetzten Komponenten - bevorzugt in Kombination zu der vorbeschriebenen OH-Funktionalität - eine Reinheit von 99 Gewichtsprozent auf. Dadurch werden Polyurethan-Polymere in ihren Werkstoffeigenschaften gleichmäßiger, qualitativ wertiger und können auch optisch in Bereichen regelmäßiger Belastung dauerhaft und langlebig die gewünschte Funktion bei stabilem Erscheinungsbild bereitstellen.

Es versteht sich, dass das Hydroxyfettsäurekondensat oder das Gemisch von Hydroxyfettsäurekondensaten auch als Ausgangsstoff für Polyether, Polyester, Polymere, Ester, Öle, Schmierstoffe, rheologiemodifizierende Additive, Weichmacher, Kosmetika, Wachse oder Lacke dienen kann.

### Das Polyurethan

Weiterhin betrifft die vorliegende Erfindung ein Polyurethan, das durch Umsetzung einer Zusammensetzung umfassend das Hydroxyfettsäurekondensat oder das Gemisch von Hydroxyfettsäurekondensaten mit einem Diisocyanat, Triisocyanat, Tetraisocyanat oder sonstigen für die Herstellung von Polyurethanen üblichen Polyisocyanaten erhältlich ist.

Es versteht sich, dass zusätzlich zu dem durch das Verfahren der vorliegenden Erfindung hergestellten Hydroxyfettsäurekondensat oder Gemisch von Hydroxyfettsäurekondensaten auch andere Hydroxyfettsäurekondensate oder Gemische von Hydroxyfettsäurekondensaten bei der Herstellung des Polyurethans eingesetzt werden können. Bevorzugt werden zu mindestens 30 Gew.-%, stärker bevorzugt mindestens 50 Gew.-%, noch stärker bevorzugt mindestens 70 Gew.-% des durch das Verfahren der vorliegenden Erfindung hergestellten Hydroxyfettsäurekondensats oder Gemischs von Hydroxyfettsäurekondensaten, bezogen auf das Gesamtgewicht der Polyolkomponente, bei der Herstellung des Polyurethans eingesetzt.

Im Falle der Umsetzung eines mit zwei Monohydroxyfettsäuren veresterten Diols mit einem Diisocyanat lässt sich das Polyurethanprodukt beispielsweise wie folgt darstellen: 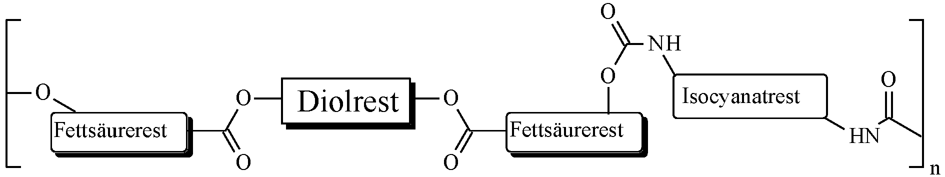 wobei "Fettsäurerest" für eine Teilstruktur steht, die einer Fettsäure ohne Carboxylgruppe entspricht, und wobei "Diolrest" für ein Diol ohne die beiden Hydroxygruppen steht, sowie "Isocyanantrest" für eine Diisocyanatverbindung ohne die beiden NCO-Gruppen steht. Dabei gibt "n" die Anzahl von Wiederholungseinheiten in dem Polyurethan an. Im Falle eines hier gezeigten linearen Polyurethans kann n beispielsweise im Bereich von 10 bis 1000, insbesondere 50 bis 200, liegen. Jedoch können diese Werte auch wesentlich höher liegen, insbesondere bei verzweigten Polyurethanen, sodass n in den Polyurethanen der vorliegenden Erfindung nicht besonders eingeschränkt ist und vom Fachmann basierend auf dem allgemeinen Fachwissen geeignet eingestellt werden kann, eine Charakterisierung der Polyurethane auf Basis des Polymerisationsgrades jedoch nicht relevant ist.

Wie für den Fachmann erkenntlich ist, können durch Variation der Polyole, Hydroxyfettsäure sowie der Isocyanate zahlreiche lineare sowie verzweigte, bzw. vernetzte Polyurethanprodukte hergestellt werden. Um Verzweigungen einzuführen, kann beispielsweise ein Teil der Diole durch höherwertige Polyole, wie Triole oder Tetraole, ersetzt werden, und/oder ein Teil der Monohydroxyfettsäuren durch Di- oder Trihydroxyfettsäuren ersetzt werden und/oder ein Teil der Diisocyanate durch Tri- oder Tetraisocyanate ersetzt werden.

Geeignete Diisocyanate, Triisocyanate und Tetraisocyanate sind dem Fachmann bekannt. Als Diisocyanate kommen beispielsweise aliphatische, cycloaliphatische, aryliphatische, heterocyclische und aromatische Diisocyanate in Betracht, wie sie in Justus Liebigs Annalen der Chemie (1949), 562, S. 75-136 beschrieben werden. Bevorzugt sind aliphatische und cycloaliphatische Diisocyanate.

Aliphatische Diisocyanate umfassen beispielsweise Hexamethylendiisocyanat, insbesondere 1,6-Hexamethylendiisocyanat.

Cycloaliphatische Diisocyanate umfassen beispielsweise Isophorondiisocyanat. 1,4-Cyclohexan-diisocyanat, 1-Methyl-2,4-cyclohexan-diisocyanat und 1-Methyl-2,6-cyclohexandiisocyanat sowie die entsprechenden Isomerengemische, 4,4'-Di-cyclohexylmethandiisocyanat, 2,4'-Dicyclohexylmethandiisocyanat und 2,2'-Dicyclohexylmethan-diisocyanat sowie die entsprechenden Isomerengemische. Ein bevorzugtes cycloaliphatisches Diisocyanat ist Isophorondiisocyanat.

Aromatische Diisocyanate umfassen beispielsweise 2,4-Toluylendiisocyanat, Gemische aus 2,4-Toluylendiisocyanat und 2,6-Toluylendiisocyanat, 4,4'-Diphenylmethandiisocyanat, 2,4'-Diphenylmethandiisocyanat und 2,2'-Diphenylmethandiisocyanat, Gemische aus 2,4'-Diphenylmethandiisocyanat und 4,4'-Diphenylmethandiisocyanat, urethanmodifizierte flüssige 4,4'-Diphenylmethandiisocyanate und 2,4'-Diphenylmethandiisocyanate, 4,4'-Diisocyanatodiphenyl-ethan-(1,2) und 1,5-Naphthylendiisocyanat.

Beispiele für geeignete aromatische asymmetrische Diisocyanate sind alle Isomeren des Toluylendiisocyanats (TDI) entweder in isomerenreiner Form oder als Mischung mehrerer Isomere, Naphthalin-1,5-diisocyanat (NDI), Naphthalin-1,4-diisocyanat (NDI), Diphenylmethan-2,4'-diisocyanat (MDI) sowie Mischungen des 4,4'-Diphenylmethandiisocyanats mit dem 2,4'-MDI-lsomeren und 1,3-Phenylendiisocyanat. Beispiele für aliphatische asymmetrische Diisocyanate sind 1,6-Diisocyanato-2,2,4-trimethylhexan, 1,6-Diisocyanato-2,4,4-trimethylhexan und Lysindiisocyanat.

Beispiele für bevorzugte Isocyanate sind monomere MDIs, polymere MDI, TDIs, HDI- und IPDI-Isomere insbesondere auch polymerisierte Varianten wie bspw. Isocyanurate aus HDI oder IPDI (Isophorondiisocyanat) und auch Gemische daraus sowie daraus erhältliche Präpolymere.

Asymmetrische Polyisocyanate können bei der stufenweisen Herstellung von Polyurethanen von besonderem Interesse sein, da sich durch ihre Verwendung, unter Reaktion der reaktiveren Isocyanatgruppe Präpolymere (im Fall der Verwendung von asymmetrischen Diisocyanaten) herstellen lassen, die in einem weiteren Schritt mit weiteren Polyolen zu den gewünschten Polyurethanen umgesetzt werden können, bzw. (im Fall der Verwendung von asymmetrischen Triisocyanaten) zunächst thermoplastische lineare Polyurethane bilden lassen, die durch Zugabe weiterer Polyole vernetzt werden können. Die Verwendung von asymmetrischen Polyisocyanaten ist beispielsweise aus der EP 1 765 900 A1 bekannt.

Die zur Bildung von Polyurethanen aus Polyalkoholen und Polyisocyanaten geeigneten Reaktionsbedingungen sind dem Fachmann hinlänglich bekannt (siehe Oertel, Becker, Braun, Kunststoffhandbuch - Band 7 Polyurethane, 1993; J.H. Saunders, K.C. Frisch Polyurethanes: Chemistry and Technology Part II, 1964; C. Defonseka, Flexible Polyurethane Foams: A Practical Guide, 2019; M. Szycher Syzchers Handbook of Polyurethanes 2017; und U. Meier-Westhues, K. Danielmeier, Polyurethanes: Coatings, Adhesives and Sealants 2019).

Beispielsweise können 100 Massenteile Hydroxyfettsäurekondensat (oder Gemische von Hydroxyfettsäurekondensaten) auf 70°C bis 100°C erwärmt werden, 0,5 bis 5 Massenteile Molekularsieb 3Å sowie 0,001 bis 0,5 Massenteile eines Katalysators (z.B. eine Bismutverbindung) zugegeben werden und dieses Gemisch nach Homogenisierung mit einem Diisocyanat, Triisocyanat, Tetraisocyanat, Pentaisocyanat oder einer Kombination davon versetzt werden, wobei das Verhältnis der Isocyanatgruppen zu Hydroxygruppen bevorzugt bei 1,0 bis 1,1 (insbesondere bei etwa 1,05) liegt. Dieses Gemisch kann nach erfolgtem Mischen in eine gewünschte Form gegossen und bei einer Temperatur von 70°C bis 100°C ausgehärtet werden.

In einem konkreten Beispiel werden 100 g Hexane-1,6-diyl bis (10-hydroxyoctadecanoat) (OH-Zahl 164) aufgeschmolzen und bei 85 °C mit 1,5 Gew.-% Molekularsieb 3 A (UOP L Powder) versetzt sowie 0,2 Gew.-% Byk A 535 und 0,03 Gew.-% eines Bismuth-Katalysators (bspw. Borchi Kat 315 EU). Diese Mischung wird unter Vakuum 2 min bei 1000 U/min homogenisiert und entgast. Dann werden 58,3 g eines HDI-basierten difunktionellen Diisocyanates hinzugefügt (bspw. Desmodur N3400, NCO-Gehalt: 21,8 Gew.-%), wobei das Verhältnis von NCO- zu OH-Gruppen 1,05 beträgt. Diese Mischung wird 30 sec unter Vakuum bei 1000 U/min vermischt und dann bei 85 °C in eine Rechteckform gegossen und bei dieser Temperatur ausgehärtet. Nach 10 min bei 85 °C kann entformt werden. Man erhält einen transparenten Polyurethankörper, dessen physikalischen Endeigenschaften nach 7 Tagen Lagerung bei 23 °C und 50 % rF erreicht werden.

Sofern ein späteres, ungewolltes Freisetzen oder Abreagieren eines lokalen Isocyanat-Überschusses vermieden werden soll oder eine Koppelung des Produkts in Polyestersysteme erwünscht ist, kann in der Reaktivmischung die Menge an Isocyanaten etwas kleiner gehalten werden als das stöchiometrische Äquivalent relativ zu den verfügbaren OH-Gruppen der hydratisierten Carbonsäuren. Üblicherweise ist jedoch ein Verhältnis der Isocyanatgruppen zu Hydroxygruppen von 1,0 bis 1,1 bevorzugt.

In bevorzugter Ausführungsform umfasst eine Polyurethan-Reaktivmasse neben dem erfindungsgemäßen Gegenstand mehrere Komponenten. Die Komponenten umfassen mindestens einen Alkohol, mindestens ein Isocyanat, Rest Stell- und Hilfs-Stoffe sowie unvermeidbare Verunreinigungen.

Bevorzugt erfolgt die Herstellung der erfindungsgemäßen Polyurethane als Ein-Topf-Prozess. Dabei können Stell- und Hilfs-Stoffe, wie zum Beispiel mineralische und/oder anorganische Füllstoffe, wie z.B. Kreide, Tonerde, Kieselgur, Holz-Zellulose-Fasern oder auch Polyester-Füllkörper und -Folien, chemisch in die Polyurethan-Struktur eingebunden werden. Dies kann zur Zugabe dieser Stell- und Hilfs-Stoffe vor, während, oder nach der Polymerisation erfolgen.

Wie für den Fachmann ersichtlich ist, können durch die vorliegende Erfindung aus vorwiegend nachwachsenden Rohstoffen Polyurethane mit ausgezeichneten Produkteigenschaften hergestellt werden, die den bislang verwendeten petrochemisch erhaltenen Polyurethanen zumindest ebenbürtig sind. Darüber hinaus ermöglicht es das vorliegend beschriebe Verfahren erstmalig, diese Polyurethane mit wirtschaftlich vertretbarem Aufwand herzustellen, wodurch zu erwarten ist, dass endlich der Bedarf an kosteneffizienten biobasierten Polyurethanen gedeckt werden kann. Darüber hinaus ist es denkbar, dass die biologische Abbaubarkeit der vorliegend erhaltenen Polyurethane (z.B. mittels Enzymen) gegenüber bislang erhältlichen Polyurethanen deutlich verbessert ist. Die liegt u.a. an der Estergrundstruktur, die für zahlreiche Enzyme als Ausgangsstoff geeignet ist.

Die vorliegende Erfindung wird unter Bezugnahme auf die folgenden Beispiele näher beschrieben, ohne jedoch auf diese Beispiele beschränkt zu sein.

### Beispiele

### Beispiel 1: Herstellung des Sn-Ohy Rohextrakts

Ein Sn-Ohy Rohextrakt wurde verwendet, in dem Sn-Ohy mit der in Uniprot als A0A172MLH9 hinterlegten Sequenz verwendet wurde, mit dem einzigen Unterschied, dass neben dieser Sequenz von Sn-Ohy in pET28(a) außerdem ein N-terminaler His₆-Tag (pET28-Sn-Ohy(N)His₆) mit der Sequenz ATGGGCAGCAGCCATCATCATCATCATCACAG CAGCGGCCTGGTGCCGCGCGGCAGCCAT (SEQ ID NO.3) angefügt wurde, womit sich eine Nucleotidsequenz von 2022 bp ergab (SEQ ID NO. 1):

ATGGGCAGCAGCCATCATCATCATCATCACAGCAGCGGCCTGGTGCCGCGCGGCAGCCATAT
GGAAGAAGTGAGTTATCCCAAAGCTGGACCGAGCATTGAAGCGAACGTAGGGGATGGGCACTGGC
GAAAGGGGCCCTCGGATACGCTGCCGCCTCCGGACACTGTTGGACCCTATATGCGCAACCGCCCCC
TGCCTGTGGATCAAGTGGAAGGCAGGAAAGCATGGATCATCGGAAGTGGAATCGCGGGTCTGGCC

TCTGCCTTTTACTTGATCCGCGACGGGCGGATGAAGGGGCAGGACATAACCATCCTCGATGCCGTG
GGCACTCCAGGCGGATCACTGGACGGCTCAGGGAACGCCGAAGATGGCTACCTGATCCGAGGCGG
GCGCGAGATGAACTGGAACTACGATCACTTTTGGGATCTCTTCCAGGACATTCCCGCGCTGGAGTA
CCCGTCCCCTTACTCGGTCTTGGATGAGTATCGGGCGGTGAACGACAATGATCCTAATTGGTCCAA
GTCCCGATTGATGCACAAGCAAGGCCAAATTCGGGATTTCAGCACCTTGGGGCTTTCTTCCGCCCA
CCAATGGGAATTGATCAAGCTTCTCCTGAAGCGCAAGGAGGACCTCGATGACATCACCATCGAACA
GTACTTCAGCGATAGCTTTCTGGAGACCAACTTCTGGTACCTCTGGCGCTCGATGTTTGCGTTCCAG
AACTGGCAAAGTCTGCTGGAAGTGAAGTTGTACATGCATCGCTTTTTGGATGCAATCGACGGCTTG
ACGGATATGTCAGCGCTCGTGTTCCCAAAATACAACCAGTACGACAGCTTCGTCGTCCCCCTGGTC
AACTACCTCAAGGGCCAAGGCGTCAACGTAGAATTCGGCACGCGCGTCTACGACCTGGACATGAC
GGACAACAACGGCGAGCGTACCGTGACCTCCATTCTTGCGAAGGTAGACGGGCGGGATCAGAAGA
TTGACATCGGCGCGAAGGACGTGGTTTTTGCCCTGACTGGATCGATGACGGAGGGTACAGCCTACG
GCGATCTGGATACTGCTCCCGACCTCACTCGAGCCACCACGCCCCCTGGCGACTCAAGCGATTGGG
CGTTGTGGCAGAACCTGGCCAAGAAGTCCCACGTCTTTGGTAAGCCTGAAAAGTTCTGCGGGCAAC
CCAGTCGCTCGATGTGGGAGTCTGCCACCCTGACGTGCAAGCCTTCGCCGTTGACCGAGCGCCTCA
AAGATCTCTCAATCAATGACCCTTATTCGGGAAAAACGGTGACCGGTGGAATCATCACCTTTACCG
ACTCGAACTGGGTTCTCAGCTTCACCTGCAATCGTCAACCGCATTTCCCCACACAACCAGACGACG
TACTGGTGCTTTGGGTCTATGCCTTGGTCATGGACAGCAAAGGCAACCATGTACTAAAACCAATGC
CTGAGTGTACGGGCCGCGAAATTCTTGCTGAGCTTTGCTACCACCTCGGCATTGTGGATCAGGTGG
ATGAAGTGGCCAGACAGACCAAGGTTCGCCTTGCCCTGATGCCATTCATCACGGCTCAATTTATGC
CACGAGCTGCTGGAGATCGACCGCGTGTTGTTCCAGCCGGGTGCACCAATCTCGCTCTGCTGGGCC
AATTCGTGGAGACGTCTAATGACATCATCTTCACCATGGAGAGTTCCGTCAGGACTGCGCGGATTG
GCGTGTACACGCTTCTGGGGCTACGAAAGCAGGTCGCCGATATCAGCCCCACGCAATACGACGTCC
GAAATCTGATCAAGGGTGCTCGTGCCCTGAACAACAACGAGCCGTTCATGGGCGAGCGGCTGCTCC
ATCGACTGCTCGACAACACCTACTTCGCCCACATCCTCCCGCCGCTGCCAGCAGGAGACGGTGGAT
CCAGCGATCAAGCGGCAAGCTCGCGTATGAAGGCCAACCACACTGCGGCGGCGGCACTTGGAGCG
GTGTCTGATTGGATCCATCATGTTCGGGATAAACTGAAGCCGGGCGCCTGA

Die entsprechende Aminosäuresequenz, die 673 Aminosäuren und eine Masse von 75,0 kDa aufwies, war somit wie folgt (SEQ ID NO.2): 
MGSSHHHHHHSSGLVPRGSHMEEVSYPKAGPSIEANVGDGHWRKGPSDTLPPPDTVGPYMRNRPLP
VDQVEGRKAWIIGSGIAGLASAFYLIRDGRMKGQDITILDAVGTPGGSLDGSGNAEDGYLIRGGREMN
WNYDHFWDLFQDIPALEYPSPYSVLDEYRAVNDNDPNWSKSRLMHKQGQIRDFSTLGLSSAHQWELIK
LLLKRKEDLDDITIEQYFSDSFLETNFWYLWRSMFAFQNWQSLLEVKLYMHRFLDAIDGLTDMSALVF
PKYNQYDSFVVPLVNYLKGQGVNVEFGTRVYDLDMTDNNGERTVTSILAKVDGRDQKIDIGAKDVVF
ALTGSMTEGTAYGDLDTAPDLTRATTPPGDSSDWALWQNLAKKSHVFGKPEKFCGQPSRSMWESATL
TCKPSPLTERLKDLSINDPYSGKTVTGGIITFTDSNWVLSFTCNRQPHFPTQPDDVLVLWVYALVMDSK
GNHVLKPMPECTGREILAELCYHLGIVDQVDEVARQTKVRLALMPFITAQFMPRAAGDRPRVVPAGCT
NLALLGQFVETSNDIIFTMESSVRTARIGVYTLLGLRKQVADISPTQYDVRNLIKGARALNNNEPFMGER
LLHRLLDNTYFAHILPPLPAGDGGSSDQAASSRMKANHTAAAALGAVSDWIHHVRDKLKPGA
 wobei der fett gedruckte Teil den His-Tag enthält und die restliche Sequenz der in Uniprot als A0A172MLH9 hinterlegten Sequenz entspricht.

### Herstellung kompetenter E.coli Zellen

Zur Herstellung der Hauptkultur wurde von einer *E.coli* BL21(DE3) Vorkultur 250 µL in einen sterilen Kolben mit 25 mL LB-Medium überführt. Für die Vorkultur wurden 5 mL LB-Medium in sterile Reagenzgläser gegeben. Es wurde jeweils eine Einzelkolonie von einer Agarplatte mit *E.coli* BL21(DE3) Zellen isoliert und in ein Reagenzglas überführt. Die Vorkultur wurde über Nacht bei 37 °C und 180 rpm inkubiert. Die Hauptkultur wurde nach Erreichen von einer OD von 0,35 - 0,4 geerntet und auf 4 °C gekühlt. Die Zellen wurden in sterile Gefäße überführt und zentrifugiert (800x g und 4 °C für 15 Minuten). Der Überstand wurde verworfen. Das Zellpellet wurde in 5 mL Calciumchloridlösung (100 mM) resuspendiert und die Suspension 20 Minuten auf 4 °C gekühlt. Anschließend wurde die Suspension abzentrifugiert (800x g und 4 °C für 10 Minuten) und der Überstand verworfen. Die Zellen wurden in 500 µL einer Lösung aus Calciumchlorid (100 mM) und 15 %-iger wässriger Glycerinlösung resuspendiert. Die Resuspension wurde in sterile Gefäße (50 µL) aliquotiert und bei -80 °C gelagert.

### Transformation von pET28-Sn-Ohy(N)His6 in E.coli BL21(DE3)

Für die Transformation von pET28 Sn-Ohy in *E.coli* BL21(DE3) wurde das Plasmid (pET28-Sn-Ohy(N)His₆, 0,5 µL) zu den kompetenten Zellen (50 µL) gegeben und für 30 min bei 4 °C inkubiert. Nach erfolgtem Hitzeschock bei 42 °C für 90 s wurden die Zellen weitere 5 min auf bei 4 °C inkubiert. LB-Medium (1 mL) wurde zum Transformationsansatz ergeben und für 1-2 h bei 37 °C und 160-180 rpm inkubiert. Anschließend wurde die Zellsuspension auf Agarplatten mit dem entsprechenden Antibiotikum (Kanamycin 50 µg mL⁻¹) ausplattiert und für bei 37 °C für 18 h im Brutschrank inkubiert.

### Überexpression von Sn-Ohy in E.coli BL21(DE3)

Zur Überexpression von Sn-Ohy in *E.coli* BL21(DE3) wurden Vorkulturen in Reagenzgläsern angeimpft. Dafür wurde zu LB-Medium und Kanamycin 50 µg mL⁻¹ (und eine Kolonie des transformierten *E.coli* (*E.coli* BL21(DE3)-pET28-Sn-Ohy(N)His₆,) gegeben. Die Kulturen wurden für 17-21 h bei 37 °C und 170-180 rpm inkubiert.

Für die Hauptkulturen wurde TB-Medium und Kanamycin (50 µg mL⁻¹) in einem Erlenmeyerkolben vorgelegt und mit 1% der Vorkultur angeimpft. Die Kulturen wurden bei 37 °C und 180 rpm inkubiert. Bei einer optischen Dichte (OD) von 0,6-1,0 wurde mit IPTG (0,5 mM mL⁻¹) induziert und die Inkubationstemperatur auf 20 °C gesenkt. 18-20 h nach der Induktion wurden die Bakterien durch Zentrifugieren der Hauptkulturen (4000x·g, 4 °C, 30 min) pelletiert. Die Zellpellets wurden anschließend bei -20 °C gelagert.

### Zellaufschluss und Gewinnung des Rohextraktes

Für den Zellaufschluss wurden die Zellpellets in KPi-Puffer (50 mM, pH 6,3) resuspendiert (25%ige Zellsuspension). Die Zellen wurden mittels Ultraschall (3x 3-5 min, ca. 20% Leistung) bei 4 °C aufgeschlossen. Durch anschließendes Zentrifugieren (20 min, 20000x·g, 4 °C) des Zelllysates wurden die Zelltrümmer von dem löslichen Protein abgetrennt.

### Bradford-Assay

Zur Bestimmung der Proteinkonzentration wurde die Proteinlösung in unterschiedlichen Verdünnungen (1:10 bis 1:100, je 5 µL) und als Referenz die BSA-Standardreihe mit bekannten Proteinkonzentrationen (je 5 µL) auf die Mikrotiterplatte aufgetragen. Anschließend wurde Bradford-Reagenz (250 µL) hinzugegeben und nach einer Inkubationszeit von 15 min bei 25 °C die Absorption bei 595 nm gemessen. Die Konzentrationsmessung erfolgte als Dreifachbestimmung.

### Beispiel 2: Hydratisierung von Ölsäure

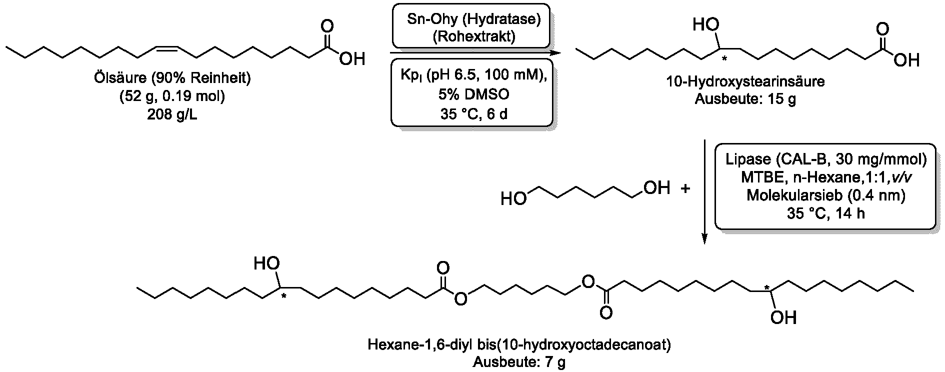

Ölsäure (90% Reinheit, 52 g, 0,18 mol) wurde mit DMSO (12,5 mL), 237,5 mL KPi-Puffer (pH 6,5, 100 mM), und 20 mL Sn-Ohy Rohextrakt (Aufschluss mittels Ultraschall, 25% Zellsuspension, 5x 5 min, 5x5 Zyklen, 20%; Proteinkonzentration des Rohextrakts 15 mg/mL) vorgelegt. Sn-Ohy ist eine Hydratase des Organismus *Stenotrophomonas nitritireducens* und wurde z. B. von Kim et al. 2010 beschrieben (Conversion of oleic acid to 10-hydroxystearic acid by whole cells of Stenotrophomonas nitritireducens, Biotechnology letters, vol 33, pp. 993-997, May 2010).

Die Reaktionslösung wurde bei 35 °C für sechs Tage gerührt, anschließend filtriert, mit MTBE gewaschen und aus MTBE abschließend umkristallisiert. Nach dem Trocknen wurden 15 g (0,05 mol) 10-Hydroxystearinsäure isoliert und per ¹H-NMR-Spektroskopie charakterisiert.

¹H-NMR (500 MHz, CDCl₃) δ/ppm = 3,61 (m, 1H), 2,37 (t, ³*J*= 7,5 Hz, 2H), 1,66 (q, ³*J*= 7,2 Hz, 2H), 1,47 (m, 4H) 1,31 (m, 22H), 0,90 (t, ³*J*= 6,8 Hz, 3H)

### Beispiel 3: Hydratisierung von Linolsäure

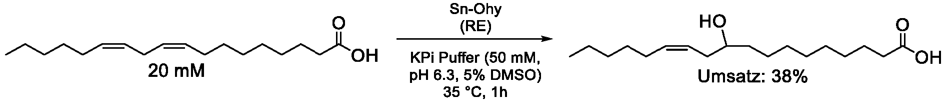

Linolsäure (5,6 mg, 0,02 mmol, 20 mM) wurde in DMSO (50 µL) gelöst. Danach wurden KPi-Puffer (pH 6,3, 50 mM, 850 µL), Sn-Ohy-Rohextrakt (100 µL, 15 mg mL⁻¹ Proteinkonzentration) der Reaktionsmischung zugegeben und bei 35 °C und 850 rpm für 1 h gerührt. Das Rohprodukt wurde mit CDCl₃ (2x 700 µL) extrahiert und mittels ¹H-NMR-Spektroskopie analysiert. Für (*Z*)-10-Hydroxyoctadec-12-ensäure konnte ein Umsatz von 38% erhalten werden.

### (Z)-10-Hydroxyoctadec-12-ensäure:

¹H-NMR (500 MHz, CDCl₃): [ppm]= 5,64 - 5,54 (m, 1H), 5,48 - 5,38 (m, 1H), 3,64 (p, ³*J*= 6,1 Hz, 1H), 2,37 (t, ³*J*= 7,5 Hz, 2H), 2,24 (t, ³*J*= 6,8 Hz, 2H), 2,07 (m, 2H), 1,66 (p, ³*J*= 7,6 Hz, 2H), 1,48 (h, ³*J*= 8,2, 6,8 Hz, 2H) , 1,43 - 1,31 (m, 16H), 0,91 (t, ³*J*= 6,8 Hz, 3H).

### Beispiel 4: Hydratisierung von Linolensäure

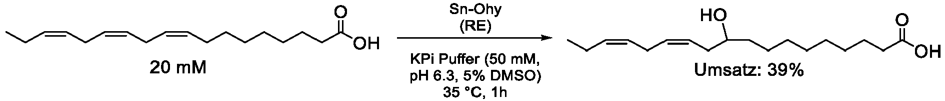

Linolensäure (5,6 mg, 0,02 mmol, 20 mM) wurde in DMSO (50 µL) gelöst. Danach wurden KPi-Puffer (pH 6,3, 50 mM, 850 µL), Sn-Ohy-Rohextrakt (100 µL, 15 mg mL⁻¹ Proteinkonzentration) der Reaktionsmischung zugegeben und bei 35 °C und 850 rpm für 1 h gerührt. Das Rohprodukt wurde mit CDCl₃ (2x 700 µL) extrahiert und mittels ¹H-NMR-Spektroskopie analysiert. Für (12*Z*,15*Z*)-10-Hydroxyoctadeca-12,15-diensäure konnte ein Umsatz von 39% erhalten werden.

### (12Z,15Z)-10-Hydroxyoctadeca-12,15-diensäure:

¹H-NMR (500 MHz, CDCl₃): [ppm]= 5,64 - 5,40 (m, 4H), 3,64 (p, ³*J*= 6,1 Hz, 1H), 2,37 (t, ³*J*= 7,5 Hz, 2H), 2,86 - 2,75 (m, 2H), 2,24 (t, ³*J*= 6,8 Hz, 2H), 1,66 (p, ³*J*= 7,6 Hz, 2H), 1,48 (h, ³*J*= 8,2, 6,8 Hz, 2H) , 1,43 - 1,31 (m, 12H), 0,91 (t, ³*J*= 6,8 Hz, 3H).

### Beispiel 5: Bildung eines Esterpolyols aus hydratisierter Ölsäure und einem Diol

Die erhaltene 10-Hydroxystearinsäure (15 g (0,05 mol)) wurde in gleichen Volumina von MTBE und n-Heptan gelöst. Anschließend wurden Hexan-1,6-diol (5,61 g, 0,05 mmol), CAL-B (1,5 g) und Molekularsieb (0,4 nm, 20 g) hinzugegeben und für 14 h bei 35 °C im *SpinChem*^{®} -Reaktor gerührt.

CAL-B ist die Lipase B des Organismus *Candida antarctica* und wurde z. B. von Haeffner et al. 1998 beschrieben (F. Haeffner, T. Norin and K. Hult 'Molecular modeling of the enantioselectivity in lipase-catalyzed transestrifications reactions', Biophys. J, 74(3), p. 1251-1262, March 1998). Diese in diesem experimentellen Beispiel verwendete Lipase CAL-B ist hierbei in immobilisierter Form kommerziell erhältlich (54326 Sigma-Aldrich Lipase B *Candida antarctica* immobilized on Immobead 150, recombinant from *Aspergillus oryzae).*

Das Lösungsmittel wurde unter Vakuum entfernt und das Rohprodukt per Säulenchromatographie isoliert (Cyclohexan:EtOAc, 3:1).

7 g Hexan-1,6-diyl-bis(10-hydroxyoctadecanoat) wurden abgetrennt; die Verbindung wurde per NMR und MS charakterisiert.

1H-NMR (500 MHz, CDCl₃) δ/ppm = 4,08 (t, ³*J*= 6,7 Hz, 4H), 3,60 (q, ³*J* = 5,8, 5,1 Hz, 2H), 2,31 (t, ³*J* = 7,5 Hz, 4H), 1,69 - 1,59 (m, 8H), 1,46 - 1,38 (m, 16H), 1,31 (d, ³*J* = 9,2 Hz, 40H), 0,90 (t, ³*J*= 6,7 Hz, 6H); ¹³C-NMR (126 MHz, CDCl₃) δ/ppm = ¹³C NMR (126 MHz, CDCl₃) δ/ppm = 173,96, 72,00, 64,17, 29,73, 29,64, 29,61, 29,43, 29,29, 29,21, 29,14, 28,56, 25,66, 24,99, 22,68, 14,12; MS (ESI): m/z = 706,2 [M+Na⁺].

Das erhaltene Esterpolyol weist im Vergleich zu etablierten Diolen eine deutlich stärkere Neigung auf, beim Erreichen eines in der Temperatur eng gefassten Erstarrungspunkts kristalline, durchgehende Strukturen auszubilden, was für eine sehr niedrige Schwankungsbreite und hohe Reinheit spricht. Mit äquimolarer Menge an aufgereinigtem Rizinus-Öl vermischt ergibt sich eine gleichsinnig reine und emulsionsstabile Mischkomponente mit einer OH-Funktionalität von 2,37 +- 0,01.

Mit einem optischen Referenz-Farbstoff (1,2 % Reaktiv-Farbstoff mit hydroxy-funktioneller Einbindbarkeit in PU-Strukturen; die OH-Funktionalität wird dadurch um 0,01 verringert) erweist sich die Gleichmäßigkeit der PU-Reaktivmasse nach Zugabe von HMDI und etablierten Stell- und Hilfs-Stoffen schon optisch als deutlich gleichmäßiger. Schlieren oder schwankende Helligkeiten während des Einrührens und Homogenisierens sind nicht sichtbar.

### Beispiel 6: Bildung weiterer Esterpolyole aus hydratisierter Ölsäure und Polyolen

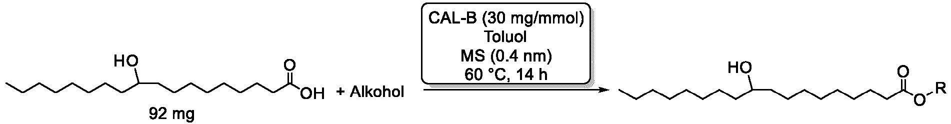

Es wurden 10-Hydroxystearinsäure (92 mg, 0,25 mmol) in Toluol (3 mL) gelöst und im Anschluss die jeweilige Alkohol-Komponente sowie der Biokatalysator (Lipase B aus *Candida antarctica* in immobilisierter Form, CAL-B) und das Molekularsieb hinzugefügt. Danach wurde die daraus resultierende Reaktionsmischung 14 Stunden bei einer Reaktionstemperatur von 60 °C gerührt. Anschließend wurde das Lösungsmittel unter vermindertem Druck entfernt und das daraus resultierende Rohprodukt massenspektrometrisch analysiert. Hierbei wurden in den Rohprodukten sowohl das Ausgangsmaterial, als auch die Mono-, Di- oder Tri-, Tetraester detektiert. Eine Übersicht über die verwendeten Alkohol-Komponenten sowie die zu diesen Versuchen zugehörigen Mengen an Molekularsieb und Biokatalysator (Lipase CAL-B) sind der nachfolgenden Tabelle 1 aufgeführt.

**Tabelle 1. Übersicht über die in den Veresterungsexperimenten gemäß der in diesem Beispiel verwendeten Alkohol-Komponenten sowie die zu diesen Versuchen zugehörigen Mengen an Molekularsieb und Biokatalysator (Lipase CAL-B)**

| Reaktions-Nr. | Alkohol-Komponente | Alkohol | MS (0,4 nM)/ mg | Lipase CAL-B/mg |
|---|---|---|---|---|
| **6-1** | 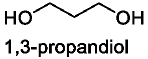 | 12,1 µL (0,50 Äq) | 136 | 11 |
| **6-2** | 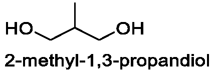 | 14,7 µL (0,50 Äq) | 101 | 13 |
| **6-3** | 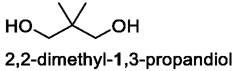 | 18,0 mg (0,50 Äq) | 128 | 12 |
| **6-4** | 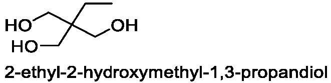 | 15,1 mg (0,33 Äq) | 105 | 12 |

In diesen Versuchen wurde die Bildung der folgenden gewünschten Produkte mittels Massenspektrometrie nachgewiesen:

### Beispiel 6-1:

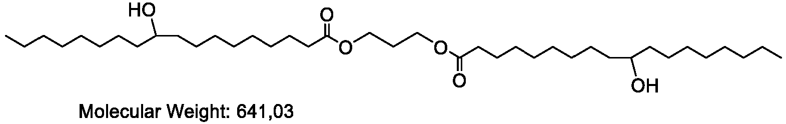

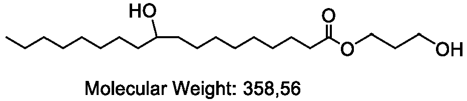

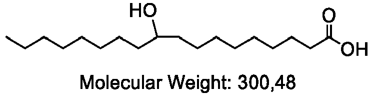

### Beispiel 6-2:

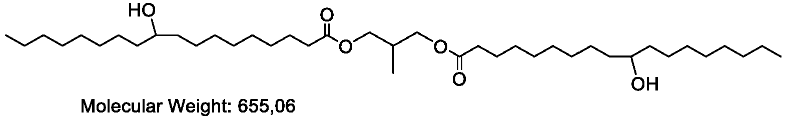

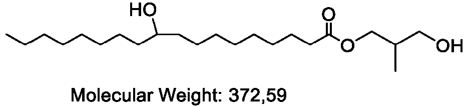

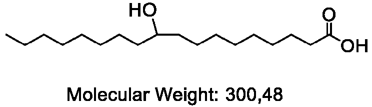

### Beispiel 6-3:

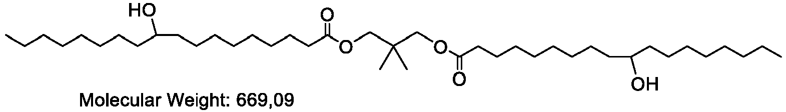

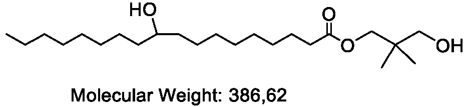

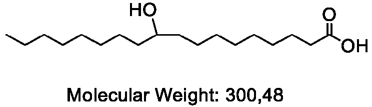

### Beispiel 6-4:

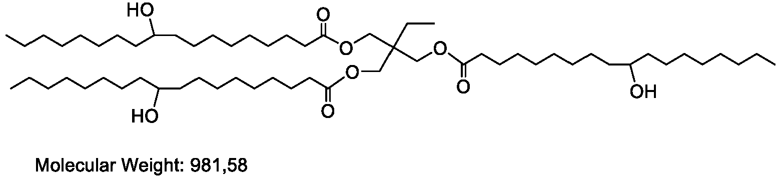

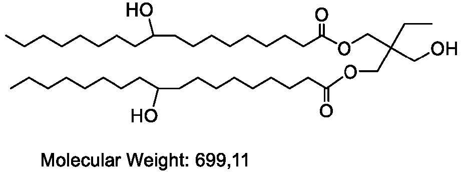

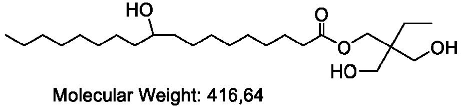

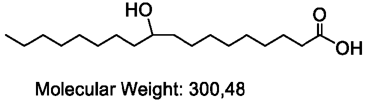

Anhand dieser Versuche ist erkennbar, dass die Bildung der folgenden gewünschten Esterdiole (d.h. Diole mit mindestens zwei Esterbindungen im Molekül) bzw. Estertriole mittels biotechnologischer Umsetzung von Diolen bzw. Triolen und Hydroxyfettsäuren gezielt durchführbar ist. Entsprechende Umsetzungen mit Polyaminen bzw. (Poly)amino(poly)alkoholen sind für den Fachmann basierend auf dem allgemeinen Fachwissen mittels Amidasen oder synthetisch ebenfalls durchführbar.

### Beispiel 7: Umsetzung von 12-Hydroxystearinsäure mit verschiedenen Linkern

Aufgrund der besseren Verfügbarkeit wurden die nachfolgenden Reaktionen mit 12-Hydroxystearinsäure (12-HSS), anstatt mit 10-Hydroxystearinsäure, welches von der Sn-Ohy generiert wird, durchgeführt. Die Ergebnisse zur Veresterung von 10-Hydroxystearinsäure und 12-Hydroxystearinsäure sind jedoch vergleichbar.

Die Reaktion von 12-Hydroxystearinsäure mit CAL-B zeigt, dass die Enzym-katalysierte Umsetzung nicht zur Selbstkondensation führt, wie sowohl aus dem ¹H-NMR-Spektrum als auch aus dem Massenspektrum ersichtlich ist. Daher kann hier ein eindeutiger Vorteil in der Enzym-katalysierten Veresterung aufgezeigt werden, da weder eine Oligomerisationsreaktion, noch eine andere unerwünschte Reaktion stattfindet.

Verschiedene Immobilisierungsformen der Lipase CAL-B sind für die enzymatisch-katalysierte Veresterung von 12-HSS mit 1,6-Hexandiol geeignet. So konnten bereits nach sechs Stunden der Mono- sowie der Diester detektiert werden.

In weiteren Versuchen wurde die Lipase CalB 165G genutzt, um 12-HSS mit verschiedenen Linkern zu verestern. Dafür wurden Reaktionen für sechs und für 14 Stunden durchgeführt. Es zeigte sich, dass die Veresterung erfolgreich war, was wiederum im ¹H-NMR-Spektrum sowie im Massenspektrum erkennbar ist. Auch hier konnte nach sechs Stunden bereits der Mono- sowie der Diester und bei 2-Ethyl-2-(hydroxymethyl)1,3-propandiol der Triester detektiert werden.

2-Ethyl-2-(hydroxymethyl)1,3-propandiol (0,11 mol) wurde im 100 g Maßstab erneut mit 12-HSS (0,33 mol) verestert. Nach der Reaktion konnte eine OH-Zahl von 172 mgKOH/g ermittelt werden. Dieses Triol (M=981,6 g/mol) wurde genutzt, um daraus ein Polyurethan herzustellen. Zur Herstellung wurde das oben erhaltene Triol mit einem Gemisch aliphatischer Isocyanate (HDI-Isocyanurat und IPDI-Isocyanurat) stöchiometrisch vermischt, ausgerakelt und bei 83-85 °C ausgehärtet. Es wurde ein transparenter Film erhalten, der bei 150 µm Dicke farblos war.

### Beispiel 7a: Chemisch-katalysierte Selbstkondensation von 12-Hydroxystearinsäure

Ein Gemisch aus 12-Hydroxystearinsäure (10,00 g, 33,28 mmol), MTBE (20 mL) und Schwefelsäure (98%, 0,3 mL, 5,63 mmol) wurde mit Molsieb (4 Å, 1,00 g) für 6 h oder 14 h bei 60°C gerührt. Das resultierende Rohprodukt wurde mit MTBE (40 mL) versetzt, mit gesättigter Natriumchloridlösung (2x 25 mL) gewaschen und über Natriumsulfat getrocknet. Das Lösungsmittel wurde unter vermindertem Druck eingedampft und das Produkt als Feststoff (8,36 g, 27,85 mmol, 84%) isoliert.

### Beispiel 7b: Enzymatisch-katalysierte Selbstkondensation von 12-Hydroxystearinsäure

Ein Gemisch aus 12-Hydroxystearinsäure (10,00 g, 33,28 mmol), MTBE (20 mL) und CAL-B (1,00 g) wurde mit Molsieb (4 Å, 1,00 g) für 6 h oder 14 h bei 60°C gerührt. Das resultierende Rohprodukt wurde mit MTBE (40 mL) versetzt, mit gesättigter Natriumchloridlösung (2x 25 mL) gewaschen und über Natriumsulfat getrocknet. Das Lösungsmittel wurde unter vermindertem Druck eingedampft und das Produkt quantitativ isoliert. Das ¹H-NMR-Spektrum sowie das Massenspektrum zeigten, dass es bei der Katalyse mit CAL-B zu keiner Oligomerisierung der 12-Hydroxystearinsäure kam.

### Beispiel 7c: Veresterung 12-Hydroxystearinsäure mit Hexan-1,6-diol und verschiedenen Lipasen

12-Hydroxystearinsäure (0,20 g, 0,67 mmol) wurde in MTBE (6 mL) gelöst. Danach wurden Hexan-1,6-diol (0,04 g, 0,34 mmol), Lipase (24 mg) und Molekularsieb (4 Å, 240 mg) in die Reaktionslösung überführt und bei 60 °C gerührt. Nach sechs und 14 h wurde eine Probe (2 mL) entnommen, zentrifugiert und die organische Phase isoliert. Das Lösungsmittel wurde entfernt. Es wurde eine Analytik mittels ¹H-NMR-Spektroskopie sowie Massenspektrometrie durchgeführt.

**Tabelle 2: Veresterung von 12-Hydroxystearinsäure mit Hexan-I, 6-diol und verschiedenen Lipasen.**

| **Reaktion-Nr.** | **Lipase** | **Umsetzung stattgefunden** |
|---|---|---|
| **1** | Addzyme CalB 165G | Ja |
| **2** | CalB immo 8285 | Ja |
| **3** | CalB immo 8806 | Ja |

### 12-Hydroxystearinsäure (zum Vergleich)

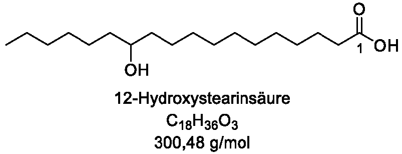

¹H-NMR (500 MHz, DMSO-d6) δ/ppm = 4,18 (d, ³*J* = 5,4 Hz, 1H, OH), 3,34 (m, (C12)*H*), 2,18 (t, ³*J* = 7,4 Hz, 2H, (C2)H), 1,48 (t, ³*J* = 7,2 Hz, 2H, (C3)H), 1,31 (m, 26H, *H*_{alkyl}), 0,90 (t, ³*J*= 6,9 Hz, 3H, C(18)H); MS (ESI): m/z = 323,48 [M+Na⁺], 623,96 [2M+ Na⁺].

### Hexan-1,6-diyl bis(12-hydroxyoctadecanoat)

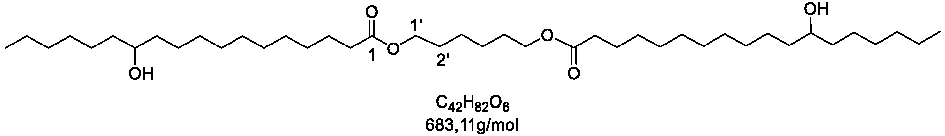

¹H-NMR (500 MHz, CDCl₃) δ/ppm = 4,06 (d, ³*J* = 6,7 Hz, (C1')*H*), 3,58 (m, (C12)H), 2,28 (t, ³*J* = 7,6 Hz, (C2)*H*), 1,62 (m, (C3,2')H), 1,40 (m, *H*_{alkyl}), 1,28 (m, *H*_{alkyl}), 0,89 (m, C(18)H); MS (ESI): m/z = 423,4 [M+Na⁺] (Monoester), 705,6 [M+Na⁺] (Diester).

### Beispiel 7d: Veresterung von 12-Hydroxystearinsäure mit CAL-B und verschiedenen Alkohol-Linkern

12-Hydroxystearinsäure (0,20 g, 0,67 mmol) wurde in MTBE (6 mL) gelöst. Danach wurden Alkohol, Addzyme CalB 165G (24 mg) und Molekularsieb (4 Å, 240 mg) in die Reaktionslösung überführt und bei 60 °C gerührt. Nach sechs und 14 h wurde eine Probe (2 mL) entnommen, zentrifugiert und die organische Phase isoliert. Das Lösungsmittel wurde entfernt. Es wurde eine Analytik mittels ¹H-NMR-Spektroskopie sowie Massenspektrometrie durchgeführt.

**Tabelle 3: Veresterung 12-Hydroxystearinsäure mit CAL-B und verschiedenen Alkohol-Linkern.**

| **Reaktions-Nr.** | **Alkohol** | **mg** | **mmol** | **Umsetzung stattgefunden** |
|---|---|---|---|---|
| **1** | 2-Butyl-2-ethyl-1,3-propandiol | 53,5 | 0,33 | Ja |
| **2** | 2-Ethyl-2-(hydroxymethyl)1,3-propandiol | 30,0 | 0,22 | Ja |
| **3** | 1,2-Propandiol | 25,3 | 0,33 | Ja |
| **4** | 1,3-Propandiol | 25,3 | 0,33 | Ja |
| **5** | 1,4-Butandiol | 30,0 | 0,33 | Ja |
| **6** | 1,3-Butandiol | 30,0 | 0,33 | Ja |
| **7** | 2-Methyl-1,3-propanediol | 30,0 | 0,33 | Ja |
| **8** | 1,5-Pentandiol | 34,7 | 0,33 | Ja |

### 2-Butyl-2-ethylpropan-1,3-diyl bis(12-hydroxyoctadecanoat

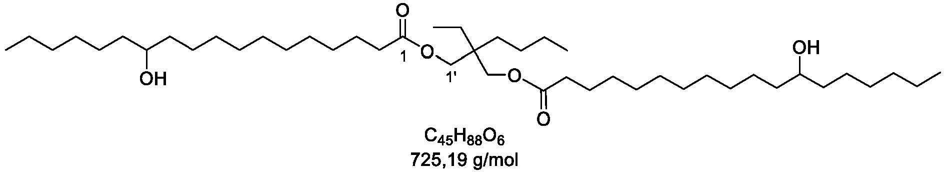

¹H-NMR (500 MHz, CDCl₃) δ/ppm = 3,97 (s, (C1')*H*), 3,59 (m, (C12)H), 2,34 (m, (C2)H), 1,62 (m, (C3)H), 1,43 (m, *H*_{alkyl}), 1,29 (m, *H*_{alkyl}), 0,89 (m, C(18)H); MS (ESI): m/z = 465,7 [M+Na⁺] (Monoester), 748,2 [M+Na⁺] (Diester).

### 2-Ethyl-2-(((12-hydroxyoctadecanoyl)oxy)methyl)propan-1,3-diyl bis(12-hydroxyoctadecanoat)

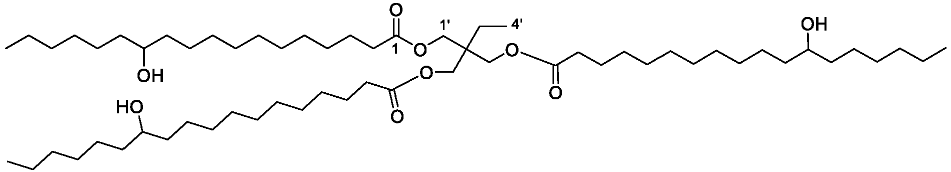

¹H-NMR (500 MHz, CDCl₃) δ/ppm = 4,03 (s, (C1')*H*), 3,58 (m, (C12)H), 2,33 (m, (C2)H), 1,63 (m, (C3)H), 1,42 (m, *H*_{alkyl}), 1,28 (m, *H*_{alkyl}), 0,88 (t, ³*J*= 6,7 Hz, C(18, 4')H); MS (ESI): m/z = 439,6 [M+Na⁺] (Monoester), 722,1 [M+Na⁺] (Diester), 1004,6 (Triester). Theoretische OH-Zahl: 171,5, experimentell gefunden: 172, Schmelzbereich: 50-53 °C

### Propan-1,2-diyl bis(12-hydroxyoctadecanoat)

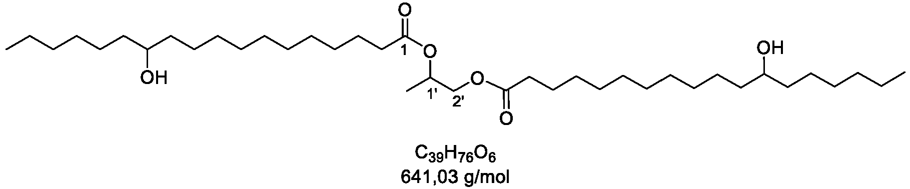

¹H-NMR (500 MHz, CDCl₃) δ/ppm = 5,13 (qd, ³*J* = 6,7 Hz, ⁴*J* = 3,7 Hz, C(1')H), 4,22 (dd, ⁴*J* = 11,7, 3,6 Hz, C(2')H) 4,05 (dd, ³*J* = 6,6, ⁴*J* = 11,7, C(2')H), 3,58 (m, (C12)H), 2,30 (m, C(2)H), 1,62 (m, (C3)H), 1,42 (m, *H*_{alkyl}), 1,28 (m, *H*_{alkyl}), 0,88 (m, C(18)H); MS (ESI): m/z = 381,5 [M+Na⁺] (Monoester), 664,0 [M+Na⁺] (Diester).

### Propan-1,3-diyl bis(12-hydroxyoctadecanoat)

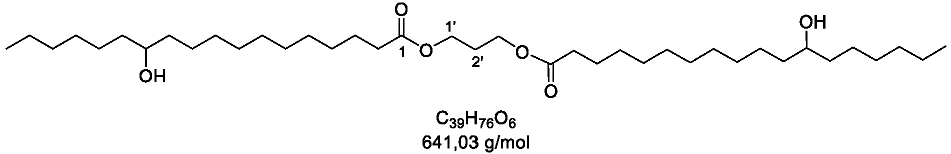

¹H-NMR (500 MHz, CDCl₃) δ/ppm = 4,15 (t, ³*J* = 6,3 Hz, (C1')*H*), 3,58 (m, (C12)H), 2,29 (t, ³*J* = 7,6 Hz (C2)H), 1,96 (q, ³*J* = 6,3 Hz, (C3,2')H), 1,61 (m, (C3)H), 1,42 (m, *H*_{alkyl}), 1,27 (m, *H*_{alkyl}), 0,89 (m, C(18)H); MS (ESI): m/z = 381,5 [M+Na⁺] (Monoester), 664,0 [M+Na⁺] (Diester).

### Butan-1,4-diyl bis(12-hydroxyoctadecanoat)

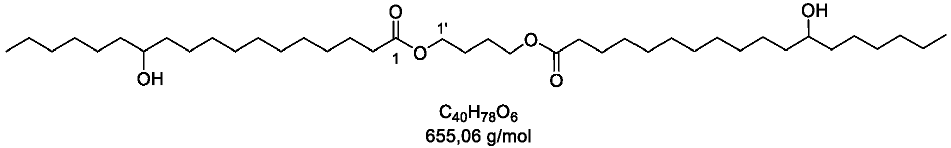

¹H-NMR (500 MHz, CDCl₃) δ/ppm = 4,09 (m, (C1')*H*), 3,58 (m, (C12)H), 2,29 (t, ³*J* = 7,6 Hz, (C2)H), 1,69 1,62 (m, (C3)*H*), 1,42 (m, *H*_{alkyl}), 1,28 (m, *H*_{alkyl}), 0,88 (t, ³*J*= 7,0 Hz, C(18)H); MS (ESI): m/z = 395,6 [M+Na⁺] (Monoester), 678,0 [M+Na⁺] (Diester).

### Butan-1,3-diyl bis(12-hydroxyoctadecanoat)

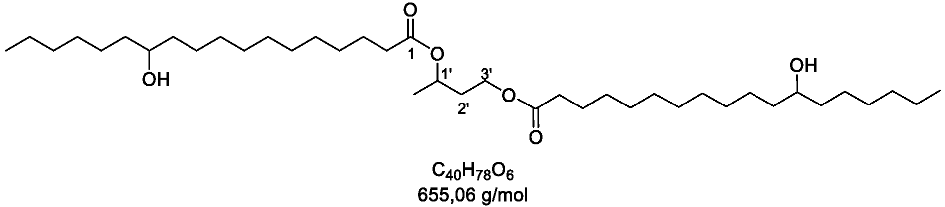

¹H-NMR (500 MHz, CDCl₃) δ/ppm = 5,01 (m, (C1')H), 4,11 (m, (C3')*H*)), 3,57 (m, (C12)*H*), 2,27 (m, (C2)H), 1,87 (m, (C4')H)), 1,59 (m, (C3)H), 1,42 (m, *H*_{alkyl}), 1,27 (m, *H*_{alkyl}), 0,88 (m, C(18)H); MS (ESI): m/z = 395,6 [M+Na⁺] (Monoester), 678,0 [M+Na⁺] (Diester).

### 2-Methylpropane-1,3-diyl bis(12-hydroxyoctadecanoat)

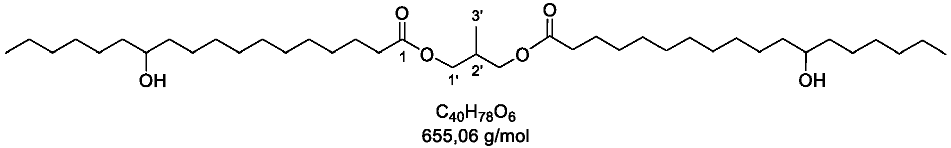

¹H-NMR (500 MHz, CDCl₃) δ/ppm = 4,01 (m, C(1')H), 3,58 (m, (C12)H), 2,30 (t, ³*J* = 7,6 Hz, (C2)H), 2,16 (m, C(2')*H*), 1,62 (t, ³*J* = 7,2 Hz, (C3)H), 1,43 (m, *H*_{alkyl}), 1,28 (m, *H*_{alkyl}), 0,88 (m, C(18)H); MS (ESI): m/z = 395,6 [M+Na⁺] (Monoester), 678,0 [M+Na⁺] (Diester).

### Pentan-1,5-diyl bis(12-hydroxyoctadecanoat)

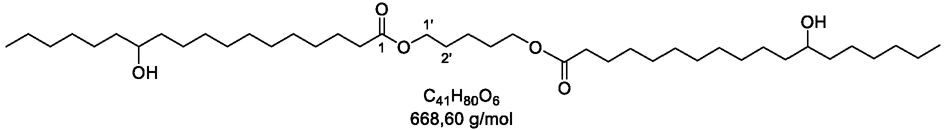

¹H-NMR (500 MHz, CDCl₃) δ/ppm = 4,06 (d, ³*J* = 5,2; 6,7 Hz, (C1')H), 3,58 (m, (C12)*H*), 2,28 (m, (C2)H), 1,62 (m, (C3,2')*H*), 1,40 (m, *H*_{alkyl}), 1,28 (m, *H*_{alkyl}), 0,89 (m, C(18)H); MS (ESI): m/z = 409,3 [M+Na⁺] (Monoester), 692,06 [M+Na⁺] (Diester).

### Beispiel 7e: Veresterung von 12-Hydroxystearinsäure mit CAL-B und 2-Ethyl-2-(hydroxymethyl)1,3-propandiol

12-Hydroxystearinsäure (100 g, 0,33 mol) wurde in MTBE (500 mL) gelöst. Danach wurden 2-Ethyl-2-(hydroxymethyl)1,3-propandiol (14,9 g, 0,11 mol), Addzyme CalB 165G (12 g) und Molekularsieb (4 Å, 50 g) in die Reaktionslösung überführt und bei 60 °C gerührt für 16 h gerührt. Die Probe wurde zentrifugiert, die organische Phase isoliert und das Lösungsmittel entfernt. Die OH-Zahl wurde anhand der Norm DIN 53240-2 bestimmt.

### Beispiel 7f: Herstellung eines Polyurethans

Das oben beschriebene Triol aus 2-Ethyl-2-(hydroxymethyl)1,3-propandiol und 12-Hydroxystearinsäure, OH-Zahl 172 wurde mit einem Gemisch aliphatischer Isocyanate (HDI-Isocyanurat, IPDI-Isocyanurat, Isocyanatgehalt: 21 %) zu einem klaren, ungefärbten, blasenfreien Polyurethan umgesetzt.

Dazu wurden 5 g Triol eingewogen und auf 60 °C erwärmt. Nach dem Aufschmelzen wurde das Material in einem Zentrifugalmischer unter Vakuum entlüftet. Anschließend wurden 3,17 g des oben beschriebenen Polyisocyanats (ebenfalls auf 60 °C vorgewärmt) hinzugegeben und im Zentrifugalmischer unter Vakuum intensiv vermischt. Die so erhaltene Mischung wurde auf einem Heiztisch mittels eines Rakels aufgetragen und bei 83-85 °C ausgehärtet. Nach 150 sec erhielt man ein festes Polymer. Um die Reaktion zu vervollständigen, wurde der Film 7 Tage bei 23°C und 50 %rF gelagert. Die Shore-Härtemessung ergab Shore A 60 und Shore D 15. Das Infrarotspektrum zeigte die zu erwartenden Absorptionsbanden eines aliphatischen Polyurethans.

## Patentansprüche

1. Ein Verfahren zur Herstellung eines Hydroxyfettsäurekondensats oder eines Gemischs von Hydroxyfettsäurekondensaten, das Verfahren umfassend die Schritte:
- Bereitstellen einer oder mehrerer Fettsäuren mit mindestens einer C=C Doppelbindungsfunktionalität,
- biotechnologische H₂O-Addition mittels einer Hydratase an mindestens eine C=C-Doppelbindungsfunktionalität der einen oder mehreren Fettsäuren, wobei die Doppelbindung keine terminale Doppelbindung ist, unter Erhalt einer oder mehrerer Hydroxyfettsäuren,
- Umsetzung der einen oder mehreren Hydroxyfettsäure(n) mit einer oder mehreren mindestens zweiwertigen Linkergruppe(n), ausgewählt aus Polyolen, Polyaminen und (Poly)amino(poly)alkoholen, unter Erhalt eines Hydroxyfettsäurekondensats oder eines Gemischs von Hydroxyfettsäurekondensaten, wobei die Umsetzung der einen oder mehreren Hydroxyfettsäure(n) mit der einen oder den mehreren mindestens zweiwertigen Linkergruppe(n) biotechnologisch erfolgt.

2. Das Verfahren nach Anspruch 1, wobei die eine oder mehreren mindestens zweiwertigen Linkergruppen ein oder mehrere Polyole sind, wobei die Polyole bevorzugt ausgewählt sind aus Diol, Triol, Tetraol, Pentaol, Hexaol, oder Kombinationen davon, stärker bevorzugt ausgewählt sind aus Diol, Triol, Tetraol, oder Kombinationen davon.

3. Das Verfahren nach Anspruch 1 oder 2, wobei die eine oder mehreren mindestens zweiwertigen Linkergruppen ein oder mehrere aliphatische Polyole, bevorzugt ein oder mehrere aliphatische C₂₋₄₀ Polyole sind, wobei die eine oder mehreren aliphatischen C₂₋₄₀ Polyole bevorzugt ausgewählt sind aus aliphatischem Alkandiol, Alkantriol, Alkantetraol, Alkanpentaol, Alkanhexaol oder Kombinationen davon.

4. Das Verfahren nach einem der vorherigen Ansprüche, wobei die eine oder mehreren mindestens zweiwertigen Linkergruppen ausgewählt sind aus aliphatischem α-ω-Alkandiol, α-ω-Alkantriol, α-ω-Alkantetraol, α-ω-Alkanpentaol, α-ω-Alkanhexaol oder Kombinationen davon.

5. Das Verfahren nach einem der vorherigen Ansprüche, wobei die eine oder mehreren mindestens zweiwertigen Linkergruppen ausgewählt sind aus 1,3-Propandiol, 2-Methyl-1,3-propandiol, 2,2-Dimethyl-1,3-propandiol, 2-Ethyl-2-hydroxymethyl-1,3-propandiol und 1,6-Hexandiol und Kombinationen davon.

6. Das Verfahren nach einem der vorherigen Ansprüche, wobei mindestens eine, bevorzugt zwei, drei oder vier, der Hydroxygruppen der mindestens zweiwertigen Linkergruppe mit Hydroxyfettsäure verestert werden.

7. Das Verfahren nach einem der vorherigen Ansprüche, wobei die eine oder mehreren Fettsäuren mit mindestens einer C=C Doppelbindungsfunktionalität ausgewählt sind aus der Gruppe bestehend aus aliphatischen C₆₋₄₀-Carbonsäuren mit einer, zwei oder drei C=C Doppelbindungsfunktionalitäten, wobei die C₆₋₄₀-Carbonsäuren bevorzugt C₁₀₋₃₀, stärker bevorzugt C₁₂₋₂₄-Carbonsäuren sind.

8. Das Verfahren nach einem der vorherigen Ansprüche, wobei die eine oder mehreren Fettsäuren mit mindestens einer C=C Doppelbindungsfunktionalität (i) ausgewählt sind aus der Gruppe bestehend aus einfach ungesättigten Fettsäuren, bevorzugt ausgewählt aus Undecylensäure, Myristoleinsäure, Palmitoleinsäure, Margaroleinsäure, Petroselinsäure, Ölsäure, Elaidinsäure, Vaccensäure, Gadoleinsäure, Gondosäure, Cetoleinsäure, Erucasäure, Vernolsäure, cis-5-Eicosensäure, Brassidinsäure, Nervonsäure und Kombinationen davon, und/oder aus der Gruppe bestehend aus mehrfach ungesättigten Fettsäuren, bevorzugt ausgewählt aus Linolsäure, Linolensäure, Calendulasäure, Punicinsäure, Elaeostearinsäure, Arachidonsäure, Timnodonsäure, Clupanodonsäure, Lesquerolsäure, Licansäure, Cervonsäure und Kombinationen davon; oder
(ii) ausgewählt sind aus Ölsäure, Linolsäure, Linolensäure, Palmitoleinsäure und Kombinationen davon, wobei die Fettsäure bevorzugt ausgewählt ist aus Ölsäure, Linolsäure, Linolensäure und Kombinationen davon, und stärker bevorzugt Ölsäure ist; oder
(iii) zu mindestens 70 mol-%, bevorzugt mindestens 85 mol-%, stärker bevorzugt mindestens 90 % eines oder mehrere, bevorzugt zwei oder drei, ausgewählt aus Ölsäure, Linolsäure und Linolensäure enthalten.

9. Das Verfahren nach einem der vorherigen Ansprüche, wobei die biotechnologische H₂O-Addition mittels einer Hydratase von *Stenotrophomonas nitritireducens,* durchgeführt wird.

10. Das Verfahren nach einem der vorherigen Ansprüche, wobei die Umsetzung mit einer oder mehreren mindestens zweiwertigen Linkergruppen mittels mindestens einem Enzym ausgewählt aus der Gruppe bestehend aus Hydroxylasen, bevorzugt Esterasen, Lipasen und/oder Proteasen, stärker bevorzugt Lipasen, durchgeführt wird.

11. Das Verfahren nach Anspruch 10, wobei die Umsetzung mit einer oder mehreren mindestens zweiwertigen Linkergruppe mittels mindestens einer Lipase durchgeführt wird, wobei die Lipase bevorzugt die Lipase B von *Candida antarctica* ist.

12. Das Verfahren nach einem der vorherigen Ansprüche, wobei die Umsetzung der einen oder mehreren Hydroxyfettsäuren mit einer oder mehreren mindestens zweiwertigen Linkergruppen mittels einer Lipase von *Candida antarctica,* besonders bevorzugt Lipase B von *Candida antarctica,* durchgeführt wird, und die eine oder mehreren mindestens zweiwertigen Linkergruppen aus Alkandiolen, Alkantriolen, Alkantetraolen und Kombinationen davon ausgewählt sind.

13. Das Verfahren nach Anspruch 12, wobei die eine oder mehreren mindestens zweiwertigen Linkergruppen
(i) aus Alkandiolen, Alkantriolen und Kombinationen davon ausgewählt sind; oder
(ii) aus Ethandiol, Propandiolen, Butandiolen, Pentandiolen, Hexandiolen, Heptandiolen, Octandiolen, Butantriolen, Pentantriolen, Hexantriolen, Heptantriolen, Octantriolen, Butantetrolen, Pentantetrolen, Hexantetrolen, Heptantetrolen, Octantetrolen und Kombinationen davon ausgewählt sind; oder
(iii) aus Ethandiol, Propandiolen, Butandiolen, Pentandiolen, Hexandiolen, Heptandiolen, Octandiolen, Butantriolen, Pentantriolen, Hexantriolen, Heptantriolen, Octantriolen und Kombinationen davon ausgewählt sind.

14. Ein Polyurethan, das erhältlich ist durch Umsetzung einer Zusammensetzung umfassend ein durch das Verfahren nach einem der vorherigen Ansprüche erhältlichen Hydroxyfettsäurekondensats oder Gemischs von Hydroxyfettsäurekondensaten mit einem Diisocyanat, Triisocyanat, Tetraisocyanat, Pentaisocyanat oder Hexaisocyanat oder Kombinationen davon.

## Claims

1. A method for producing a hydroxy fatty acid condensate or a mixture of hydroxy fatty acid condensates, the method comprising the steps of:
- providing one or more fatty acids having at least one C=C double bond functionality,
- biotechnologically adding H₂O to at least one C=C double bond functionality of the one or more fatty acids by means of hydratase, wherein the double bond is not a terminal double bond, thus obtaining one or more hydroxy fatty acids,
- reacting the one or more hydroxy fatty acid(s) with one or more at least divalent linker group(s) selected from polyols, polyamines and (poly)amino(poly)alcohols, thus obtaining a hydroxy fatty acid condensate or a mixture of hydroxy fatty acid condensates, wherein the reaction of the one or more hydroxy fatty acid(s) with the one or more at least divalent linker group(s) is carried out biotechnologically.

2. The method according to claim 1, wherein the one or more at least divalent linker groups are one or more polyols, wherein the polyols are selected from diol, triol, tetraol, pentaol, hexaol, or combinations thereof, more preferably selected from diol, triol, tetraol, or combinations thereof.

3. The method according to claim 1 or 2, wherein the one or more at least divalent linker groups are one or more aliphatic polyols, preferably one or more aliphatic C₂₋₄₀ polyols, wherein the one or more aliphatic C₂₋₄₀ polyols are preferaly selected from an aliphatic alkanediol, alkanetriol, alkanetetraol, alkanepentaol, alkanehexaol, or combinations thereof.

4. The method according to any of the preceding claims, wherein the one or more at least divalent linker groups are selected from aliphatic α-ω-alkanediol, α-ω-alkanetriol, α-ω-alkantetraol, α-ω-alkanepentaol, α-ω-alkanehexaol, or combinations thereof.

5. The method according to any of the preceding claims, wherein the one or more at least divalent linker groups are selected from 1,3-propanediol, 2-methyl-1,3-propanediol, 2,2-dimethyl-1,3-propanediol, 2-ethyl-2-hydroxymethyl-1,3-propanediol and 1,6-hexanediol, and combinations thereof.

6. The method according to any of the preceding claims, wherein at least one, preferably two, three, or four of the hydroxy groups of the at least divalent linker groups are esterified with hydroxy fatty acid.

7. The method according to any of the preceding claims, wherein the one or more fatty acids having at least one C=C double bond functionality are selected from the group consisting of aliphatic C₆₋₄₀ carboxylic acids having one, two or three C=C double bond functionalities, wherein the C₆₋₄₀ carboxylic acids are preferably C₁₀₋₃₀, more preferably C₁₂₋₂₄ carboxylic acids.

8. The method according to any of the preceding claims, wherein the one or more fatty acids having at least one C=C double bond functionality
(i) are selected from the group consisting of monounsaturated fatty acids, preferably selected from undecylenic acid, myristoleic acid, palmitoleic acid, margaroleic acid, petroselinic acid, oleic acid, elaidic acid, vaccenic acid, gadoleic acid, gondoic acid, cetoleic acid, erucic acid, vernolic acid, cis-5-eicosenoic acid, brassidic acid, nervonic acid, and combinations thereof, and/or from the group consisting of polyunsaturated fatty acids, preferably selected from linoleic acid, linolenic acid, calendulic acid, punicinic acid, eleostearic acid, arachidonic acid, timnodonic acid, clupanodonic acid, lesquerolic acid, licanoic acid, cervonic acid, and combinations thereof; or
(ii) are selected from oleic acid, linoleic acid, linolenic acid, palmitoleic acid, and combinations thereof, wherein the fatty acid is preferably selected from oleic acid, linoleic acid, linolenic acid, and combinations thereof, and more preferred it is oleic acid; or
(iii) comprise at least 70 mol-%, preferably at least 85 mol-%, more preferably at least 90 mol-% of one or more, preferably two or three, selected from oleic acid, linoleic acid, and linolenic acid.

9. The method according to any of the preceding claims, wherein the biotechnological addition of H₂O is carried out by means of a hydratase of *Stenotrophomonas nitritireducens.*

10. The method according to any of the preceding claims, wherein the reaction with one or more at least divalent linker groups is carried out with at least one enzyme selected from the group consisting of hydroxylases, preferably esterases, lipases and/or proteases, more preferably lipases.

11. The method according to claim 10, wherein the reaction with one or more at least divalent linker groups is carried out with at least one lipase, wherein the lipase is preferably lipase B of *Candida antarctica.*

12. The method according to any of the preceding claims, wherein the reaction of the one or more hydroxy fatty acids with one or more at least divalent linker groups is carried out with a lipase of *Candida antarctica,* especially preferred lipase B of *Candida antarctica,* and the one or more at least divalent linker groups are selected from alkanediols, alkanetriols, alkantetraols, and combinations thereof.

13. The method according claim 12, wherein the one or more at least divalent linker groups
(i) are selected from alkanediols, alkanetriols, and combinations thereof; or
(ii) are selected from ethanediol, propanediols, butanediols, pentanediols, hexanediols, heptanediols, octanediols, butanetriols, pentanetriols, hexanetriols, heptanetriols, octanetriols, butanetetrols, pentanetetrols, hexanetetrols, heptanetetrols, octanetetrols, and combinations thereof; or
(iii) are selected from ethanediol, propanediols, butanediols, pentanediols, hexanediols, heptanediols, octanediols, butanetriols, pentanetriols, hexanetriols, heptanetriols, octanetriols, and combinations thereof.

14. A polyurethane obtainable by reacting a composition comprising a hydroxy fatty acid condensate or the mixture of hydroxy fatty acid condensates obtainable by the method according to any of the preceding claims with a diisocyanate, triisocyanate, tetraisocyanate, pentaisocyanate, or hexaisocyanate, or combinations thereof.

## Revendications

1. Procédé de fabrication d'un condensat d'hydroxy-acide gras ou d'un mélange de condensats d'hydroxy-acide, ce procédé comprenant les étapes suivantes :
- mise à disposition d'un ou plusieurs acides gras avec au moins une fonctionnalité de double liaison C=C,
- ajout biotechnologique de H₂O au moyen d'une hydratase au niveau d'au moins une fonctionnalité de double liaison C=C du ou des acides gras, dans lequel la double liaison n'est pas une double liaison terminale, afin d'obtenir un ou plusieurs hydroxy-acides gras,
- transformation du ou des hydroxy-acides gras avec un ou plusieurs groupes de liaison au moins bivalents, sélectionnés parmi des polyols, des polyamines et des (poly)amino(poly)alcools, afin d'obtenir un condensat d'hydroxy-acide gras ou un mélange de condensats d'hydroxy-acide, dans lequel la transformation du ou des hydroxy-acides gras est effectuée de manière biotechnologique avec le ou les groupes de liaison au moins bivalents.

2. Procédé selon la revendication 1, dans lequel le ou les groupes de liaison au moins bivalents sont un ou plusieurs polyols, dans lequel les polyols sont de préférence sélectionnés parmi un diol, un triol, un tétraol, un pentaol, un hexaol ou des combinaisons de ceux-ci, de préférence sont sélectionnés parmi un diol, un triol, un tétraol ou des combinaisons de ceux-ci.

3. Procédé selon la revendication 1 ou 2, dans lequel le ou les groupes de liaison au moins bivalents sont un ou plusieurs polyols aliphatiques, de préférence un ou plusieurs polyols C₂₋₄₀ aliphatiques, dans lequel le ou les polyols C₂₋₄₀ aliphatiques sont de préférence sélectionnés parmi un alcanediol, un alcanetriol, un alcanetétraol, un alcanepentaol, un alcanehexaol aliphatique ou des combinaisons de ceux-ci.

4. Procédé selon l'une des revendications précédentes, dans lequel le ou les groupes de liaison au moins bivalents sont sélectionnés parmi un α-ω-alcanediol, un α-ω-alcanetriol, un α-ω-alcanetétraol, un α-ω-alcanepentaol, un α-ω-alcanehexaol ou des combinaisons de ceux-ci.

5. Procédé selon l'une des revendications précédentes, dans lequel le ou les groupes de liaison au moins bivalents sont sélectionnés parmi le 1,3-propanediol, le 2-méthyl-1,3-propanediol, 2,2-diméthyl-1,3-propanediol, 2-éthyl-2-hydroxyméthyl-1,3-propanediol et 1,6-hexanediol et des combinaisons de ceux-ci.

6. Procédé selon l'une des revendications précédentes, dans lequel au moins un, de préférence deux, trois ou quatre des groupes hydroxy du groupe de liaison au moins bivalent est estérifié avec un hydroxy-acide gras.

7. Procédé selon l'une des revendications précédentes, dans lequel le ou les acides gras avec au moins une fonctionnalité de double liaison C=C sont sélectionnées dans le groupe constitué d'acides carboxyliques C₆₋₄₀ aliphatiques avec une, deux ou trois fonctionnalités de double liaison C=C, dans lequel les acides carboxyliques C₆₋₄₀ sont de préférence des acides carboxyliques C₁₀₋₃₀, de préférence des acides carboxyliques C₁₂₋₂₄.

8. Procédé selon l'une des revendications précédentes, dans lequel le ou les acides gras avec au moins une fonctionnalité de double liaison C=C
(i) sont sélectionnés dans le groupe constitué d'acides gras mono-insaturés, de préférence sélectionnés parmi l'acide undécylénique, l'acide myristolénique, l'acide palmitoléique, l'acide margarolique, l'acide pétrosélinique, l'acide oléique, l'acide élaïdique, l'acide vaccénique, l'acide gadolénique, l'acide gondoïque, l'acide cétaooléique, l'acide érucique, l'acide vernolique, l'acide cis-5-éicosénoïque, l'acide brassidique, l'acide nervonique et des combinaisons de ceux-ci et/ou dans le groupe constitué d'acides gras poly-insaturés, de préférence sélectionnés parmi l'acide linoléique, l'acide linolénique, l'acide calendique, l'acide punicinique, l'acide élaéostéarique, l'acide arachidonique, l'acide timnodonique, l'acide clupanodonique, l'acide lesquerolique, l'acide licanique, l'acide cervonique et des combinaisons de ceux-ci ; ou
(ii) sont sélectionnés parmi l'acide oléique, l'acide linoléique, l'acide linolénique, l'acide palmitoléique et des combinaisons de ceux-ci, dans lequel l'acide gras est de préférence sélectionné parmi l'acide oléique, l'acide linoléique, l'acide linolénique et des combinaisons de ceux-ci et de préférence est de l'acide oléique ; ou
(iii) contiennent, à hauteur d'au moins 70 % en moles, de préférence d'au moins 85 % en moles, de préférence d'au moins 90 % en moles, d'un ou plusieurs, de préférence de deux ou trois, sélectionnés parmi l'acide oléique, l'acide linoléique et l'acide linolénique.

9. Procédé selon l'une des revendications précédentes, dans lequel l'addition biotechnologique de H₂O est effectuée au moyen d'une hydratase de *S tenotrophomonas nitritireducens.*

10. Procédé selon l'une des revendications précédentes, dans lequel la transformation avec un ou plusieurs groupes de liaison au moins bivalents est effectuée au moyen d'au moins une enzyme sélectionnée dans le groupe constitué d'hydroxylases, de préférence d'estérases, de lipases et/ou de protéases, de préférence de lipases.

11. Procédé selon la revendication 10, dans lequel la transformation avec un ou plusieurs groupes de liaison au moins bivalents est effectuée au moyen d'au moins une lipase, dans lequel la lipase est de préférence la lipase B de *Candida antartica.*

12. Procédé selon l'une des revendications précédentes, dans lequel la transformation du ou des hydroxy-acides gras avec un ou plusieurs groupes de liaison au moins bivalents est effectuée au moyen d'une lipase de *Candida antartica,* plus particulièrement de préférence la lipase B de *Candida antartica* et le ou les groupes de liaison au moins bivalents sont sélectionnés parmi des alcanediols, des alcanetriols, des alcanetétraols et des combinaisons de ceux-ci.

13. Procédé selon la revendication 12, dans lequel le ou les groupes de liaison au moins bivalents
(i) sont sélectionnés parmi des alcanediols, des alcanetriols et des combinaisons de ceux-ci ; ou
(ii) sont sélectionnés parmi l'éthanediol, des propanediols, des butanediols, des pentanediols, des hexanediols, des heptanediols, des octanediols, des butanetriols, des pentanetriols, des hexanetriols, des heptanetriols, des octanetriols, des butantétraols, des pentanetétraols, des hexanetétraols, des heptanetétraols, des octanetétraols et des combinaisons de ceux-ci ; ou
(iii) sont sélectionnés parmi l'éthanediol, des propanediols, des butanediols, des pentanediols, des hexanediols, des heptanediols, des octanediols, des butanetriols, des pentanetriols, des hexanetriols, des heptanetriols, des octanetriols et des combinaisons de ceux-ci.

14. Polyuréthane pouvant être obtenu par transformation d'une composition comprenant un condensat d'hydroxy-acide gras ou un mélange de condensats d'hydroxy-acides gras pouvant être obtenu grâce au procédé selon l'une des revendications précédentes, avec un diisocyanate, un triisocyanate, un tétraisocyanate, un pentaisocyanate ou un hexaisocyanate ou des combinaisons de ceux-ci.
